(19) Europäisches Patentamt
European Patent Office
Office européen des brevets

(11) EP 1 444 206 B1

# (12) EUROPÄISCHE PATENTSCHRIFT

(45) Veröffentlichungstag und Bekanntmachung des Hinweises auf die Patenterteilung:
**15.03.2006 Patentblatt 2006/11**

(21) Anmeldenummer: **02781280.9**

(22) Anmeldetag: **23.10.2002**

(51) Int Cl.:
*C07D 223/16* (2006.01) *A61K 31/55* (2006.01)
*A61P 29/00* (2006.01)

(86) Internationale Anmeldenummer:
**PCT/EP2002/011830**

(87) Internationale Veröffentlichungsnummer:
**WO 2003/037873 (08.05.2003 Gazette 2003/19)**

## (54) SUBSTITUIERTE BENZO[B]AZEPIN-2-ON-VERBINDUNGEN ALS SCHMERZMITTEL

SUBSTITUTED BENZO[B]AZEPIN-2-ONE COMPOUNDS AS ANALGESICS

COMPOSES BENZO[B]AZEPINE-2-ONE SUBSTITUES UTILISES COMME ANALGESIQUES

(84) Benannte Vertragsstaaten:
**AT BE BG CH CY CZ DE DK EE ES FI FR GB GR IE IT LI LU MC NL PT SE SK TR**
Benannte Erstreckungsstaaten:
**LT LV RO SI**

(30) Priorität: **29.10.2001 DE 10153348**

(43) Veröffentlichungstag der Anmeldung:
**11.08.2004 Patentblatt 2004/33**

(73) Patentinhaber: **Grünenthal GmbH**
**52078 Aachen (DE)**

(72) Erfinder:
- **SATTLEGGER, Michael**
  **53123 Bonn (DE)**
- **BUSCHMANN, Helmut**
  **E-08950 Esplugues de Llobregat (ES)**
- **PRZEWOSNY, Michael**
  **52062 Aachen (DE)**
- **ENGLBERGER, Werner**
  **52223 Stolberg (DE)**
- **KOEGEL, Babette-Yvonne**
  **52379 Langerwehe-Hamich (DE)**
- **SCHICK, Hans**
  **10405 Berlin (DE)**

(74) Vertreter: **Kutzenberger, Helga et al**
**Kutzenberger & Wolff,**
**Theodor-Heuss-Ring 23**
**50668 Köln (DE)**

(56) Entgegenhaltungen:
**WO-A-93/15059** **US-A- 5 733 936**

EP 1 444 206 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft substituierte Benzo[b]azepin-2-on-Verbindungen, Verfahren zu deren Herstellung, Arzneimittel enthaltend diese Verbindungen sowie die Verwendung dieser Verbindungen zur Herstellung von Arzneimitteln.

Die Behandlung von Schmerz hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie beispielsweise das Morphin, sind bei der Therapie starker bis sehr starker Schmerzen wirksam. Als unerwünschte Begleiterscheinungen weisen sie jedoch unter anderem Atemdepression, Erbrechen, Sedierung, Obstipation sowie Toleranzentwicklung auf. Sie sind außerdem bei neuropathischen oder inzidentiellen Schmerzen, wie sie insbesondere bei Tumorpatienten häufig auftreten, weniger wirksam.

Aufgabe der vorliegenden Erfindung war es daher, neue Verbindungen zur Verfügung zu stellen, die sich als pharmazeutische Wirkstoffe in Arzneimitteln, vorzugsweise als pharmazeutische Wirkstoffe zur Schmerzbekämpfung, vorzugsweise von chronischen oder neuropathischen Schmerzen, eignen und zur Behandlung oder Prophylaxe von neurodegenerativen Erkrankungen, vorzugsweise Morbus Alzheimer, Morbus Huntington oder Morbus Parkinson, Schlaganfall, cerebralem Infarkt, cerebraler Ischämie, Hirnödem, Unterversorgungszuständen des zentralen Nervensystems, vorzugsweise Hypoxie oder Anoxie, Epilepsie, Schizophrenie, Psychosen bedingt durch erhöhten Aminosäurespiegel, AIDS-Demenz, Encephalomyelitis, Tourette-Syndrom, perinataler Asphyxie, Tinnitus, Migräne, inflammatorischen und/oder allergischen Reaktionen, Depressionen, seelischen Erkrankungen, Harninkontinenz, Juckreiz oder Diarrhoe oder zur Anxiolyse oder Anästhesie eingesetzt werden können.

Erfindungsgemäß wird diese Aufgabe durch Bereitstellung der substituierten Benzo[b]azepin-2-on-Verbindungen den nachstehenden allgemeinen Formeln I und II und jeweils deren Tautomeren, gegebenenfalls in Form ihrer Diastereomeren, reinen Enantiomeren, Racematen, nichtracemischen Gemischen der Enantiomeren oder Diastereomeren sowie jeweils gegebenenfalls in Form entsprechender Basen, Salze und Solvate gelöst, wobei diese Verbindungen insbesondere eine ausgeprägte analgetische Wirkung aufweisen.

Gegenstand der Erfindung sind daher substituierte Benzo[b]azepin-2-on-Verbindungen der allgemeinen Formeln I und II und jeweils deren Tautomere,

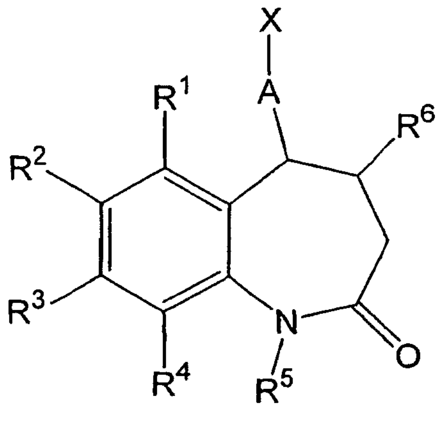

I

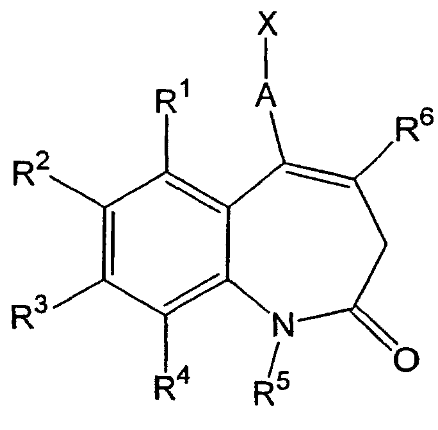

II

worin

$R^1$, $R^2$, $R^3$ und $R^4$, gleich oder verschieden, für einen linearen oder verzweigten, gesättigten oder ungesättigten aliphatischen $C_{1-10}$-Rest oder einen gesättigten oder ungesättigten cycloaliphatischen $C_{3-7}$-Rest, wobei jeder der vorstehend genannten Reste gegebenenfalls über eine Etherbrücke gebunden sein kann, oder Wasserstoff, ein Halogen oder eine Hydroxygruppe stehen,

$R^5$ für Wasserstoff, einen linearen oder verzweigten, gesättigten oder ungesättigten aliphatischen $C_{1-10}$-Rest, einen Aryl- oder einen Heteroarylrest steht,

$R^6$ für Wasserstoff oder einen Rest der Formel $-CH_2-NR^7_2$ steht, wobei die beiden Reste gleich oder verschieden sind und die nachstehende Bedeutung haben oder gemeinsam mit dem sie verbindenden Stickstoffatom als Ringglied einen 3-8-gliedrigen Ring bilden können,

$R^7$ für einen linearen oder verzweigten, gesättigten oder ungesättigten aliphatischen $C_{1-6}$-Rest oder einen gesättigten oder ungesättigten cycloaliphatischen $C_{3-6}$-Rest steht,

A für eine Brücke mit einer der folgenden Formeln steht: $-(CH_2)_{n+2}-$, $-(CH_2)_n-CH=CH-$, $-(CH_2)_nCOO-$, $-(CH_2)_nCONH-$, $-(CH_2)_{n+1}O(CH_2)_pCO-$, $-(CH_2)_{n+1}O-$, $-(CH_2)_{n+1}NR^{1'}-$ steht, worin n für 0,1,2 oder 3 und p für 0 oder 1 steht, $R^{1'}$ die nachstehend angegebene Bedeutung hat und die Bindung zum Rest X immer zuletzt genannt ist,

und X für einen der folgenden Reste der allgemeinen Formeln $X^1$ bis $X^6$ und $X^{16}$ steht, worin der freie Strich die Bindung zur Brücke A symbolisiert und

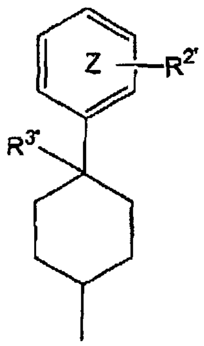

$X^1$

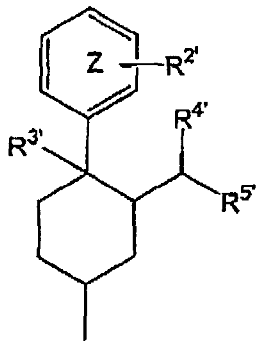

$X^2$

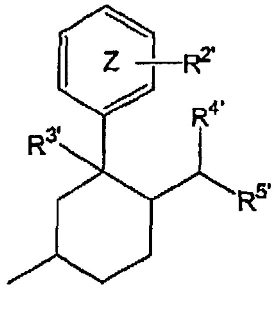

$X^3$

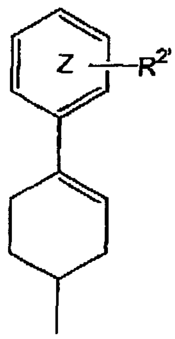

$X^4$

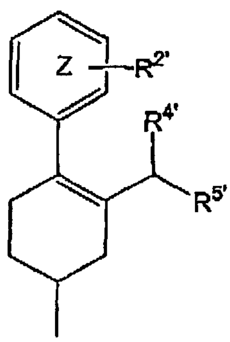

$X^5$

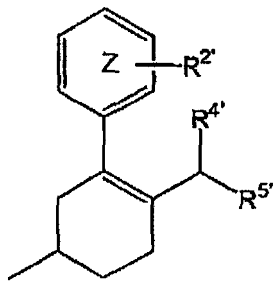

$X^6$

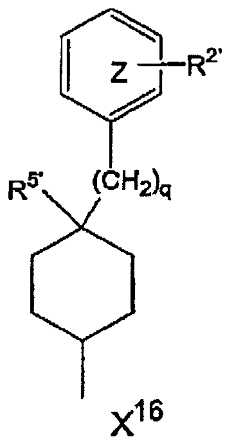

$X^{16}$

worin

$R^{1'}$ für Wasserstoff, einen linearen oder verzweigten, gesättigten oder ungesättigten aliphatischen $C_{1-10}$-Rest, einen gesättigten oder ungesättigten cycloaliphatischen $C_{3-7}$-Rest, einen Aryl- oder Heteroarylrest steht,
$R^{2'}$ für einen linearen oder verzweigten, gesättigten oder ungesättigten aliphatischen $C_{1-10}$. Rest, einen gesättigten oder ungesättigten cycloaliphatischen $C_{3-7}$-Rest oder einen Aryl- oder Heteroarylrest, wobei alle vorstehend genannten Reste gegebenenfalls über eine Ether-, Thioether- oder $SO_2$-Brücke gebunden sein können, oder Wasserstoff, ein Halogen, eine Hydroxy-, Thiol-, Cyano- oder Nitrogruppe oder eine Gruppe der Formel $-NR^{1'}_2$ steht, wobei die beiden Reste $R^{1'}$ gleich oder verschieden sind und die vorstehend angegebene Bedeutung haben,
$R^{3'}$ für einen linearen oder verzweigten, gesättigten oder ungesättigten aliphatischen $C_{1-10}$-Rest , einen gesättigten oder ungesättigten cycloaliphatischen $C_{3-7}$-Rest, einen Aryl- oder Heteroarylrest, wobei alle vorstehend genannten Reste gegebenenfalls über eine Ether- oder eine Esterbrücke gebunden sein können, Wasserstoff, ein Halogen, eine Hydroxygruppe steht,
$R^{4'}$ für Wasserstoff, einen Aryl- oder Heteroarylrest steht, wobei der Aryl- oder Heteroarylrest mindestens einen Substituenten $R^{2'}$ mit der vorstehenden Bedeutung mit Ausnahme von Wasserstoff aufweisen kann,
$R^{5'}$ für einen Rest der Formel $-NR^{6'}_2$ steht, wobei die beiden Reste $R^{6'}$ gleich oder verschieden sein können und die nachstehende Bedeutung haben oder gemeinsam mit dem sie verbindenden Stickstoffatom als Ringglied einen 3-7-gliedrigen Ring bilden können, der gegebenenfalls mindestens einen Sauerstoff und/oder mindestens einen weiteren Stickstoff als Ringatom enthalten kann, wobei der Stickstoff einen Substituenten $R^{10'}$ mit der nachstehenden Bedeutung aufweisen kann,
$R^{6'}$ für einen linearen oder verzweigten, gesättigten oder ungesättigten aliphatischen $C_{1-6}$-Rest, einen gesättigten oder ungesättigten cycloaliphatischen $C_{3-7}$-Rest, einen Aryl- oder Heteroarylrest steht,
$R^{10'}$ für Wasserstoff, einen linearen oder verzweigten, gesättigten oder ungesättigten aliphatischen $C_{1-10}$-Rest, einen Aryl- oder Heteroarylrest steht und
Z für mindestens einen gegebenenfalls vorhandenen Sauerstoff, Schwefel oder Stickstoff als Ringatom steht,
sowie q für 0,1,2 oder 3 steht,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate,

Tautomere der Verbindungen der allgemeinen Formeln I und II sind gegeben, wenn $R^5$ für Wasserstoff steht Es wird immer auch auf diese möglichen Tautomere Bezug genommen.

Bevorzugt sind substituierte Benzo[b]azepin-2-on-Verbindungen der allgemeinen Formeln I und II und jeweils deren Tautomere, worin $R^2$ und $R^3$, gleich oder verschieden, für einen linearen oder verzweigten, gesättigten oder ungesättigten aliphatischen $C_{1-3}$-Rest oder ein Halogen und $R^1$ und $R^4$ jeweils für Wasserstoff, $R^5$ für Wasserstoff oder einen linearen oder verzweigten, gesättigten oder ungesättigten aliphatischen $C_{1-3}$-Rest und $R^6$ für Wasserstoff oder einen Rest der Formel $-CH_2-NR^7_2$ stehen, worin $R^7$ für einen linearen oder verzweigten, gesättigten oder ungesättigten aliphatischen $C_{1-3}$-Rest steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Besonders bevorzugt sind substituierte Benzo[b]azepin-2-on-Verbindungen der allgemeinen Formeln I und II und jeweils deren Tautomere, worin $R^2$ und $R^3$ jeweils für eine Methylgruppe oder ein Chlor und $R^1$ und $R^4$ jeweils für Wasserstoff, $R^5$ für Wasserstoff oder eine Methylgruppe und $R^6$ für Wasserstoff oder einen Rest der Formel $-CH_2-N(CH_3)_2$ stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Bevorzugt sind auch substituierte Benzo[b]azepin-2-on-Verbindungen der allgemeinen Formeln I und II und jeweils deren Tautomere, worin $R^3$ für einen linearen oder verzweigten, gesättigten oder ungesättigten aliphatischen $C_{1-3}$-Rest oder ein Halogen und $R^1$, $R^2$ und $R^4$ jeweils für Wasserstoff, $R^5$ für Wasserstoff oder einen linearen oder verzweigten, gesättigten oder ungesättigten aliphatischen $C_{1-3}$-Rest und $R^6$ für Wasserstoff oder einen Rest der Formel $-CH_2-N(R^7)_2$ stehen, worin $R^7$ für einen linearen oder verzweigten, gesättigten oder ungesättigten aliphatischen $C_{1-3}$-Rest steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Besonders bevorzugt sind auch substituierte Benzo[b]azepin-2-on-Verbindungen der allgemeinen Formeln I und II und jeweils deren Tautomere, worin $R^3$ für eine Methylgruppe oder ein Chlor und $R^1$, $R^2$ und $R^4$ jeweils für

Wasserstoff, $R^5$ für Wasserstoff oder eine Methylgruppe und $R^6$ für Wasserstoff oder einen Rest der Formel $-CH_2-N(CH_3)_2$ stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Bevorzugt sind auch substituierte Benzo[b]azepin-2-on-Verbindungen der allgemeinen Formeln I und II und jeweils deren Tautomere, worin $R^1$ und $R^3$, gleich oder verschieden, für einen linearen oder verzweigten, gesättigten oder ungesättigten aliphatischen $C_{1-3}$-Rest oder ein Halogen und $R^2$ und $R^4$ jeweils für Wasserstoff, $R^5$ für Wasserstoff oder einen linearen oder verzweigten, gesättigten oder ungesättigten aliphatischen $C_{1-3}$-Rest und $R^6$ für Wasserstoff oder einen Rest der Formel $-CH_2-NR^7{}_2$ stehen, worin $R^7$ für einen linearen oder verzweigten, gesättigten oder ungesättigten aliphatischen $C_{1-3}$-Rest steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Besonders bevorzugt sind auch substituierte Benzo[b]azepin-2-on-Verbindungen der allgemeinen Formeln I und II und jeweils deren Tautomere, worin $R^1$ und $R^3$ jeweils für eine Methylgruppe oder ein Chlor und $R^2$ und $R^4$ jeweils für Wasserstoff, $R^5$ für Wasserstoff oder eine Methylgruppe und $R^6$ für Wasserstoff oder einen Rest der Formel $-CH_2-N(CH_3)_2$ stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Bevorzugt sind ferner substituierte Benzo[b]azepin-2-on-Verbindungen der allgemeinen Formeln I und II und jeweils deren Tautomere, worin A für eine Brücke der Formel $-CH_2-COO-$ oder $-CH_2CONH-$ steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Bevorzugt sind ferner substituierte Benzo[b]azepin-2-on-Verbindungen der allgemeinen Formeln I und II und jeweils deren Tautomere, worin X für einen Rest der folgenden Formel steht:

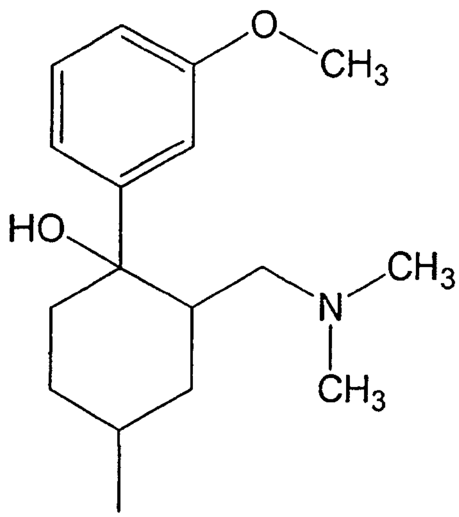

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Ganz besonders bevorzugt sind die folgenden substituierten Benzo[b]azepin-2-on-Verbindungen und ggf. deren Tautomere:

2'-(8-Chlor-2-oxo-2,3-dihydro-1H-benzo[b]azepin-5-yl)essigsäure-[3"-(N,N-dimethylaminomethyl)-4"-hydroxy-4"-(m-methoxyphenyl)cyclohexyl]ester,
2'-(8-Chlor-1-methyl-2-oxo-2,3-dihydro-1H-benzo[b]azepin-5-yl)essigsäure-[3"-(N,N-dimethylaminomethyl)-4"-hydroxy-4"-(m-methoxyphenyl)cyclohexyl]ester,
2'-(8-Chlor-2-oxo-2,3-dihydro-1H-benzo[b]azepin-5-yl)-N-[3"-(N,N-dimethylaminomethyl)-4"-hydroxy-4"-(m-methoxyphenyl)cyclohexyl]acetamid,
2'-(8-Chlor-1-methyl-2-oxo-2,3-dihydro-1H-benzo[b]azepin-5-yl)-N-[3"-(N,N-dimethylaminomethyl)-4"-hydroxy-4"-(m-methoxyphenyl)cyclohexyl]acetamid

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Ein weiterer Gegenstand der vorliegenden Erfindung ist ein Verfahren zur Herstellung von substituierten Benzo [b]azepin-2-on-Verbindungen der vorstehend angegebenen allgemeinen Formeln I und II und jeweils deren Tautomeren und entsprechenden Stereoisomeren, dadurch gekennzeichnet, daß man

A) einen gegebenenfalls substituierten 2-Aminobenzoesäurealkylester der allgemeinen Formel (1), worin $R^1$, $R^2$, $R^3$, $R^4$ und $R^5$ die vorstehend genannte Bedeutung haben und R für eine Alkylgruppe, vorzugsweise eine Methyl- oder Ethylgruppe, steht,

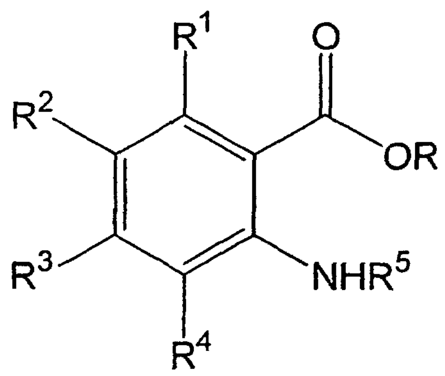

(1)

mit Bernsteinsäuredialkylester der allgemeinen Formel (2), worin R' für eine Alkylgruppe, vorzugsweise eine Methyl- oder Ethylgruppe, und $R^x$ für ein Chlor oder eine Alkoxygruppe, vorzugsweise eine Methoxy- oder Ethoxygruppe, steht,

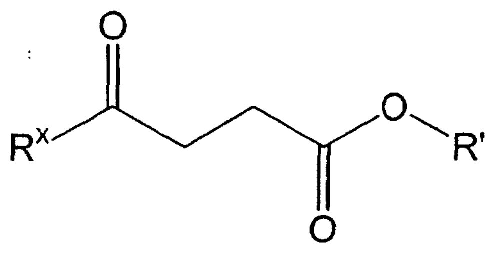

(2)

unter geeigneten Reaktionsbedingungen, in einem geeigneten Lösungsmittel, vorzugsweise Pyridin, umsetzt und anschließend aufarbeitet und gegebenenfalls das gebildete gegebenenfalls substituierte N-(2-Carbalkoxyphenyl) bernsteinsäurealkylesteramid der allgemeinen Formel (3), worin R, R', $R^1$, $R^2$, $R^3$, $R^4$ und $R^5$ die vorstehend genannte Bedeutung haben, reinigt,

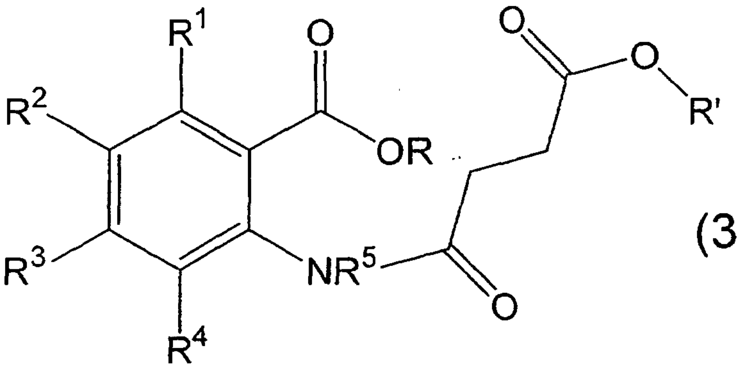

(3)

B) ein gegebenenfalls substituiertes N-(2-Carbomethoxyphenyl)bemsteinsäurealkylesteramid der allgemeinen Formel (3) in Gegenwart von Kalium-tert-butanolat in einem geeigneten Lösungsmittel umsetzt und anschließend aufarbeitet und gegebenenfalls den gebildeten gegebenenfalls substituierten 5-Hydroxy-2-oxo-2,3-dihydro-1H-benzo [b]azepin-4-carbonsäurealkylester der allgemeinen Formel (4), worin R', $R^1$, $R^2$, $R^3$, $R^4$ und $R^5$ die vorstehend genannte Bedeutung haben, reinigt,

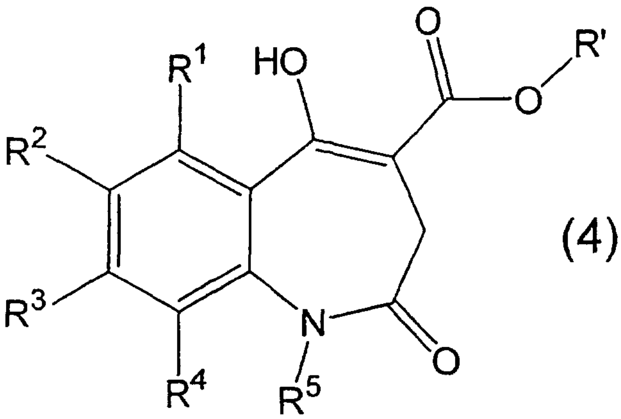

(4)

C) einen gegebenenfalls substituierten 5-Hydroxy-2-oxo-2,3-dihydro-1H-benzo[b]azepin-4-carbonsäurealkylester der allgemeinen Formel (4) in einem Dimethylsulfoxid/Wassergemisch bei erhöhter Temperatur umsetzt und anschließend aufarbeitet und gegebenenfalls das gebildete gegebenenfalls substituierte 2,3,4,5-Tetrahydro-1H-benzo [b]azepin-2,5-dion der allgemeinen Formel (5), worin $R^1$, $R^2$, $R^3$, $R^4$ und $R^5$ die vorstehend genannte Bedeutung haben, reinigt,

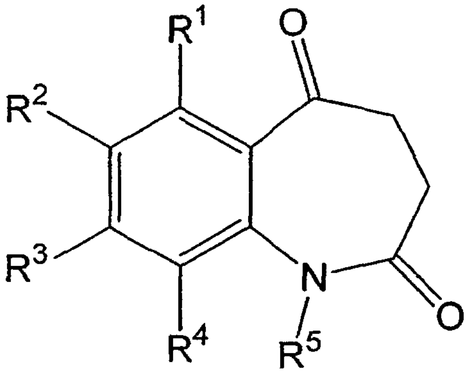

(5)

D) gegebenenfalls ein gegebenenfalls substituiertes 2,3,4,5-Tetrahydro-1H-benzo[b]azepin-2,5-dion der allgemeinen Formel (5) mit einem substituierten Aminomethylen-Hydrochlorid der allgemeinen Formel (6), worin der Rest $R^7$ die vorstehend genannte Bedeutung hat,

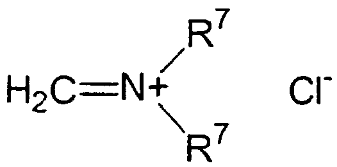

(6)

in Gegenwart einer Säure, vorzugsweise Acetylchlorid, in einem geeigneten Lösungsmittel, vorzugsweise Acetonitril, umsetzt und anschließend aufarbeitet und gegebenenfalls das gebildete gegebenenfalls substituierte Aminomethyl-2,3,4,5-tetrahydro-1H-benzo[b]azepin-2,5-dion der allgemeinen Formel (7), worin $R^1$, $R^2$, $R^3$, $R^4$, $R^5$ und $R^7$ die vorstehend genannte Bedeutung haben, reinigt,

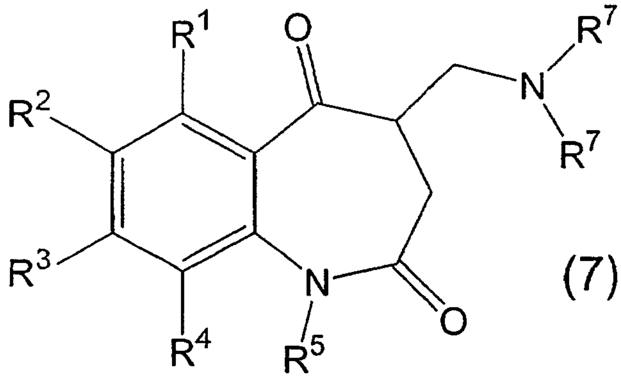

(7)

E) ein gegebenenfalls substituiertes 2,3,4,5-Tetrahydro-1H-benzo[b]azepin-2,5-dion der allgemeinen Formel (8), worin $R^1$, $R^2$, $R^3$, $R^4$, $R^5$ und $R^6$ die vorstehend genannte Bedeutung haben und welches die Verbindungen der allgemeinen Formeln (5) und (7) zusammenfaßt,

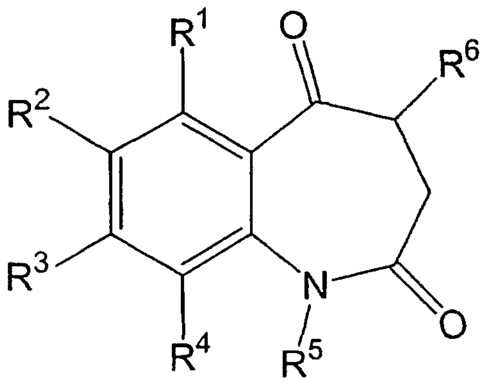

(8)

mit einem Phosphonoalkansäuretrialkylester der allgemeinen Formel (9), worin n die vorstehend genannte Bedeutung hat und R" für eine Alkylgruppe, vorzugsweise eine Methyl- oder Ethylgruppe, steht,

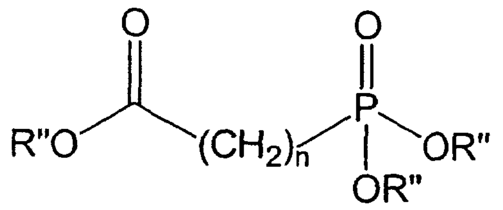

(9)

in Gegenwart einer Base, vorzugsweise Kalium-tert-butanolat, in einem geeigneten Lösungsmittel, vorzugsweise Dimethylformamid, umsetzt und anschließend aufarbeitet und gegebenenfalls die gebildete Verbindung der Formel Y-COOR", worin R" die vorstehend genannte Bedeutung hat und Y für einen Rest der allgemeinen Formel Y steht, worin der freie Strich die Bindung zum Rest -COOR" symbolisiert und

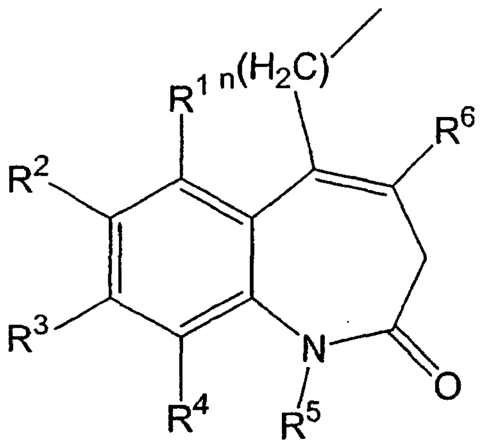

Y

worin $R^1$, $R^2$, $R^3$, $R^4$, $R^5$, $R^6$ und n die vorstehend genannte Bedeutung haben, reinigt,
F) gegebenenfalls einen Ester der Formel Y-COOR" in Gegenwart einer Base, vorzugsweise Natrium- oder Kaliumhydroxid, in einem geeigneten Lösungsmittel, vorzugsweise einem Alkohol/Wassergemisch, umsetzt und anschließend aufarbeitet und gegebenenfalls die gebildete Carbonsäure der Formel Y-COOH, worin Y die vorstehend genannte Bedeutung hat, reinigt,
G) gegebenenfalls eine Carbonsäure der Formel Y-COOH oder einen Carbonsäureester der Formel Y-COOR", worin Y und R" die vorstehend genannte Bedeutung haben, derivatisiert, dadurch daß man

a) eine Carbonsäure oder einen Carbonsäureester der Formel Y-COOH oder Y-COOR" mit Hilfe von Reduktionsmitteln, vorzugsweise Lithiumaluminiumhydrid, in einem geeigneten Lösungsmittel, vorzugsweise Tetrahydrofuran, zu dem entsprechenden Alkohol der Formel Y-$CH_2$-OH reduziert,
b) eine Carbonsäure oder einen Carbonsäureester der Formel Y-COOH oder Y-COOR" mit Hilfe von Reduktionsmitteln, vorzugsweise Diisobutylaluminiumhydrid, in einem geeigneten Lösungsmittel, vorzugsweise Hexan, zu dem entsprechenden Aldehyd der Formel Y-CHO reduziert oder
c) einen Alkohol der Formel Y-$CH_2$-OH gemäß a) mit einem Bromierungsmittel, vorzugsweise $PBr_3$ oder $Ph_3PBr_2$ (mit Ph für Phenylrest) zu dem entsprechenden Bromid der Formel Y-$CH_2$-Br umsetzt

und anschließend aufarbeitet und gegebenenfalls das Produkt reinigt,
H) gegebenenfalls eine Verbindung der Formel $X^1$-$R^{IV}$, worin $X^1$ die vorstehend genannte Bedeutung hat und $R^{IV}$ für eine funktionelle Gruppe steht, herstellt, dadurch daß man

a) 1,4-Cyclohexandionmonoethylenketal, 4-Aminocyclohexan-1-onethylenketal oder 4-Oxocyclohexancarbonsäure mit Magnesium und einem bromierten oder chlorierten, gegebenenfalls substituierten Aromaten oder Heteroaromaten in einem geeigneten Lösungsmittel, vorzugsweise trockenen Diethylether, bei erhöhter Temperatur zu dem entsprechenden Kupplungsprodukt umsetzt und anschließend gegebenenfalls das Ketal durch Umsetzung mit Salzsäure in einem geeigneten Lösungsmittel, vorzugsweise Tetrahydrofuran, spaltet, aufarbeitet und gegebenenfalls das Produkt der Formel $X^{1a}$=O, $X^{1a}$-$NHR^{1'}$ oder $X^{1a}$-$CO_2H$, worin $X^{1a}$ für einen Rest

der Formel $X^{1a}$ steht und $R^{1'}$, $R^{2'}$ und Z die vorstehend genannte Bedeutung haben und der freie Strich die Bindung zum Rest =O, -$NHR^{1'}$ oder -$CO_2H$ symbolisiert, reinigt,

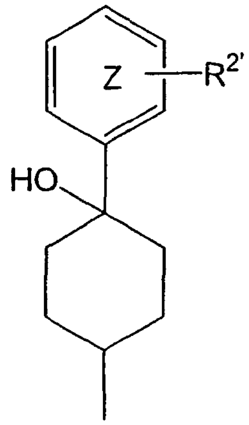

$X^{1a}$

b) gegebenenfalls ein Keton der Formel $X^{1a}$=O in Gegenwart eines geeigneten Reduktionsmittels, vorzugsweise Natriumborhydrid, in einem geeigneten Lösungsmittel, vorzugsweise Methanol, zu dem entsprechenden Alkohol der Formel $X^{1a}$-OH umsetzt, aufarbeitet und gegebenenfalls das Produkt reinigt,
c) gegebenenfalls ein Keton der Formel $X^{1a}$=O unter Stickstoff in einem geeigneten Lösungsmittel, vorzugsweise Tetrahydrofuran, zunächst mit Ammoniumtrifluoracetat und anschließend mit Eisessig und Natriumtriacetoxyborhydrid zu dem entsprechenden Amin der Formel $X^{1a}$-$NH_2$ umsetzt, aufarbeitet und gegebenenfalls das Produkt reinigt,
d) gegebenenfalls eine Carbonsäure der Formel $X^{1a}$-$CO_2H$ durch Umsetzung mit Dicyclohexylcarbodiimid oder durch Überführen in das Carbonsäurechlorid oder ein gemischtes Anhydrid aktiviert, mit Diazomethan in einem geeigneten Lösungsmittel, vorzugsweise Ether, umsetzt und anschließend mit Wasser behandelt, aufarbeitet und gegebenenfalls das Produkt der Formel $X^{1a}$-CO-$CH_2$-OH reinigt,
e) gegebenenfalls die Hydroxygruppe in 4-Position des Cyclohexanringes in dem Rest $X^{1a}$ in Wasserstoff, ein Halogen, eine Ether-, Ester-, Alkyl-, Aryl- oder Heteroarylgruppe umwandelt, dadurch daß man

α) eine Verbindung aus einem der Schritte a)-d) zur Einführung einer Ethergruppe mit einer aliphatischen oder cycloaliphatischen Verbindung in Gegenwart eines geeigneten Katalysators in einem geeigneten Lösungsmittel, vorzugsweise in Gegenwart von Natriumhydrid in Dimethylformamid oder in Gegenwart von Kaliumhydroxid in Dimethylsulfoxid, oder mit einem Alkylierungsmittel in einem geeigneten Lösungsmittel, vorzugsweise mit einer Diazoverbindung in Diethylether, oder mit einer Aryl- oder Heteroarylverbindung in Gegenwart von Diethylazodicarboxylat und Triphenylphosphin umsetzt,
β) eine Verbindung aus einem der Schritte a)-d) zur Einführung eines Halogens mit einem Halogenierungsmittel in einem geeigneten Lösungsmittel, vorzugsweise mit $POCl_3$ in Dimethylformamid, mit $PPh_3/Cl_2$, mit $PPh_3/Br_2$, mit Triphenylphosphin/n-Chlorsuccinimid oder mit $HCl/ZnCl_2$, umsetzt,
γ) eine Verbindung aus Schritt β) zur Einführung eines Wasserstoffs mit Wasserstoff in Gegenwart eines geeigneten Katalysators, vorzugsweise Palladium/Kohle, in einem geeigneten Lösungsmittel umsetzt,
δ) eine Verbindung aus Schritt β) zur Einführung eines aliphatischen oder cycloaliphatischen Restes oder einer Aryl- oder Heteroarylgruppe mit einer aliphatischen oder cycloaliphatischen Boronsäure bzw. einem Boronsäureester oder einer Aryl- oder Heteroarylbordihydroxid-Verbindung in Gegenwart von Palladium (II)acetat und Kaliumcarbonat in einem geeigneten Lösungsmittel, vorzugsweise einem Dimethylformamid/Wasser-Gemisch, umsetzt oder
ε) eine Verbindung aus einem der Schritte a)-d) zur Einführung einer Estergruppe mit einem Carbonsäurechlorid in Gegenwart eines geeigneten Katalysators in einem geeigneten Lösungsmittel umsetzt

und anschließend aufarbeitet und gegebenenfalls die gebildete Verbindung der Formel $X^1$-$R^{IV}$, worin $X^1$ für die Formel $X^1$ steht

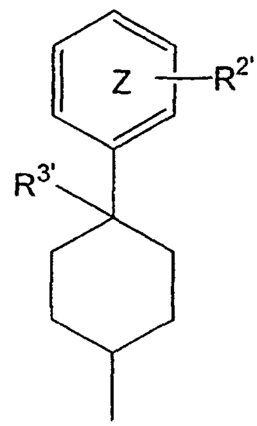

$X^1$

und $R^{IV}$, $R^{2'}$ und $R^{3'}$ die vorstehend genannte Bedeutung haben, reinigt,

I) gegebenenfalls eine Verbindung der Formel X-$R^{IV}$, worin X die vorstehend genannte Bedeutung hat und $R^{IV}$ für eine funktionelle Gruppe steht, derivatisiert, dadurch daß man

a) ein Keton der Formel X=O 1) mit Methoxymethyltriphenylphosphiniumchlorid unter Schutzgas in einem geeigneten Lösungsmittel, vorzugsweise in Dimethylformamid, in Gegenwart von Natriumhydrid und anschließend mit Salzsäure oder 2) mit $Me_3S^+BF_4^-$ zu dem entsprechenden, um ein Kohlenstoffatom verlängerten Aldehyd X-CHO umsetzt,
b) einen Aldehyd der Formel X-CHO gemäß a) mit einem Reduktionsmittel, vorzugsweise Natriumborhydrid, in einem geeigneten Lösungsmittel, vorzugsweise einem Ethanol/Wasser-Gemisch zu dem entsprechenden Alkohol X-$CH_2$-OH umsetzt,
c) einen Alkohol X-$CH_2$-OH gemäß b) oder der Formel X-OH mit einem Bromierungsmittel, vorzugsweise Triphenylphosphindibromid, in einem geeigneten Lösungsmittel, vorzugsweise Acetonitril, zu dem entsprechenden Bromid der Formel X-$CH_2$-Br bzw. X-Br umsetzt,
d) ein Bromid der Formel X-$CH_2$-Br gemäß c) mit einem Phosphin der Formel $PR^V_3$, worin $R^V$ für einen organischen Rest steht, vorzugsweise für einen Phenylrest, in einem geeigneten Lösungsmittel, vorzugsweise Toluol, Ether, Tetrahydrofuran oder Aceton, unter Kühlung und Schutzgas zu dem entsprechenden Phosphoniumsalz $R^V_3P^+$-$CHX^-$ umsetzt oder
e) ein Bromid der Formel X-$CH_2$-Br gemäß c) mit einem Phosphit der Formel HP(O)($OR^{VI}$)$_2$, worin $R^{VI}$ für einen organischen Rest steht, bei erhöhter Temperatur, vorzugsweise 200°C, zu dem entsprechenden Phosphonat ($R^{VI}O$)$_2$P(O)-$CH_2$-X umsetzt

und anschließend aufarbeitet und gegebenenfalls das Produkt reinigt,
J) eine Verbindung aus dem Schritt F) oder G), worin Y die vorstehend genannte Bedeutung hat, mit einer Verbindung der Formel $X^1$-$R^{IV}$ aus Schritt H) oder einer Verbindung X-$R^{IV}$ aus Schritt I), worin X, $X^1$ und $R^{IV}$ die vorstehend genannte Bedeutung haben, umsetzt, dadurch daß man

a) eine Carbonsäure der Formel Y-COOH mit einem Amin der Formel X-$NH_2$ in Gegenwart eines geeigneten Kondensationsmittels, vorzugsweise Dicyclohexylcarbodiimid, 1-Hydroxybenzotriazol und N-Methylmorphin, in einem geeigneten Lösungsmittel, vorzugsweise Dimethylformamid, unter Ausbildung einer Amidbrücke umsetzt,
b) eine Carbonsäure der Formel Y-COOH mit einem Alkohol der Formel X-OH in Gegenwart eines geeigneten Kondensationsmittels in einem geeigneten Lösungsmittel unter Ausbildung einer Esterbrücke umsetzt, vorzugsweise erfolgt die Umsetzung in Gegenwart von Methylimidazol und 1-(Mesitylen-2'-sulfonyl)-3-nitro-1,2,4-triazol in Tetrahydrofuran oder in Gegenwart von Dicyclohexylcarbodiimid, 1-Hydroxybenzotriazol und N-Methylmorphin in Dimethylformamid,
c) ein Bromid der Formel Y-$CH_2$-Br mit einer Verbindung der Formel X-CO($CH_2$)$_p$-OH, worin p die vorstehend genannte Bedeutung hat, unter Schutzgas in Gegenwart eines geeigneten Katalysators, vorzugsweise Natriumhydrid oder Kalium-tert-butylat, in einem geeigneten Lösungsmittel, vorzugsweise Dimethylformamid, unter Ausbildung einer Brücke der Formel -CO($CH_2$)$_p$-O-$CH_2$- umsetzt,
d) einen Alkohol der Formel Y-$CH_2$-OH mit einem Bromid der Formel X-Br unter Schutzgas in Gegenwart eines

geeigneten Kondensationsmittels, vorzugsweise Natriumhydrid oder Kalium-tert-butylat, in einem geeigneten Lösungsmittel, vorzugsweise Dimethylformamid, unter Ausbildung einer Etherbrücke umsetzt,

e) ein Bromid der Formel Y-$CH_2$-Br mit einem Alkohol der Formel X-OH unter Schutzgas in Gegenwart eines geeigneten Kondensationsmittels, vorzugsweise Natriumhydrid oder Kalium-tert-butylat, in einem geeigneten Lösungsmittel, vorzugsweise Dimethylformamid, unter Ausbildung einer Etherbrücke umsetzt,

f) einen Aldehyd der Formel Y-CHO mit einem Amin der Formel X-$NHR^{1'}$ in Gegenwart eines geeigneten Reduktionsmittels, vorzugsweise Natriumcyanoborhydrid und Natriumtriacetoxyborhydrid, in einem geeigneten Lösungsmittel, vorzugsweise einem Gemisch aus Tetrahydrofuran und 1,2-Dichlorethan, unter Ausbildung einer Aminobrücke umsetzt,

g) einen Aldehyd der Formel Y-CHO mit einem Phosphoniumsalz $R^V{}_3P^+$-$CHX^-$, worin $R^V$ die vorstehend genannte Bedeutung hat, unter Schutzgas in Gegenwart geeigneter Katalysatoren in einem geeigneten Lösungsmittel, vorzugsweise in Gegenwart von Natriummethanolat in einem Gemisch aus Hexan, Diethylether und/oder Diisopropylether oder in Gegenwart von Natriumhydrid, Kalium-tert-butylat oder einem Lithiumamid in Dimethylformamid oder Dimethylsulfoxid, unter Ausbildung einer -CH=CH-Brücke umsetzt oder

h) einen Aldehyd der Formel Y-CHO mit einem Phosphonat der Formel $(R^{VI}O)_2P(O)$-$CH_2$-X, worin $R^{VI}$ die vorstehend genannte Bedeutung hat, unter Schutzgas in Gegenwart geeigneter Katalysatoren, vorzugsweise Natriummethanolat, Natriumhydroxid, Kaliumhydroxid, Natriumhydrid, Kalium-tert-butylat oder einem Lithiumamid, in einem geeigneten Lösungsmittel, vorzugsweise Dimethylformamid, Dimethylsufoxid, Diethylether, Tetrahydrofuran, unter Ausbildung einer -CH=CH-Brücke umsetzt und

i) gegebenenfalls die -CH=CH-Brücke aus dem Schritt g) oder h) durch Wasserstoff, vorzugsweise bei Normaldruck oder erhöhtem Druck bis zu 100 bar, in Gegenwart geeigneter Katalysatoren, vorzugsweise Übergangsmetallen oder Übergangsmetallverbindungen, vorzugsweise Palladium oder seine Salze, Rhodium oder seine Komplexe, in einem geeigneten Lösungsmittel, vorzugsweise Dimethylformamid, Methanol oder Ethanol, bei einer Temperatur zwischen 20 und 100°C unter Ausbildung einer -$CH_2$-$CH_2$-Brücke hydriert

und anschließend aufarbeitet und gegebenenfalls das Produkt reinigt,

K) gegebenenfalls die Doppelbindung im 7-Ring eines der Reaktionsprodukte aus dem Schritt J) durch Wasserstoff, vorzugsweise bei Normaldruck oder erhöhtem Druck bis zu 100 bar, in Gegenwart geeigneter Katalysatoren, vorzugsweise Übergangsmetallen oder Übergangsmetallverbindungen, vorzugsweise Palladium oder seine Salze, Rhodium oder seine Komplexe, in einem geeigneten Lösungsmittel, vorzugsweise Dimethylformamid, Methanol oder Ethanol, bei einer Temperatur zwischen 20 und 100°C hydriert und anschließend aufarbeitet und gegebenenfalls das Produkt reinigt.

Die zum Einsatz kommenden Lösungsmittel und Umsetzungsbedingungen entsprechen den für diese Typen von Reaktionen üblichen Lösungsmitteln und Umsetzungsbedingungen.

Die verwendeten Ausgangsverbindungen zum Aufbau des Benzo[b]azepin-2-on-Gerüstes Bernsteinsäuredialkylester der allgemeinen Formel (2) und gegebenenfalls substituierte 2-Aminobenzoesäurealkylester der allgemeinen Formel (1) sind am Markt erhältlich.

Die Umsetzung von Bernsteinsäuredialkylester und 2-Aminobenzoesäurealkylestern zur Vorstufe des Benzo [b]azepin-2-on-Gerüstes ist dem Fachmann als Schotten-Baumann-Reaktion aus der Literatur bekannt. Die Reaktion, die zum Ringschluß führt, und die anschließend Umwandlung zum 2,3,4,5-Tetrahydro-1H-benzo[b]azepin-2,5-dion ist aus H.B. MacPhillamy et al, Journal of the American Chemical Society, 80, 2172 (1958) und der darin zitierten Literatur bekannt. Die Umsetzung mit Aminomethylen-Verbindungen ist aus H. Böhme, K. Hartke, Chemische Berichte, 93, 1305 (1960) und G. Kinast, L.-F. Tietze, Angewandte Chemie, 88, 261 (1976) und der jeweils darin zitierten Literatur bekannt. Die Umsetzung mit Phosphonoalkansäuretrialkylestern ist in G. Drefahl, K. Ponsold; H. Schick, Chemische Berichte, 97, 2011 (1964) und der darin zitierten Literatur beschrieben.

Gegebenenfalls sind Derivatisierungsreaktionen erforderlich, die die funktionellen Gruppen zur Verknüpfung des Benzo[b]azepin-2-on-Gerüstes über die Brücke A mit dem Rest X einführen. Die Verseifung von Estern erfolgt nach den üblichen, dem Fachmann bekannten Methoden. Die übrigen Umsetzungen sind aus der folgenden Literatur und der darin zitierten Literatur bekannt: die Reduktion von Carbonsäuren oder Carbonsäureestern zu Alkoholen aus O. Vogl, M. Pöhm, Monatsh. Chem. 83, 541 (1952); A.K. Saund, N.K. Mathur; Ind. J. Chem. 9, 936 (1971), die Reduktion von Carbonsäuren oder Carbonsäureestern zu Aldehyden aus A. Ito, R. Takahashi, Y. Baba; Chem. Pharm. Bull. 23, 3081 (1975); E. Winterfeld; Synthesis (1975), 617; H. Khatri, C.H. Stammer; J. Chem. Soc., Chem. Commun. (1979), 79; D.H. Rich, E.T.O. Sun; J. Med. Chem. 23, 27 (1980), die Umsetzung von Alkoholen zu Bromiden aus J. Am Chem. Soc. 48, 1080 (1926); J. Chem. Soc., 636 (1943); Org. Synth. Coll., Vol. 2, 358 (1943); Liebigs Ann. Chem. 626, 26 (1959); J. Am. Chem. Soc. 86, 964 (1964); J. Am. Chem. Soc. 99, 1612 (1977).

Die Ausgangsverbindungen zur Synthese von Verbindungen mit dem Rest $X^1$, 1,4-Cyclohexandionmonoethylenketal, 4-Oxocyclohexancarbonsäure und 4-Aminocyclohexan-1-onethylenketal, sind bekannt. 1,4-Cyclohexandionmonoethylketal und 4-Oxocyclohexancarbonsäure sind am Markt erhältlich oder können nach üblichen, dem Fachmann bekannten Methoden gewonnen werden. 4-Aminocyclohexan-1-onethylenketal ist aus H.-J. Teuber, Liebigs Ann. Chem., 781 (1990) und M. Mimura, Chem. Pharm. Bull., 41, 1971 (1993) bekannt.

Die Umsetzungen zur Synthese von Verbindungen $X^1$-$R^{IV}$ erfolgen nach üblichen, dem Fachmann bekannten Methoden. Die Umsetzung eines Cyclohexanons mit einen chlorierten oder bromierten, gegebenenfalls substituierten Aromaten oder Heteroaromaten ist aus Chem. Ber. 68, 1068 (1935), An. Quim. 64, 607 (1968) und Indian J. Biochem. 5, 79 (1968) bekannt.

Gegebenenfalls erfolgt eine Veränderung oder ein Austausch der Hydroxygruppe in 4-Position des Cyclohexanringes im Rest $X^1$. Die Umsetzungen können nach üblichen, dem Fachmann bekannten Methoden durchgeführt werden und sind aus der folgenden Literatur und der darin zitierten Literatur bekannt: die Alkylierung der Hydroxygruppe aus R.M. Bowman et al, Journal of the Chemical Society (C), 2368 (967); C.G. Neville et al, Journal of the Chemical Society, Perkin Trans. I, 259 (1991); F. Arnt et al, Chemische Berichte, 86, 951 (1953), Journal of Organic Chemistry, 52, 4665 (1987) und Tetrahedron 35, 2169 (1979), die Arylierung oder Heteroarylierung der Hydroxygruppe aus Journal of the American Chemical Society, 107, 3891 (1985), die Einführung eines Halogens aus Journal of the American Chemical Society, 76, 6073 (1954) und Journal of the American Chemical Society, 86, 964 (1964), Journal of the Chemical Society, 636 (1943), Journal of the American Chemical Society, 106, 3286 (1984), Journal of the Chemical Society, 2281 (1954) und Synthesis, 746 (1980), die Einführung eines Alkyl-, Aryl- oder Heteroarylrestes aus A. Suzuki, Acc. Chem. Res., 15, 178 (1982); A. Suzuki, Pure Appl. Chem., 57, 1749 (1985); A Suzuki, Pure Appl. Chem., 63, 419 (1991), A. Suzuki, Pure Appl. Chem., 66, 213 (1994), die Umwandlung von Chloriden zu Alkanen aus Journal of Organic Chemistry, 23, 1938 (1958), die Veresterung der Hydroxygruppe aus W. König, R. Geiger, Chem. Ber. 103, 788 (1970).

Verbindungen mit Resten, die unter die allgemeinen Reste $X^2$ bis $X^6$ und $X^{16}$ fallen, sind aus der folgenden Literatur bekannt: $X^2$ und $X^5$ aus den deutschen Patentanmeldung P 3217639, $X^4$ aus D. Lednicer, J. Med. Chem., 15, 1235 (1972), $X^3$ und $X^6$ aus der deutschen Patentanmeldung P 19525137, $X^{18}$ aus den deutschen Patentanmelttungen P 101356366 und P 101356374,

Aus der Literatur sind Verbindungen X-OH, X-$NHR^{1'}$, X-$CO(CH_2)_pOH$ und X=O bekannt oder sie können aus bekannten, am Markt erhältlichen Verbindungen nach üblichen, dem Fachmann bekannten Methoden oder nach Methoden, wie sie in der deutschen Patentanmeldung P100494811 beschrieben sind, hergestellt werden.

Gegebenenfalls sind Derivatisierungsreaktionen erforderlich, die die funktionellen Gruppen zur Verknüpfung des Restes X mit dem Benzo[b]azepin-2-on-Gerüst über die Brücke A einführen. Diese Umsetzungen können nach üblichen, dem Fachmann bekannten Methoden erfolgen und sind aus der folgenden Literatur und der darin zitierten Literatur bekannt: die Umsetzung von Ketonen zu um ein Kohlenstoff verlängerten Aldehyden aus der deutschen Patentanmeldung P 140494811; J. Nat. Prod. 44, 557 (1981) und Synth. Commun. 12, 613 (1982), die Reduktion von Aldehyden zu Alkoholen aus der deutschen Patentanmeldung P 100494811 und Chem. Commun. 535 (1975), die Umsetzung von Alkoholen zu Bromiden aus J. Am Chem. Soc. 48, 1080 (1926); J. Chem. Soc., 636 (1943); Org. Synth. Coll., Vol. 2, 358 (1943); Liebigs Ann. Chem. 626, 26 (1959); J. Am. Chem. Soc. 86, 964 (1964); J. Am. Chem. Soc. 99, 1612 (1977), die Darstellung der Phosphonate und der Phosphoniumsalze ist aus M. Schlosser, Top. Stereochem. 5, 1 (1970); R. Broos, D. Tavernier, M. Anteunis, J. Chem. Educ. 55, 813 (1978); G. Wittig, Angew. Chem. 92, 671 (1980); H.J. Bestmann; Pure Appl. Chem. 52, 771 (1980) und L. Horner, H. Hoffmann, H.G. Wippel, G. Klahre; Chem. Ber. 92, 2499 (1959); J. Gillois, G. Guillerm, M. Savignac, E. Stephan, L. Vo Quang, J. Chem. Educ. 57, 161 (1980); B.A. Arbusov; Pure Appl. Chem. 9, 307 (1964); A.K. Bhattacharya, G. Thyagarajan; Chem. Rev. 81, 415 (1981).

Die Verknüpfung des Restes X mit dem Benzo[b]azepin-2-on-Gerüst über die Brücke A kann nach üblichen, dem Fachmann bekannten Methoden erfolgen und ist aus der folgenden Literatur und der jeweils darin zitierten Literatur bekannt: die Umsetzung von Carbonsäuren mit Alkoholen oder Aminen in Gegenwart von Dicyclohexylcarbodiimid aus W. König, R. Geiger, Chem. Ber. 103, 788 (1970), die Umsetzung von Carbonsäuren mit Alkoholen in Gegenwart von 1-(Mesitylen-2'-sulfonyl)-3-nitro-1,2,4-triazol aus Tetrahedron 36, 3075 (1980), die Veretherung aus Tetrahedron 35, 2169 (1979), Tetrahedron Lett. (1973), 21; Synthesis, 434 (1974); J. Org. Chem. 52, 4665 (1987), die reduktive Aminierung aus Org. React. 3, 174 (1948); J. Am. Chem. Soc. 91, 3996 (1969); Org. Prep. Proced. Int. 11, 201 (1979); Org. Prep. Proced. Int. 17, 317 (1985), die Wittig- oder Wittig-Horner-Emmons-Reaktion aus G. Wittig, Angew. Chem. 92, 671 (1980); H.J. Bestmann; Pure Appl. Chem. 52, 771 (1980) und L. Horner, H. Hoffmann, H.G. Wippel, G. Klahre; Chem. Ber., 92, 2499 (1959); J. Gillois, G. Guillerm, M. Savignac, E. Stephan, L. Vo Quang; J. Chem. Educ. 57, 161 (1980); B.A. Arbusov; Pure Appl. Chem. 9, 307 (1964); A.K. Bhattacharya, G. Thyagarajan; Chem. Rev. 81, 415 (1981) und die Hydrierung aus Synthesis (1978), 329; J. Org. Chem. 34, 3684 (1969); J. Am. Chem. Soc. 91, 2579 (1969).

Die entsprechenden Literaturbeschreibungen werden hiermit als Referenz eingeführt und gelten als Teil der Offenbarung.

Die erfindungsgemäßen substituierten Benzo[b]azepin-2-on-Verbindungen der allgemeinen Formeln I und II und jeweils deren Tautomere sowie entsprechende Stereoisomere können sowohl in Form ihrer freien Basen als auch in Form entsprechender Salze isoliert werden.

Die freien Basen der jeweiligen erfindungsgemäßen Verbindungen der allgemeinen Formel I oder II, ihrer

Tautomere sowie entsprechender Stereoisomere können durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden physiologisch verträglichen Salze überführt werden.

Die freien Basen der jeweiligen erfindungsgemäßen Verbindungen der allgemeinen Formel I oder II, ihrer Tautomere sowie entsprechender Stereoisomere können ebenfalls bevorzugt durch Versetzen der in einem geeigneten organischen Lösungsmittel, wie z.B. Butan-2-on (Methylethylketon), gelösten erfindungsgemäßen Verbindungen der allgemeinen Formel I oder II, ihrer Tautomere und entsprechender Stereoisomere als freie Basen mit Trimethylsilylchlorid (TMSCl) in die entsprechenden Hydrochloride überführt werden.

Die freien Basen der jeweiligen erfindungsgemäßen Verbindungen der allgemeinen Formel I oder II, ihrer Tautomeren und entsprechender Stereoisomere können mit der freien Säure oder einem Salz eines Zuckerersatzstoffes, wie z.B. Saccharin, Cyclamat oder Acesulfam, in die entsprechenden physiologisch verträglichen Salze überführt werden.

Die erfindungsgemäßen Verbindungen der allgemeinen Formeln I und II, ihre Tautomere und jeweils entsprechende Stereoisomere können gegebenenfalls, ebenso wie die entsprechenden Säuren, die entsprechenden Basen oder Salze dieser Verbindungen, auch in Form ihrer Solvate, vorzugsweise ihrer Hydrate, erhalten werden.

Sofern die erfindungsgemäßen substituierten Benzo[b]azepin-2-on-Verbindungen der allgemeinen Formeln I und II und deren Tautomere nach dem erfindungsgemäßen Herstellungsverfahren in Form von Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Benzo[b]azepin-2-on-Verbindungen der allgemeinen Formeln I und II, deren Tautomere und entsprechende Stereoisomere sowie jeweils die entsprechenden Basen, Salze und Solvate sind toxikologisch unbedenklich und eignen sich daher als pharmazeutische Wirkstoffe in Arzneimitteln.

Ein weiterer Gegenstand der vorliegenden Erfindung sind daher Arzneimittel, die wenigstens eine erfindungsgemäße substituierte Benzo[b]azepin-2-on-Verbindung der allgemeinen Formel I oder II und/oder ein entsprechendes Tautomeres, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säure oder ihrer Basen oder in Form ihres Salzes, insbesondere des physiologisch verträglichen Salzes, oder in Form ihres Solvates, insbesondere des Hydrates sowie gegebenenfalls physiologisch verträgliche Hilfsstoffe enthalten. Selbstverständlich können die erfindungsgemäßen Arzneimittel auch Mischungen aus zwei oder mehr der vorstehend genannten erfindungsgemäßen Verbindungen aufweisen.

Sofern die erfindungsgemäßen substituierten Benzo[b]azepin-2-on-Verbindungen der allgemeinen Formeln I oder II oder deren Tautomere oder deren entsprechende Salze, Basen oder Solvate chiral sind, können sie - wie bereits aufgeführt - in Form ihrer Racemate, ihrer reinen Enantiomeren, ihrer reinen Diastereomeren oder in Form eines Gemisches aus wenigstens zwei der vorstehend genannten Stereoisomeren in dem erfindungsgemäßen Arzneimittel vorliegen.

Vorzugsweise eignen sich die erfindungsgemäßen Arzneimittel zur Bekämpfung von Schmerzen, vorzugsweise von chronischen oder neuropathische Schmerzen, und zur Behandlung oder Prophylaxe von neurodegenerativen Erkrankungen, vorzugsweise Morbus Alzheimer, Morbus Huntington oder Morbus Parkinson, Schlaganfall, cerebralem Infarkt, cerebraler Ischämie, Hirnödem, Unterversorgungszuständen des zentralen Nervensystems, vorzugsweise Hypoxie oder Anoxie, Epilepsie, Schizophrenie, Psychosen bedingt durch erhöhten Aminosäurespiegel, AIDS-Demenz, Encephalomyelitis, Tourette-Syndrom, perinataler Asphyxie, Tinnitus, Migräne, inflammatorischen und/oder allergischen Reaktionen, Depressionen, seelischen Erkrankungen, Harninkontinenz, Juckreiz oder Diarrhoe oder zur Anxiolyse oder Anästhesie.

Die Verwendung wenigstens einer substituierten Benzo[b]azepin-2-on-Verbindung der allgemeinen Formel I oder II oder eines Tautomeren, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säure oder ihrer Base oder in Form ihres Salzes, insbesondere eines physiologisch verträglichen Salzes, oder in Form ihres Solvates, insbesondere des Hydrates, zur Herstellung eines Arzneimittels zur Bekämpfung von Schmerzen, vorzugsweise von chronischen oder neuropathischen Schmerzen, und zur Behandlung oder Prophylaxe von neurodegenerativen Erkrankungen, vorzugsweise Morbus Alzheimer, Morbus Huntington oder Morbus Parkinson, Schlaganfall, cerebralem Infarkt, cerebraler Ischämie, Hirnödem, Unterversorgungszuständen des zentralen Nervensystems, vorzugsweise Hypoxie oder Anoxie, Epilepsie, Schizophrenie, Psychosen bedingt durch erhöhten Aminosäurespiegel, AIDS-Demenz, Encephalomyelitis, Tourette-Syndrom, perinataler Asphyxie, Tinnitus, Migräne, inflammatorischen und/oder allergischen Reaktionen, Depressionen, seelischen Erkrankungen, Harninkontinenz, Juckreiz oder Diarrhoe oder zur Anxiolyse oder Anästhesie ist ebenfalls Gegenstand der vorliegenden Erfindung.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden.

Neben wenigstens einer erfindungsgemäßen substituierten Benzo[b]azepin-2-on-Verbindung der allgemeinen Formel I oder II und/oder eines entsprechenden Tautomeren, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säure oder ihrer Base oder in Form ihres Salzes, insbesondere eines physiologisch verträglichen Salzes, oder in Form ihres Solvates, insbesondere des Hydrates, enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel I oder II oder deren Tautomere, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate, in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen auch verzögert freisetzen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen erfindungsgemäßen substituierten Benzo[b]azepin-2-on-Verbindung der allgemeinen Formel I oder II oder eines Tautomeren, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säure oder ihrer Base oder in Form ihres Salzes, insbesondere eines physiologisch verträglichen Salzes, oder in Form ihres Solvates, insbesondere des Hydrates, kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens einer erfindungsgemäßen Verbindung appliziert.

Die Untersuchung auf analgetische Wirksamkeit wurde im Phenylchinon-induzierten Writhing an der Maus (modifiziert nach I.C. Hendershot, J. Forsaith, J. Pharmacol. Exp. Ther. 125, 237-240 (1959)) durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Substanzdosis erhielten 10 Minuten nach intravenöser Gabe der getesteten Verbindungen 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden einzeln in Beobachtungskäfige gesetzt. Mittels eines Drucktastenzählers wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Als Kontrolle wurden Tiere mitgeführt, die nur physiologische Kochsalzlösung erhielten.

Die Verbindungen wurden in der Standarddosis von 10 mg/kg getestet. Die Hemmung der Writhingreaktionen durch eine Substanz wurde nach folgender Formel berechnet:

$$\% \text{ Hemmung} = 100 - \left[ \frac{\text{Writhingreaktion behan. Tiere}}{\text{Writhingreaktion Kontrolle}} \text{ X } 100 \right]$$

Im folgenden wird die Erfindung anhand von Beispielen erläutert. Diese Erläuterungen sind lediglich beispielhaft und schränken den allgemeinen Erfindungsgedanken nicht ein.

## Beispiele

Die Ausbeuten der erfindungsgemäßen Beispielverbindungen wurden nicht optimiert.

### Beispiel 1:

**Synthese von 2'-(8-Chlor-2-oxo-2,3-dihydro-1H-benzo[b]azepin-5-yl)essigsäure**

1. Schritt:

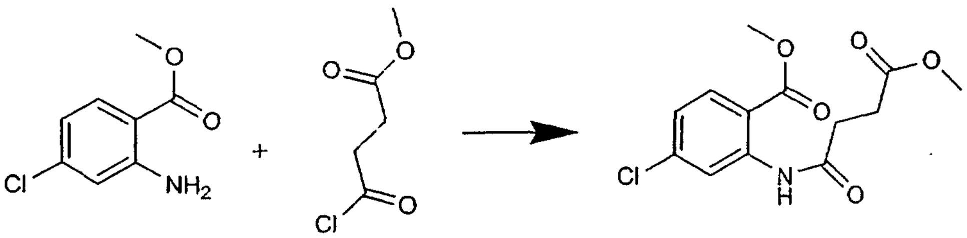

Die N-Acylierung von 2-Amino-4-chlorbenzoesäuremethylester mit Bernsteinsäuremethylesterchlorid in Pyridin lieferte N-(2-Carbomethoxy-5-chlorphenyl)bemsteinsäuremethylesteramid mit einer Ausbeute von 90 %. Der Schmelzpunkt der Verbindung lag bei 95-96°C.

2. Schritt:

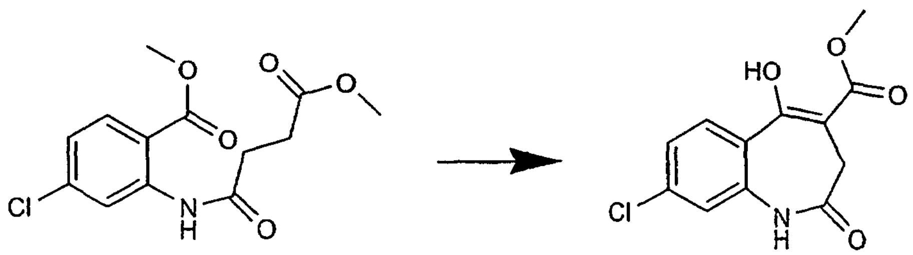

40 g (133 mmol) N-(2-Carbomethoxy-5-chlorphenyl)bernsteinsäuremethylesteramid wurden in THF in Gegenwart von Kalium-tert-butanolat innerhalb von 5h bei Raumtemperatur zu 8-Chlor-5-hydroxy-2-oxo-2,3-dihydro-1H-benzo [b]azepin-4-carbonsäuremethylester umgesetzt. Die Ausbeute betrug 81%.

3. Schritt:

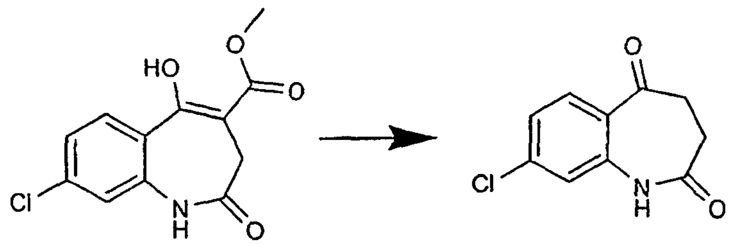

30.0 g (0,11 mol) 8-Chlor-5-hydroxy-2-oxo-2,3-dihydro-1H-benzo[b]azepin-4-carbonsäuremethylester wurden in DMSO/$H_2O$ (9:1) für 6 h auf 150°C erhitzt. Die Ausbeute an 8-Chlor-2,3,4,5-tetrahydro-1H-benzo[b]azepin-2,5-dion betrug 83%.

4. Schritt:

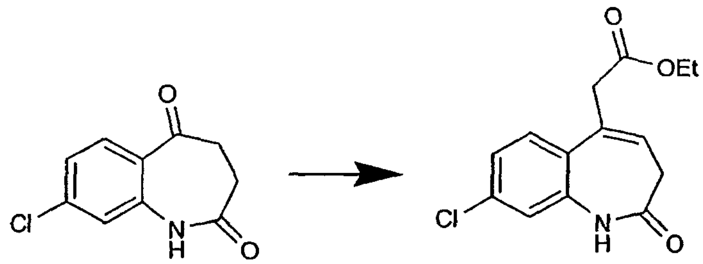

6.0 g (28.6 mmol) 8-Chlor-2,3,4,5-tetrahydro-1H-benzo[b]azepin-2,5-dion wurden mit 7.7 g (34.3 mmol) Phosphonoessigsäuretriethylester und 3.85 g (34.34 mmol) Kalium-tert-butanolat in DMF 8h bei 65°C unter Argon umgesetzt. 2'-(8-Chlor-2-oxo-2,3-dihydro-1H-benzo[b]azepin-5-yl)essigsäureethylester fiel als gelbes Öl mit einer Ausbeute von 4.23 g (53%) an.

5. Schritt:

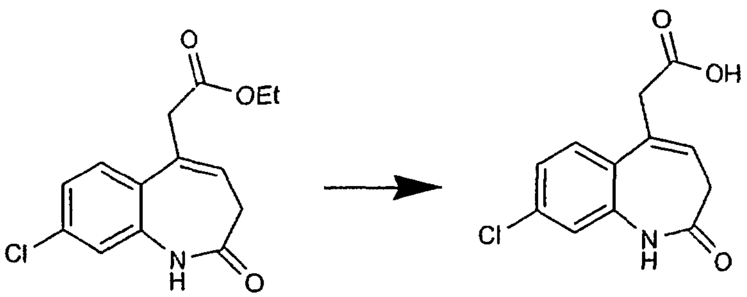

4.23 g (15 mmol) 2'-(8-Chlor-2-oxo-2,3-dihydro-1H-benzo[b]azepin-5-yl)essigsäureethylester wurde mit 5%-iger wäßriger KOH innerhalb von 4h bei Raumtemperatur verseift. Nach dem Ansäuern der Lösung fiel 2'-(8-Chlor-2-oxo-2,3-dihydro-1H-benzo[b]azepin-5-yl)essigsäure als hellgelber Niederschlag aus. Der Niederschlag wurde abgetrennt, mit Wasser und Diethylether gewaschen und getrocknet. Die Ausbeute betrug 2.27 g (60%).

**Beispiel 2:**

**Synthese von 2'-(8-Chlor-1-methyl-2-oxo-2,3-dihydro-1H-benzo[b]azepin-5-yl)essigsäure**

Die Darstellung der Verbindung erfolgte analog zu Beispiel 1 mit 2-(N-Methylamino)-4-chlorbenzoesäuremethylester als Edukt.

**Beispiel 3:**

**Synthese von 8-Chlor-4-(N,N-dimethylaminomethyl)-2,3,4,5-tetrahydro-1H-benzo[b]azepin-2,5-dion-Hydrochlorid**

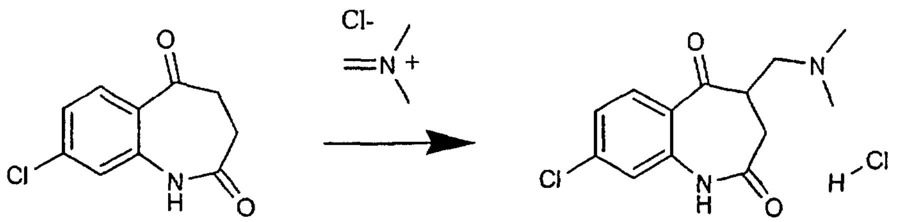

8-Chlor-2,3,4,5-tetrahydro-1H-benzo[b]azepin-2,5-dion wurde mit N,N-Dimethylaminomethylen-Hydrochlorid in Acetonitril unter Säurekatalyse mit Acetylchlorid 5h bei 20°C umgesetzt. Die Ausbeute an 8-Chloro-4-(N,N-dimethylaminomethyl)-2,3,4,5-tetrahydro-1H-benzo[b]azepin-2,5-dion-Hydrochlorid betrug 85%. Die Verbindung fiel als gelbe, feinkristalline Substanz an.

**Beispiel 4:**

**Synthese von 2'-(8-Chlor-1-methyl-2-oxo-2,3-dihydro-1H-benzo[b]azepin-5-yl)essigsäure-[3"-(N,N-dimethylaminomethyl)-4"-hydroxy-4"-(m-methoxyphenyl)cyclohexyl]ester**

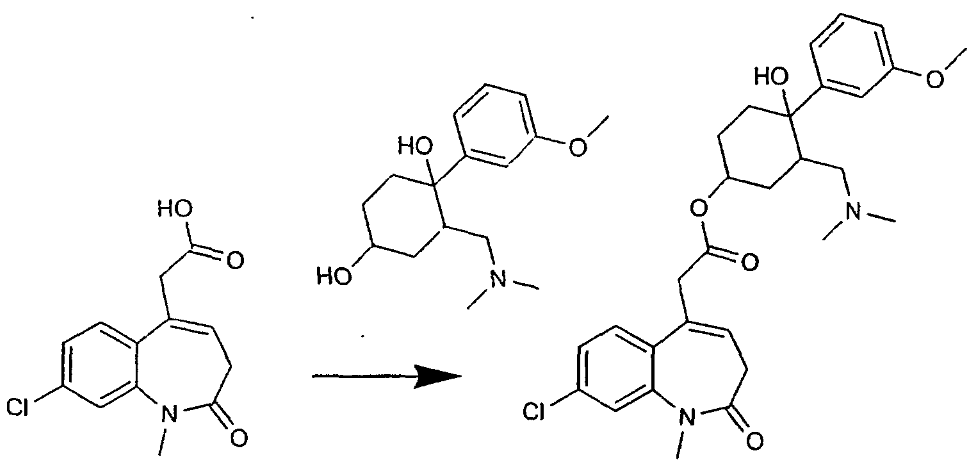

2'-(8-Chlor-1-methyl-2-oxo-2,3-dihydro-1H-benzo[b]azepin-5-yl)-O-[3"-(N,N-dimethylaminomethyl)-4"-hydroxy-4"-(m-methoxyphenyl)cyclohexyl]acetat wurde durch Veresterung von 352 mg (1.32 mmol) 2'-(8-Chlor-1-methyl-2-oxo-2,3-dihydro-1H-benzo[b]azepin-5-yl)essigsäure mit 370 mg (1.32 mmol) 2-(N,N-Dimethylaminomethyl)-1-(m-methoxyphenyl)cyclohexan-1,4-diol in Anwesenheit von 82 mg (1.0 mmol) 1-Methylimidazol und 340 mg (1.32 mmol) 1-(Mesitylen-2'-sulfonyl)-3-nitro-1,2,4-triazol in Methylenchlorid innerhalb von 48 h bei 20°C gewonnen. Das Reaktionsprodukt wurde mittels Säulenchromatographie (Chloroform/Methanol, 95:5) gereinigt. Das Produkt wurde als eine farblose Substanz mit einer Ausbeute von 378 mg (54%) erhalten.

**Beispiel 5:**

**Synthese von 2'-(8-Chlor-2-oxo-2,3-dihydro-1H-benzo[b]azepin-5-yl)essigsäure-[3"-(N,N-dimethylaminomethyl)-4"-hydroxy-4"-(m-methoxyphenyl)cyclohexyl]ester**

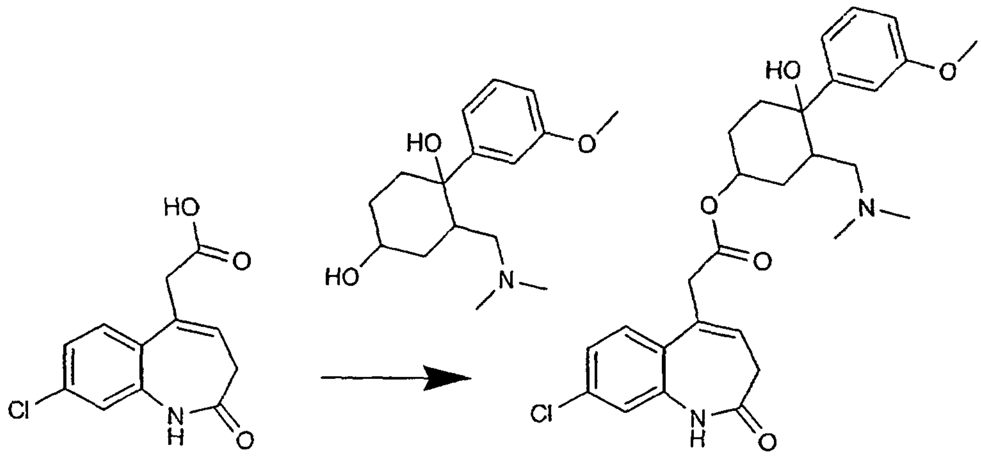

2'-(8-Chlor- 2-oxo- 2,3-dihydro- 1H-benzo [b] azepin- 5-yl) -O-[3"-(N, N-dimethylaminomethyl)- 4"-hydroxy-4"-(m-methoxyphenyl)cyclohexyl]acetat wurde analog zu **Beispiel 4** aus 2-(N,N-Dimethylaminomethyl)-1-(m-methoxyphenyl)cyclohexan-1,4-diol und 2'-(8-Chlor-2-oxo-2,3-dihydro-1H-benzo[b]azepin-5-yl)essigsäure dargestellt. Die Ausbeute betrug 503 mg (65%). Die Verbindung hatte einen Schmelzpunkt von 105-110°C.

**Beispiel 6:**

**Synthese von 2'-(8-Chlor-1-methyl-2-oxo-2,3-dihydro-1H-benzo[b]azepin-5-yl)-N-[3"-(N,N-dimethylaminomethyl)-4"-hydroxy-4"-(m-methoxyphenyl)cyclohexyl]acetamid**

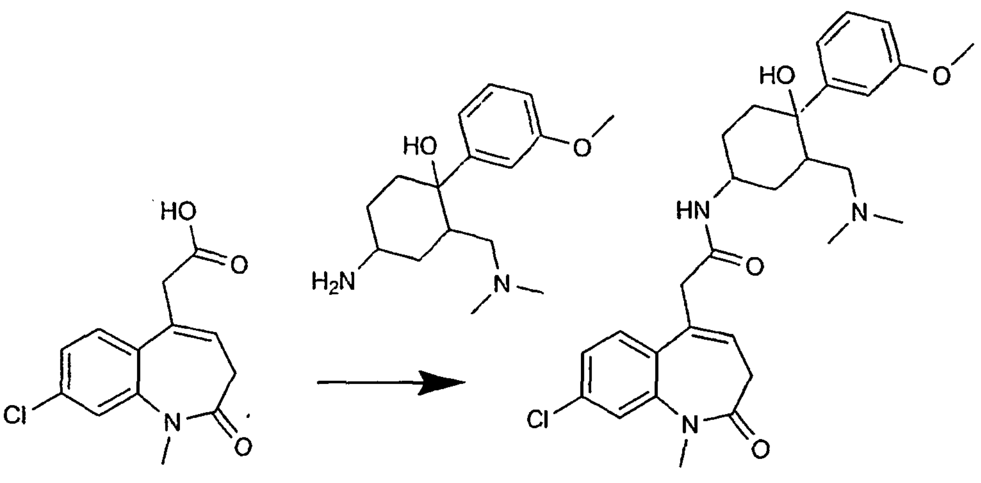

314 mg (1.13 mmol) 4-Amino-2-(N,N-dimethylaminomethyl)-1-(m-methoxyphenyl)-cyclohexan-1-ol und 300

mg (1.13 mmol) 2'-(8-Chlor-1-methyl-2-oxo-2,3-dihydro-1H-benzo[b]azepin-5-yl)essigsäure wurden in 15 ml DMF in Gegenwart von 443 mg (2.14 mmol) Dicyclohexylcarbodiimid, 217 ml (2.14 mmol) N-Methylmorpholin und 290 mg (2.14 mmol) 1-Hydroxybenzotriazol zum Amid umgesetzt. Das Produkt wurde säulenchromatographisch gereinigt (Essigester/Methanol/Essigsäure, 60:38:2). Die Ausbeute betrug 443 mg (75%).

**Beispiel 7:**

**Synthese von 2'-(8-Chlor-2-oxo-2,3-dihydro-1H-benzo[b]azepin-5-yl)-N-[3"-(N,N-dimethylaminomethyl)-4"-hydroxy-4"-(m-methoxyphenyl)cyclohexyl]acetamid**

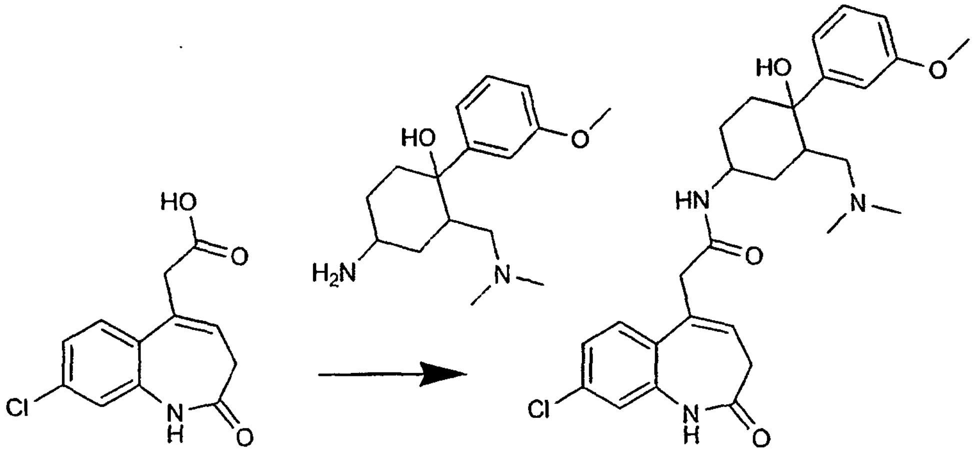

2'-(8-Chlor- 2-oxo- 2,3-dihydro- 1H-benzo [b] azepin- 5-yl) -N-[3"-(N, N-dimethylaminomethyl)- 4"-hydroxy-4"-(m-methoxyphenyl)cyclohexyl]acetamid wurde analog zu **Beispiel 6** aus 4-Amino-2-(N,N-dimethylaminomethyl)-1-(m-methoxyphenyl)cyclohexan-1-ol und 2'-(8-Chlor-2-oxo-2,3-dihydro-1H-benzo[b]azepin-5-yl)essigsäure hergestellt. Die Ausbeute betrug 40%. Das Amid hatte einen Schmelzpunkt von 205-210°C.

**Pharmakologische Untersuchungen**

Analgesieprüfung im Writhing-Test an der Maus:

Die vertiefte Untersuchung auf analgetische Wirksamkeit wurde nach obenstehend beschriebenen Phenylchinon-induzierten Writhing an der Maus durchgeführt.

Die untersuchten erfindungsgemäßen Verbindungen zeigten eine analgetische Wirkung. Die Ergebnisse ausgewählter Writhing-Untersuchungen sind in der nachfolgenden Tabelle 1 zusammengefaßt.

**Tabelle 1:**

| Beispiel Nr. | % Hemmung der Writhing Reaktionen 10 mg/kg i.v. |
|---|---|
| 4 | 39 |
| 5 | 63 |
| 6 | 25 |

**Patentansprüche**

1. Substituierte Benzo[b]azepin-2-on-Verbindungen der allgemeinen Formeln I und II und jeweils deren Tautomere,

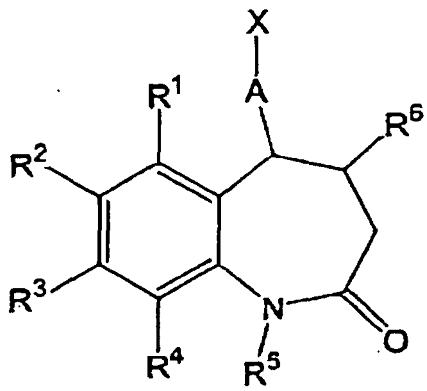

I

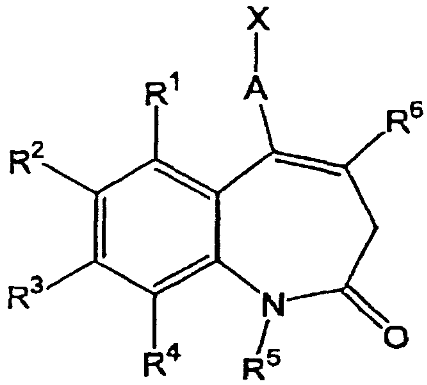

II

worin
$R^1$, $R^2$, $R^3$ und $R^4$, gleich oder verschieden, für einen linearen oder verzweigten, gesättigten oder ungesättigten aliphatischen $C_{1-10}$-Rest oder einen gesättigten oder ungesättigten cycloaliphatischen $C_{3-7}$-Rest, wobei jeder der vorstehend genannten Reste gegebenenfalls über eine Etherbrücke gebunden sein kann, oder Wasserstoff, ein Halogen oder eine Hydroxygruppe stehen,
$R^5$ für Wasserstoff, einen linearen oder verzweigten, gesättigten oder ungesättigten aliphatischen $C_{1-10}$-Rest, einen Aryl- oder einen Heteroarylrest steht,
$R^6$ für Wasserstoff oder einen Rest der Formel $-CH_2-NR^7{}_2$ steht, wobei die beiden Reste gleich oder verschieden sind und die nachstehende Bedeutung haben oder gemeinsam mit dem sie verbindenden Stickstoffatom als Ringglied einen 3-8-gliedrigen Ring bilden können,
$R^7$ für einen linearen oder verzweigten, gesättigten oder ungesättigten aliphatischen $C_{1-8}$-Rest oder einen gesättigten oder ungesättigten cycloaliphatischen $C_{3-6}$-Rest steht,
A für eine Brücke mit einer der folgenden Formeln steht: $-(CH_2)_{n+2}-$, $-(CH_2)_n-CH=CH-$, $-(CH_2)_nCOO-$, $-(CH_2)_nCONH-$, $-(CH_2)_{n+1}O(CH_2)_pCO-$, $-(CH_2)_{n+1}O-$, $-(CH_2)_{n+1}NR^{1'}-$ steht, worin n für 0,1,2,3 und p für 0 oder 1 steht, $R^{1'}$ die nachstehend angegebene Bedeutung hat und die Bindung zum Rest X immer zuletzt genannt ist,
und X für einen der folgenden Reste der allgemeinen Formeln $X^1$ bis $X^6$ und $X^{16}$ steht, worin der freie Strich die Bindung zur Brücke A symbolisiert und

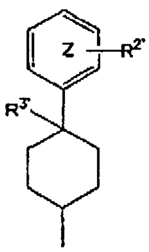

$X^1$

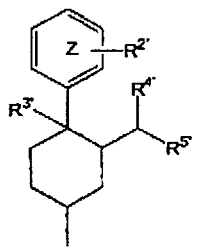

$X^2$

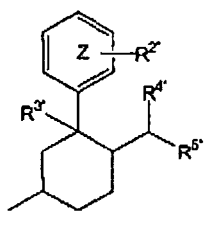

$X^3$

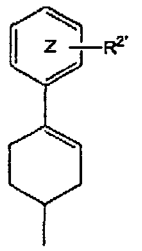

$X^4$

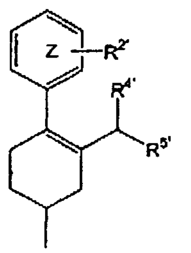

$X^5$

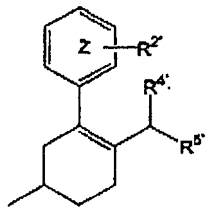

$X^6$

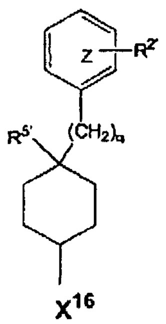

worin
$R^{1'}$ für Wasserstoff, einen linearen oder verzweigten, gesättigten oder ungesättigten aliphatischen $C_{1-10}$-Rest, einen gesättigten oder ungesättigten cycloaliphatischen $C_{3-7}$-Rest, einen Aryl- oder Heteroarylrest steht,
$R^{2'}$ für einen linearen oder verzweigten, gesättigten oder ungesättigten aliphatischen $C_{1-10}$-Rest, einen gesättigten oder ungesättigten cycloaliphatischen $C_{3-7}$-Rest oder einen Aryl- oder Heteroarylrest, wobei alle vorstehend genannten Reste gegebenenfalls über eine Ether-, Thioether- oder $SO_2$-Brücke gebunden sein können, oder Wasserstoff, ein Halogen, eine Hydroxy-, Thiol-, Cyano- oder Nitrogruppe oder eine Gruppe der Formel $-NR^{1'}_2$ steht, wobei die beiden Reste $R^{1'}$ gleich oder verschieden sind und die vorstehend angegebene Bedeutung haben,
$R^{3'}$ für einen linearen oder verzweigten, gesättigten oder ungesättigten aliphatischen $C_{1-10}$-Rest, einen gesättigten oder ungesättigten cycloaliphatischen $C_{3-7}$-Rest, einen Aryl- oder Hoteruarylrest, wobei alle vorstehend genannten Reste gegebenenfalls über eine Ether- oder eine Esterbrücke gebunden sein können, Wasserstoff, ein Halogen, eine Hydroxygruppe steht,
$R^{4'}$ für Wasserstoff, einen Aryl- oder Heteroarylrest steht, wobei der Aryl- oder Heteroarylrest mindestens einen Substituenten $R^{2'}$ mit der vorstehenden Bedeutung mit Ausnahme von Wasserstoff aufweisen kann,
$R^{5'}$ für einen Rest der Formel $-NR^{6'}_2$ steht, wobei die beiden Reste $R^{6'}$ gleich oder verschieden sein können und die nachstehende Bedeutung haben oder gemeinsam mit dem sie verbindenden Stickstoffatom als Ringglied einen 3-7-gliedrigen Ring bilden können, der gegebenenfalls mindestens einen Sauerstoff und/oder mindestens einen weiteren Stickstoff als Ringatom enthalten kann, wobei der Stickstoff einen Substituenten $R^{10'}$ mit der nachstehenden Bedeutung aufweisen kann,
$R^{6'}$ für einen linearen oder verzweigten, gesättigten oder ungesättigten aliphatischen $C_{1-6}$-Rest, einen gesättigten oder ungesättigten cycloaliphatischen $C_{3-7}$-Rest, einen Aryl- oder Heteroarylrest steht,
$R^{10'}$ für Wasserstoff, einen linearen oder verzweigten, gesättigten oder ungesättigten aliphatischen $C_{1-10}$-Rest, einen Aryl- oder Heteroarylrest steht und
Z für mindestens einen gegebenenfalls vorhandenen Sauerstoff, Schwefel oder Stickstoff als Ringatom steht,
sowie q für 0,1,2.3 steht,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

2. Substituierte Benzo[b]azepin-2-on-Verbindungen und jeweils deren Tautomeren gemäß Anspruch 1, **dadurch gekennzeichnet, daß** $R^2$ und $R^3$, gleich oder verschieden, für einen linearen oder verzweigten, gesättigten oder ungesättigten aliphatischen $C_{1-3}$-Rest oder ein Halogen und $R^1$ und $R^4$ jeweils für Wasserstoff, $R^5$ für Wasserstoff oder einen linearen oder verzweigten, gesättigten oder ungesättigten aliphatischen $C_{1-3}$-Rest und $R^6$ für Wasserstoff oder einen Rest der Formel $-CH_2-NR^7_2$ stehen, worin $R^7$ für einen linearen oder verzweigten, gesättigten oder ungesättigten aliphatischen $C_{1-3}$-Rest steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

3. Substituierte Benzo[b]azepin-2-on-Verbindungen und jeweils deren Tautomeren gemäß Anspruch 1, **dadurch gekennzeichnet, daß** $R^2$ und $R^3$ jeweils für eine Methylgruppe oder ein Chlor und $R^1$ und $R^4$ jeweils für Wasserstoff, $R^5$ für Wasserstoff oder eine Methylgruppe und $R^6$ für Wasserstoff oder einen Rest der Formel $-CH_2-N(CH_3)_2$ stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

**4.** Substituierte Benzo[b]azepin-2-on-Verbindungen und jeweils deren Tautomeren gemäß Anspruch 1, **dadurch gekennzeichnet, daß** $R^3$ für einen linearen oder verzweigten, gesättigten oder ungesättigten aliphatischen $C_{1-3}$-Rest oder ein Halogen und $R^1$, $R^2$ und $R^4$ jeweils für Wasserstoff, $R^5$ für Wasserstoff oder einen linearen oder verzweigten, gesättigten oder ungesättigten aliphatischen $C_{1-3}$-Rest und $R^6$ für Wasserstoff oder einen Rest der Formel $-CH_2-N(R^7)_2$ stehen, worin $R^7$ für einen linearen oder verzweigten, gesättigten oder ungesättigten aliphatischen $C_{1-3}$-Rest steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

**5.** Substituierte Benzo[b]azepin-2-on-Verbindungen und jeweils deren Tautomeren gemäß Anspruch 1, **dadurch gekennzeichnet, daß** $R^3$ für eine Methylgruppe oder ein Chlor und $R^1$, $R^2$ und $R^4$ jeweils für Wasserstoff, $R^5$ für Wasserstoff oder eine Methylgruppe und $R^6$ für Wasserstoff oder einen Rest der Formel $-CH_2-N(CH_3)_2$ stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

**6.** Substituierte Benzo[b]azepin-2-on-Verbindungen und jeweils deren Tautomeren gemäß Anspruch 1, **dadurch gekennzeichnet, daß** $R^1$ und $R^3$, gleich oder verschieden, für einen linearen oder verzweigten, gesättigten oder ungesättigten aliphatischen $C_{1-3}$-Rest oder ein Halogen und $R^2$ und $R^4$ jeweils für Wasserstoff, $R^5$ für Wasserstoff oder einen linearen oder verzweigten, gesättigten oder ungesättigten aliphatischen $C_{1-3}$-Rest und $R^6$ für Wasserstoff oder einen Rest der Formel $-CH_2-NR^7{}_2$ stehen, worin $R^7$ für einen linearen oder verzweigten, gesättigten oder ungesättigten aliphatischen $C_{1-3}$-Rest steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

**7.** Substituierte Benzo[b]azepin-2-on-Verbindungen und jeweils deren Tautomeren gemäß Anspruch 1, **dadurch gekennzeichnet, daß** $R^1$ und $R^3$ jeweils für eine Methylgruppe oder ein Chlor und $R^2$ und $R^4$ jeweils für Wasserstoff, $R^5$ für Wasserstoff oder eine Methylgruppe und $R^6$ für Wasserstoff oder einen Rest der Formel $-CH_2-N(CH_3)_2$ stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

**8.** Substituierte Benzo[b]azepin-2-on-Verbindungen und jeweils deren Tautomeren gemäß einem der Ansprüche 1-7, **dadurch gekennzeichnet, daß** A für eine Brücke der Formeln $-CH_2-COO-$ oder $-CH_2-CONH-$ steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

**9.** Substituierte Benzo[b]azepin-2-on-Verbindungen und jeweils deren Tautomeren gemäß einem der Ansprüche 1-8, **dadurch gekennzeichnet, daß** X für einen Rest der folgenden Formel steht:

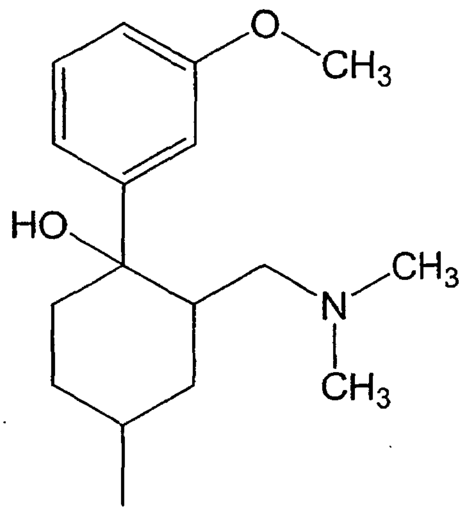

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

**10.** Substituierte Benzo[b]azepin-2-on-Verbindungen und deren Tautomeren gemäß Anspruch 1:

2'-(8-Chlor-2-oxo-2,3-dihydro-1H-benzo[b]azepin-5-yl)essigsäure-[3"-(N,N-dimethylaminomethyl)-4"-hydroxy-4"-(m-methoxyphenyl)-cyclohexyl]ester,
2'-(8-Chlor-1-methyl-2-oxo-2,3-dihydro-1H-benzo[b]azepin-5-yl)essigsäure-[3"-(N,N-dimethylaminomethyl)-4"-hydroxy-4"-(m-methoxyphenyl)cyclohexyl]ester,
2'-(8-Chlor- 2-oxo- 2,3-dihydro- 1H-benzo [b] azepin- 5-yl) -N-[3"-(N, N-dimethylaminomethyl)- 4"-hydroxy-4"-(m-methoxyphenyl)cyclohexyl]acetamid,
2'-(8-Chlor-1-methyl-2-oxo-2,3-dihydro-1H-benzo[b]azepin-5-yl)-N-[3"-(N,N-dimethylaminomethyl)-4"-hydroxy-4"-(m-methoxyphenyl)-cyclohexyl]acetamid

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diasteromeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

**11.** Verfahren zur Herstellung von substituierten Benzo[b]azepin-2-on-Verbindungen, deren Tautomeren sowie entsprechenden Stereoisomeren gemäß einem der Ansprüche 1-10, **dadurch gekennzeichnet, daß** man

A) einen gegebenenfalls substituierten 2-Aminobenzoealkylester der allgemeinen Formel (1), worin $R^1$, $R^2$, $R^3$, $R^4$ und $R^5$ dieselbe Bedeutung wie in einem der Ansprüche 1-7 haben und R für eine Alkylgruppe, vorzugsweise eine Methyl- oder Ethylgruppe, steht,

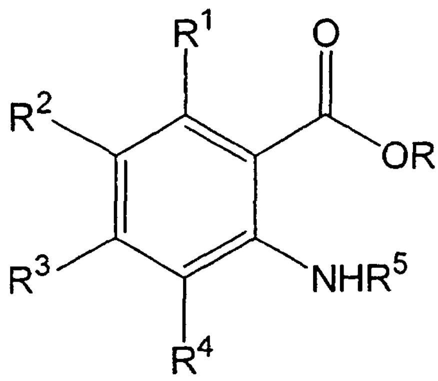

(1)

mit Bernsteinsäuredialkylester der allgemeinen Formel (2), worin R' für eine Alkylgruppe, vorzugsweise eine Methyl- oder Ethylgruppe, und $R^x$ für ein Chlor oder eine Alkoxygruppe, vorzugsweise eine Methoxy- oder Ethoxygruppe, steht,

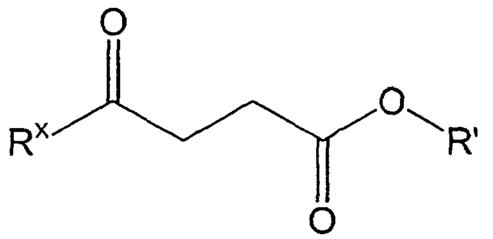

(2)

unter geeigneten Reaktionsbedingungen, in einem geeigneten Lösungsmittel, vorzugsweise Pyridin, umsetzt und anschließend aufarbeitet und gegebenenfalls das gebildete gegebenenfalls substituierte N-(2-Carbalkoxyphenyl)bernsteinsäurealkylesteramid der allgemeinen Formel (3), worin R, R', $R^1$, $R^2$, $R^3$, $R^4$ und $R^5$ die vorstehend genannte Bedeutung haben, reinigt,

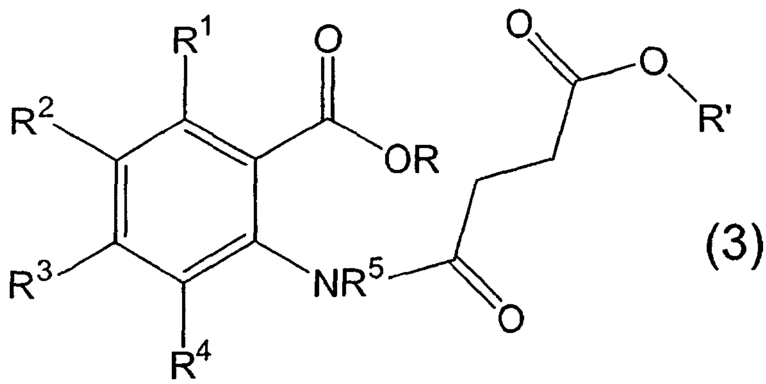

(3)

B) ein gegebenenfalls substituiertes N-(2-Carboalkoxyphenyl)bernsteinsäurealkylesteramid der allgemeinen Formel (3) in Gegenwart von Kalium-tert-butanolat in einem geeigneten Lösungsmittel umsetzt und anschließend aufarbeitet und gegebenenfalls den gebildeten gegebenenfalls substituierten 5-Hydroxy-2-oxo-2,3-dihydro-1H-benzo[b]azepin-4-carbonsäurealkylester der allgemeinen Formel (4), worin R', $R^1$, $R^2$, $R^3$, $R^4$ und $R^5$ die vorstehend genannte Bedeutung haben, reinigt,

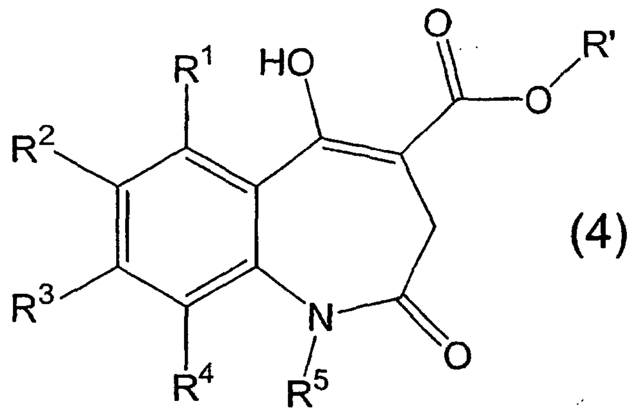

(4)

C) einen gegebenenfalls substituierten 5-Hydroxy-2-oxo-2,3-dihydro-1 H-benzo[b]azepin-4-carbonsäurealkylester der allgemeinen Formel (4) in einem Dimethylsulfoxid/Wassergemisch bei erhöhter Temperatur umsetzt und anschließend aufarbeitet und gegebenenfalls das gebildete gegebenenfalls substituierte 2,3,4,5-Tetrahydro-1H-benzo[b]azepin-2,5-dion der allgemeinen Formel (5), worin $R^1$, $R^2$, $R^3$, $R^4$ und $R^5$ die vorstehend genannte Bedeutung haben, reinigt,

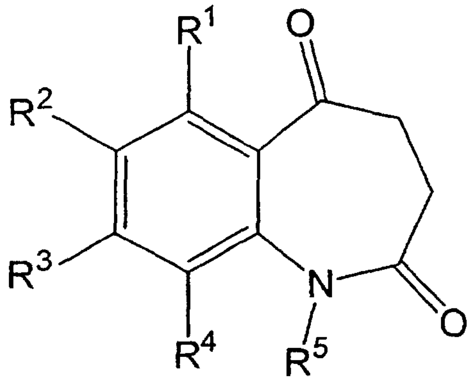

(5)

D) gegebenenfalls ein gegebenenfalls substituiertes 2,3,4,5-Tetrahydro-1H-benzo[b]azepin-2,5-dion der allgemeinen Formel (5) mit einem substituierten Aminomethylen-Hydrochlorid der allgemeinen Formel (6), worin der Rest $R^7$ die in Anspruch 1 genannte Bedeutung hat,

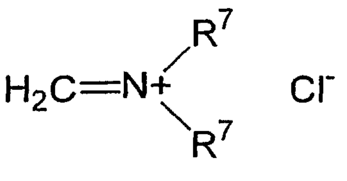

(6)

in Gegenwart einer Säure, vorzugsweise Acetylchlorid, in einem geeigneten Lösungsmittel, vorzugsweise Acetonitril, umsetzt und anschließend aufarbeitet und gegebenenfalls das gebildete gegebenenfalls substituierte Aminomethyl-2,3,4,5-tetrahydro-1 H-benzo[b]azepin-2,5-dion der allgemeinen Formel (7), worin $R^1$, $R^2$, $R^3$, $R^4$, $R^5$ und $R^7$ die vorstehend genannte Bedeutung haben, reinigt,

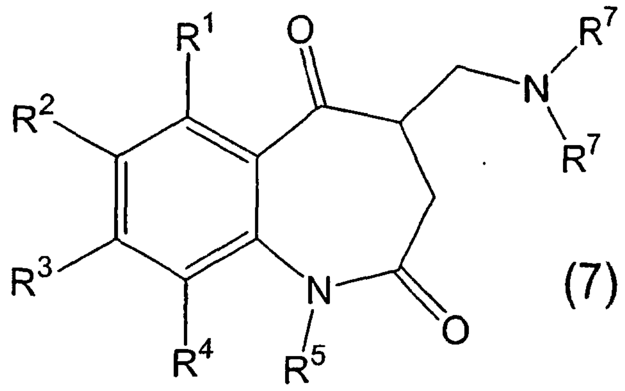

(7)

E) ein gegebenenfalls substituiertes 2,3,4,5-Tetrahydro-1H-benzo[b]azepin-2,5-dion der allgemeinen Formel (8), worin $R^1$, $R^2$, $R^3$, $R^4$, $R^5$ und $R^6$ dieselbe Bedeutung wie in einem der Ansprüche 1-7 haben und die die Verbindungen der allgemeinen Formeln (5) und (7) zusammenfaßt,

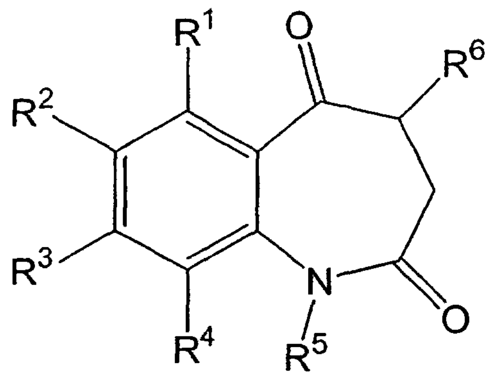

(8)

mit einem Phosphonoalkansäuretrialkylester der allgemeinen Formel (9), worin n dieselbe Bedeutung wie in Anspruch 1 hat und R" für eine Alkylgruppe, vorzugsweise eine Methyl- oder Ethylgruppe, steht,

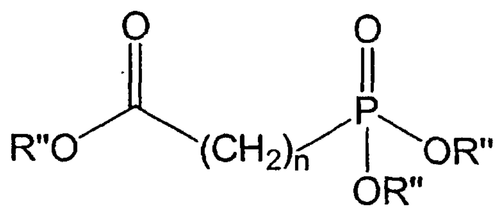

(9)

in Gegenwart einer Base, vorzugsweise Kalium-tert-butanolat, in einem geeigneten Lösungsmittel, vorzugsweise Dimethylformamid, umsetzt und anschließend aufarbeitet und gegebenenfalls die gebildete Verbindung der Formel Y-COOR", worin R" die vorstehend genannte Bedeutung hat und Y für einen Rest der allgemeinen Formel Y steht, worin der freie Strich die Bindung zum Rest -COOR" symbolisiert und

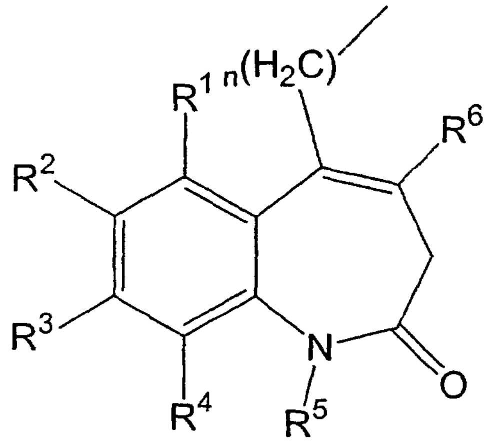

Y

worin $R^1$, $R^2$, $R^3$, $R^4$, $R^5$, $R^6$ und n die vorstehend genannte Bedeutung haben, reinigt,
F) gegebenenfalls einen Ester der Formel Y-COOR" in Gegenwart einer Base, vorzugsweise Natrium- oder Kaliumhydroxid, in einem geeigneten Lösungsmittel, vorzugsweise einem Alkohol/Wassergemisch, umsetzt und anschließend aufarbeitet und gegebenenfalls die gebildete Carbonsäure der Formel Y-COOH, worin Y die vorstehend genannte Bedeutung hat, reinigt,
G) gegebenenfalls eine Carbonsäure der Formel Y-COOH oder einen Carbonsäureester der Formeln Y-COOR", worin Y und R" die vorstehend genannte Bedeutung haben, derivatisiert, **dadurch** daß man

a) eine Carbonsäure oder einen Carbonsäureester der Formel Y-COOH oder Y-COOR" mit Hilfe von Reduktionsmitteln, vorzugsweise Lithiumaluminiumhydrid, in einem geeigneten Lösungsmittel, vorzugsweise Tetrahydrofuran, zu dem entsprechenden Alkohol der Formel Y-$CH_2$-OH reduziert,
b) eine Carbonsäure oder einen Carbonsäureester der Formel Y-COOH oder Y-COOR" mit Hilfe von Reduktionsmitteln, vorzugsweise Diisobutylaluminiumhydrid, in einem geeigneten Lösungsmittel, vorzugsweise Hexan, zu dem entsprechenden Aldehyd der Formel Y-CHO reduziert oder
c) einen Alkohol der Formel Y-$CH_2$-OH gemäß a) mit einem Bromierungsmittel, vorzugsweise $PBr_3$ oder $Ph_3PBr_2$, zu dem entsprechenden Bromid der Formel Y-$CH_2$-Br umsetzt

und anschließend aufarbeitet und gegebenenfalls das Produkt reinigt,
H) gegebenenfalls eine Verbindung der Formel $X^1$-$R^{IV}$, worin $X^1$ die vorstehend genannte Bedeutung hat und $R^{IV}$ für eine funktionelle Gruppe steht, herstellt, **dadurch** daß man

a) 1,4-Cyclohexandionmonoethylenketal, 4-Aminocyclohexan-1-onethylenketal oder 4-Oxocyclohexancarbonsäure mit Magnesium und einem bromierten oder chlorierten, gegebenenfalls substituierten Aromaten oder Heteroaromaten in einem geeigneten Lösungsmittel, vorzugsweise trockenen Diethylether, bei erhöhter Temperatur zu dem entsprechenden Kupplungsprodukt umsetzt und anschließend gegebenenfalls das Ketal durch Umsetzung mit Salzsäure in einem geeigneten Lösungsmittel, vorzugsweise Tetrahydrofuran, spaltet, aufarbeitet und gegebenenfalls das Produkt der Formel $X^{1a}$=O, $X^{1a}$-$NHR^{1'}$ oder $X^{1a}$-$CO_2H$, worin $X^{1a}$ für einen Rest der Formel $X^{1a}$ steht und $R^{1'}$, $R^{2'}$ und Z die vorstehend genannte Bedeutung haben und der freie Strich die Bindung zum Rest =O, -$NHR^{1'}$ oder -$CO_2H$ symbolisiert, reinigt,

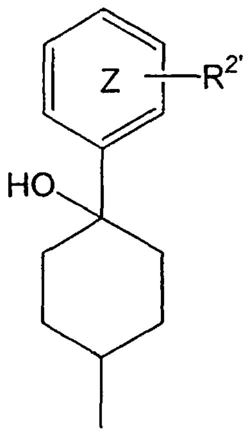

$X^{1a}$

b) gegebenenfalls ein Keton der Formel $X^{1a}$=O in Gegenwart eines geeigneten Reduktionsmittels, vorzugsweise Natriumborhydrid, in einem geeigneten Lösungsmittel, vorzugsweise Methanol, zu dem entsprechenden Alkohol der Formel $X^{1a}$-OH umsetzt, aufarbeitet und gegebenenfalls das Produkt reinigt,
c) gegebenenfalls ein Keton der Formel $X^{1a}$=O unter Stickstoff in einem geeigneten Lösungsmittel, vorzugsweise Tetrahydrofuran, zunächst mit Ammoniumtrifluoracetat und anschließend mit Eisessig und Natriumtriacetoxyborhydrid zu dem entsprechenden Amin der Formel $X^{1a}$-$NH_2$ umsetzt, aufarbeitet und gegebenenfalls das Produkt reinigt,
d) gegebenenfalls eine Carbonsäure der Formel $X^{1a}$-$CO_2H$ durch Umsetzung mit Dicydohexylcarbodiimid oder durch Überführen in das Carbonsäurechlorid oder ein gemischtes Anhydrid aktiviert, mit Diazomethan in einem geeigneten Lösungsmittel, vorzugsweise Ether, umsetzt und anschließend mit Wasser behandelt, aufarbeitet und gegebenenfalls das Produkt der Formel $X^{1a}$-CO-$CH_2$-OH reinigt,
e) gegebenenfalls die Hydroxygruppe in 4-Position des Cyclohexanringes in dem Rest $X^{1a}$ in Wasserstoff, ein Halogen, eine Ether-, Ester-, Alkyl-, Aryl- oder Heteroarylgruppe umwandelt, **dadurch** daß man

α) eine Verbindung aus einem der Schritte a)-d) zur Einführung einer Ethergruppe mit einer aliphatischen oder cycloaliphatischen Verbindung in Gegenwart eines geeigneten Katalysators in einem geeigneten Lösungsmittel, vorzugsweise in Gegenwart von Natriumhydrid in Dimethylformamid oder in Gegenwart von Kaliumhydroxid in Dimethylsulfoxid, oder mit einem Alkylierungsmittel in einem geeigneten Lösungsmittel, vorzugsweise mit einer Diazoverbindung in Diethylether, oder mit einer Aryl- oder Heteroarylverbindung in Gegenwart von Diethylazodicarboxylat und Triphenylphosphin umsetzt,
β) eine Verbindung aus einem der Schritte a)-d) zur Einführung eines Halogens mit einem Halogenierungsmittel in einem geeigneten Lösungsmittel, vorzugsweise mit $POCl_3$ in Dimethylformamid, mit $PPh_3/Cl_2$, mit $PPh_3/Br_2$, mit Triphenylphosphin/n-Chlorsuccinimid oder mit $HCl/ZnCl_2$, umsetzt,
γ) eine Verbindung aus Schritt β) zur Einführung eines Wasserstoffs mit Wasserstoff in Gegenwart eines geeigneten Katalysators, vorzugsweise Palladium/Kohle, in einem geeigneten Lösungsmittel umsetzt,
δ) eine Verbindung aus Schritt β) zur Einführung eines aliphatischen oder cycloaliphatischen Restes oder einer Aryl- oder Heteroarylgruppe mit einer aliphatischen oder cycloaliphatischen Boronsäure bzw. einem Boronsäureester oder einer Aryl- oder Heteroarylbordihydroxid-Verbindung in Gegenwart von Palladium(II)acetat und Kaliumcarbonat in einem geeigneten Lösungsmittel, vorzugsweise einem Dimethylformamid/Wasser-Gemisch, umsetzt oder
ε) eine Verbindung aus einem der Schritte a)-d) zur Einführung einer Estergruppe mit einemCarbonsäurechlorid in Gegenwart eines geeigneten Katalysators in einem geeigneten Lösungsmittel umsetzt und anschließend aufarbeitet und gegebenenfalls die gebildete Verbindung der Formel $X^1$-$R^{IV}$, worin $X^1$ für die Formel $X^1$ steht

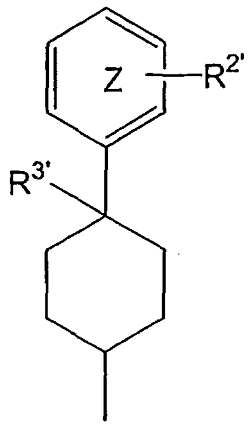

$X^1$

und $R^{IV}$, $R^{2'}$ und $R^{3'}$ die vorstehend genannte Bedeutung haben, reinigt,

I) gegebenenfalls eine Verbindung der Formel X-$R^{IV}$, worin X die vorstehend genannte Bedeutung hat und $R^{IV}$ für eine funktionelle Gruppe steht, derivatisiert, **dadurch** daß man

a) ein Keton der Formel X=O 1) mit Methoxymethyltriphenylphosphiniumchlorid unter Schutzgas in einem geeigneten Lösungsmittel, vorzugsweise in Dimethylformamid, in Gegenwart von Natriumhydrid und anschließend mit Salzsäure oder 2) mit $Me_3S^+BF_4^-$ zu dem entsprechenden, um ein Kohlenstoffatom verlängerten Aldehyd X-CHO umsetzt,
b) einen Aldehyd der Formel X-CHO gemäß a) mit einem Reduktionsmittel, vorzugsweise Natriumborhydrid, in einem geeigneten Lösungsmittel, vorzugsweise einem Ethanol/Wasser-Gemisch zu dem entsprechenden Alkohol X-$CH_2$-OH umsetzt,
c) einen Alkohol X-$CH_2$-OH gemäß b) oder der Formel X-OH mit einem Bromierungsmittel, vorzugsweise Triphenylphosphindibromid, in einem geeigneten Lösungsmittel, vorzugsweise Acetonitril, zu dem entsprechenden Bromid der Formel X-$CH_2$-Br bzw. X-Br umsetzt,
d) ein Bromid der Formel X-$CH_2$-Br gemäß c) mit einem Phosphin der Formel $PR^V_3$, worin $R^V$ für einen organischen Rest steht, vorzugsweise für einen Phenylrest, in einem geeigneten Lösungsmittel, vorzugsweise Toluol, Ether, Tetrahydrofuran oder Aceton, unter Kühlung und Schutzgas zu dem entsprechenden Phosphoniumsalz $R^V_3P^+$-$CHX^-$ umsetzt oder
e) ein Bromid der Formel X-$CH_2$-Br gemäß c) mit einem Phosphit der Formel HP(O)$(OR^{VI})_2$, worin $R^{VI}$ für einen organischen Rest steht, bei erhöhter Temperatur, vorzugsweise 200°C, zu dem entsprechenden Phosphonat $(R^{VI}O)_2P(O)$-$CH_2$-X umsetzt

und anschließend aufarbeitet und gegebenenfalls das Produkt reinigt,
J) eine Verbindung aus dem Schritt F) oder G), worin Y die vorstehend genannte Bedeutung hat, mit einer Verbindung der Formel $X^1$-$R^{1V}$ aus Schritt H) oder einer Verbindung X-$R^{IV}$ aus Schritt I), worin X, $X^1$ und $R^{IV}$ die vorstehend genannte Bedeutung haben, umsetzt, **dadurch** daß man

a) eine Carbonsäure der Formel Y-COOH mit einem Amin der Formel X-$NH_2$ in Gegenwart eines geeigneten Kondensationsmittels, vorzugsweise Dicyclohexylcarbodiimid, 1-Hydroxybenzotriazol und N-Methylmorphin, in einem geeigneten Lösungsmittel, vorzugsweise Dimethylformamid, unter Ausbildung einer Amidbrücke umsetzt,
b) eine Carbonsäure der Formel Y-COOH mit einem Alkohol der Formel X-OH in Gegenwart eines geeigneten Kondensationsmittels in einem geeigneten Lösungsmittel unter Ausbildung einer Esterbrücke umsetzt, vorzugsweise erfolgt die Umsetzung in Gegenwart von Methylimidazol und 1-(Mesitylen-2'-sulfonyl)-3-nitro-1,2,4-triazol in Tetrahydrofuran oder in Gegenwart von Dicyclohexylcarbodiimid, 1-Hydroxybenzotriazol und N-Methylmorphin in Dimethylformamid,
c) ein Bromid der Formel Y-$CH_2$-Br mit einer Verbindung der Formel X-CO$(CH_2)_p$-OH, worin p die vorstehend genannte Bedeutung hat, unter Schutzgas in Gegenwart eines geeigneten Katalysators, vorzugsweise Natriumhydrid oder Kaüum-tert-butylat, in einem geeigneten Lösungsmittel, vorzugsweise Dimethylformamid, unter Ausbildung einer Brücke der Formel -CO$(CH_2)_p$-O-$CH_2$- umsetzt,
d) einen Alkohol der Formel Y-$CH_2$-OH mit einem Bromid der Formel X-Br unter Schutzgas in Gegenwart

eines geeigneten Kondensationsmittels, vorzugsweise Natriumhydrid oder Kalium-tert-butylat, in einem geeigneten Lösungsmittel, vorzugsweise Dimethylformamid, unter Ausbildung einer Etherbrücke umsetzt,
e) ein Bromid der Formel Y-$CH_2$-Br mit einem Alkohol der Formel X-OH unter Schutzgas in Gegenwart eines geeigneten Kondensationsmittels, vorzugsweise Natriumhydrid oder Kalium-tert-butylat, in einem geeigneten Lösungsmittel, vorzugsweise Dimethylformamid, unter Ausbildung einer Etherbrücke umsetzt,
f) einen Aldehyd der Formel Y-CHO mit einem Amin der Formel X-$NHR^{1'}$ in Gegenwart eines geeigneten Reduktionsmittels, vorzugsweise Natriumcyanoborhydrid und Natriumtriacetoxyborhydrid, in einem geeigneten Lösungsmittel, vorzugsweise einem Gemisch aus Tetrahydrofuran und 1,2-Dichlorethan, unter Ausbildung einer Aminobrücke umsetzt,
g) einen Aldehyd der Formel Y-CHO mit einem Phosphoniumsalz $R^V{}_3P^+$-$CHX^-$, worin $R^V$ die vorstehend genannte Bedeutung hat, unter Schutzgas in Gegenwart geeigneter Katalysatoren in einem geeigneten Lösungsmittel, vorzugsweise in Gegenwart von Natriummethanolat in einem Gemisch aus Hexan, Diethylether und/oder Diisopropylether oder in Gegenwart von Natriumhydrid, Kalium-tert-butylat oder einem Lithiumamid in Dimethylformamid oder Dimethylsulfoxid, unter Ausbildung einer -CH=CH-Brücke umsetzt oder
h) einen Aldehyd der Formel Y-CHO mit einem Phosphonat der Formel $(R^{VI}O)_2P(O)$-$CH_2$-X, worin $R^{VI}$ die vorstehend genannte Bedeutung hat, unter Schutzgas in Gegenwart geeigneter Katalysatoren, vorzugsweise Natriummethanolat, Natriumhydroxid, Kaliumhydroxid, Natriumhydrid, Kalium-tert-butylat oder einem Lithiumamid, in einem geeigneten Lösungsmittel, vorzugsweise Dimethylformamid, Dimethylsufoxid, Diethylether, Tetrahydrofuran, unter Ausbildung einer -CH=CH-Brücke umsetzt und
i) gegebenenfalls die -CH=CH-Brücke aus dem Schritt g) oder h) durch Wasserstoff, vorzugsweise bei Normaldruck oder erhöhtem Druck bis zu 100 bar, in Gegenwart geeigneter Katalysatoren, vorzugsweise Übergangsmetallen oder Übergangsmetallverbindungen, vorzugsweise Palladium oder seine Salze, Rhodium oder seine Komplexe, in einem geeigneten Lösungsmittel, vorzugsweise Dimethylformamid, Methanol oder Ethanol, bei einer Temperatur zwischen 20 und 100°C unter Ausbildung einer -$CH_2$-$CH_2$-Brücke hydriert

und anschließend aufarbeitet und gegebenenfalls das Produkt reinigt,
K) gegebenenfalls die Doppelbindung im 7-Ring eines der Reaktionsprodukte aus dem Schritt J) durch Wasserstoff, vorzugsweise bei Normaldruck oder erhöhtem Druck bis zu 100 bar, in Gegenwart geeigneter Katalysatoren, vorzugsweise Übergangsmetallen oder Übergangsmetallverbindungen, vorzugsweise Palladium oder seine Salze, Rhodium oder seine Komplexe, in einem geeigneten Lösungsmittel, vorzugsweise Dimethylformamid, Methanol oder Ethanol, bei einer Temperatur zwischen 20 und 100°C hydriert und anschließend aufarbeitet und gegebenenfalls das Produkt reinigt.

**12.** Arzneimittel enthaltend wenigstens eine substituierte Benzo[b]azepin-2-on-Verbindung oder ein entsprechendes Tautomeres, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes oder jeweils in Form ihres Solvates, insbesondere des Hydrates, gemäß einem der Ansprüche 1-10 und gegebenenfalls physiologisch verträgliche Hilfsstoffe.

**13.** Arzneimittel gemäß Anspruch 12 zur Bekämpfung von Schmerz.

**14.** Arzneimittel gemäß Anspruch13 zur Bekämpfung von chronischem Schmerz.

**15.** Arzneimittel gemäß Anspruch13 zur Bekämpfung von neuropathischem Schmerz.

**16.** Arzneimittel gemäß Anspruch 12 zur Behandlung oder Prophylaxe von neurodegenerativen Erkrankungen, vorzugsweise von Morbus Alzheimer, Morbus Parkinson oder Morbus Huntington.

**17.** Arzneimittel gemäß Anspruch 12 zur Behandlung oder Prophylaxe von Schlaganfall.

**18.** Arzneimittel gemäß Anspruch 12 zur Behandlung oder Prophylaxe von cerebraler Ischämie.

**19.** Arzneimittel gemäß Anspruch 12 zur Behandlung oder Prophylaxe von cerebralem Infarkt.

**20.** Arzneimittel gemäß Anspruch 12 zur Behandlung oder Prophylaxe von Hirnödem.

**21.** Arzneimittel gemäß Anspruch 12 zur Behandlung oder Prophylaxe von Unterversorgungszuständen des zentralen Nervensystems, vorzugsweise der Hypoxie oder Anoxie.

**22.** Arzneimittel gemäß Anspruch 12 zur Behandlung oder Prophylaxe von Epilepsie.

**23.** Arzneimittel gemäß Anspruch 12 zur Behandlung oder Prophylaxe von Schizophrenie.

**24.** Arzneimittel gemäß Anspruch 12 zur Behandlung oder Prophylaxe von Psychosen bedingt durch erhöhten Aminosäurespiegel.

**25.** Arzneimittel gemäß Anspruch 12 zur Behandlung oder Prophylaxe von AIDS-Demenz.

**26.** Arzneimittel gemäß Anspruch 12 zur Behandlung oder Prophylaxe des Tourette-Syndroms.

**27.** Arzneimittel gemäß Anspruch 12 zur Behandlung oder Prophylaxe von Encephalomyelitis.

**28.** Arzneimittel gemäß Anspruch 12 zur Behandlung oder Prophylaxe von perinataler Asphyxie.

**29.** Arzneimittel gemäß Anspruch 12 zur Behandlung oder Prophylaxe von Tinnitus.

**30.** Arzneimittel gemäß Anspruch 12 zur Behandlung oder Prophylaxe von Migräne.

**31.** Arzneimittel gemäß Anspruch 12 zur Behandlung oder Prophylaxe von inflammatorischen und/oder allergischen Reaktionen.

**32.** Arzneimittel gemäß Anspruch 12 zur Behandlung oder Prophylaxe von Depressionen.

**33.** Arzneimittel gemäß Anspruch 12 zur Behandlung oder Prophylaxe von seelischen Erkrankungen.

**34.** Arzneimittel gemäß Anspruch 12 zur Behandlung oder Prophylaxe von Harninkontinenz.

**35.** Arzneimittel gemäß Anspruch 12 zur Behandlung oder Prophylaxe von Juckreiz.

**36.** Arzneimittel gemäß Anspruch 12 zur Behandlung oder Prophylaxe von Diarrhoe.

**37.** Arzneimittel gemäß Anspruch 12 zur Anxiolyse.

**38.** Arzneimittel gemäß Anspruch 12 zur Anästhesie.

**39.** Verwendung wenigstens einer substituierten Benzo[b]azepin-2-on-Verbindung oder ihres Tautomeren, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes oder jeweils in Form ihres Solvates, insbesondere des Hydrates, gemäß einem der Ansprüche 1-10 zur Herstellung eines Arzneimittels zur Bekämpfung von Schmerz, vorzugsweise chronischem oder neuropathischem Schmerz.

**40.** Verwendung wenigstens einer substituierten Benzo[b]azepin-2-on-Verbindung oder ihres Tautomeren, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes oder jeweils in Form ihres Solvates, insbesondere des Hydrates, gemäß einem der Ansprüche 1-10 zur Herstellung eines Arzneimittels zur Behandlung oder Prophylaxe von neurodegenerativen Erkrankungen, vorzugsweise von Morbus Alzheimer, Morbus Parkinson oder Morbus Huntington, zur Behandlung oder Prophylaxe von Schlaganfall, cerebraler Ischämie, cerebralem Infarkt, Hirnödem, Unterversorgungszuständen des zentralen Nervensystems, vorzugsweise Hypoxie oder Anoxie, Epilepsie, Schizophrenie,

Psychosen bedingt durch erhöhten Aminosäurespiegel, AIDS-Demenz, Encephalomyelitis, Tourette-Syndrom, perinataler Asphyxie, Tinnitus, Migräne, inflammatorischen und/oder allergischen Reaktionen, Depressionen, seelischen Erkrankungen, Harninkontinenz, Juckreiz oder Diarrhoe oder zur Anxiolyse oder Anästhesie.

## Claims

**1.** Substituted benzo[b]azepin-2-one compounds of the general formulae I and II and in each case the tautomers thereof,

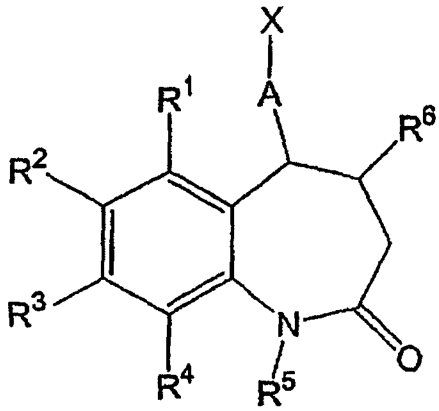

I

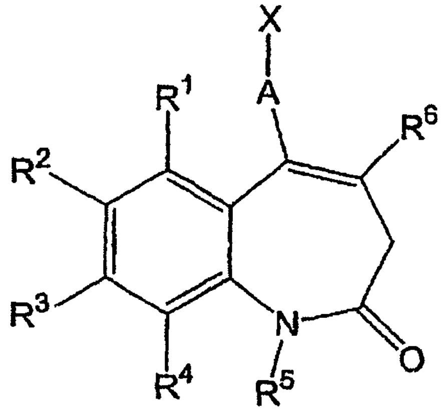

II

in which
$R^1$, $R^2$, $R^3$ and $R^4$, identical or different, denote a linear or branched, saturated or unsaturated aliphatic $C_{1-10}$ residue or a saturated or unsaturated cycloaliphatic $C_{3-7}$ residue, wherein each of the above-stated residues may optionally be joined via an ether bridge, or hydrogen, a halogen or a hydroxy group,
$R^5$ denotes hydrogen, a linear or branched, saturated or unsaturated aliphatic $C_{1-10}$ residue, an aryl or a heteroaryl residue,
$R^6$ denotes hydrogen or a residue of the formula $-CH_2-NR^7{}_2$, wherein the two residues are identical or different and have the meaning stated below or may form a 3-8-membered ring together with the nitrogen atom connecting them as a ring member,
$R^7$ denotes a linear or branched, saturated or unsaturated aliphatic $C_{1-6}$ residue or a saturated or unsaturated cycloaliphatic $C_{3-6}$ residue,
A denotes a bridge with one of the following formulae: $-(CH_2)_{n+2}-$, $-(CH_2)_n-CH=CH-$, $-(CH_2)_nCOO-$, $-(CH_2)_nCONH-$, $-(CH_2)_{n+1}O(CH_2)_pCO-$, $-(CH_2)_{n+1}O-$, $-(CH_2)_{n+1}NR^{1'}-$ in which n denotes 0, 1, 2, or 3, and p denotes 0 or 1, $R^{1'}$ has the meaning stated hereinafter and the bond to the residue X is always stated last,
and X denotes one of the following residues of the general formulae $X^1$ to $X^6$ and $X^{16}$, in which the unoccupied bond line symbolises the bond to the bridge A and

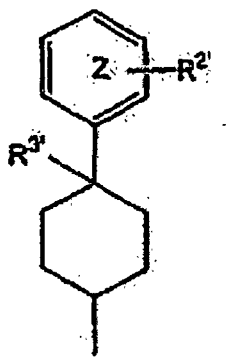

$X^1$

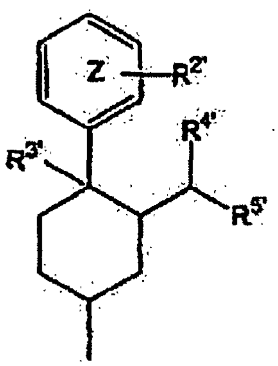

$X^2$

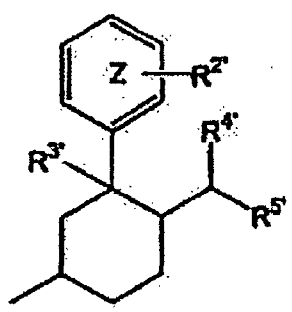

$X^3$

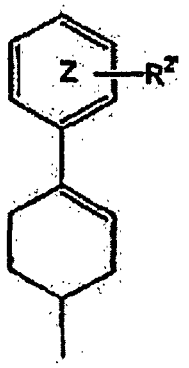

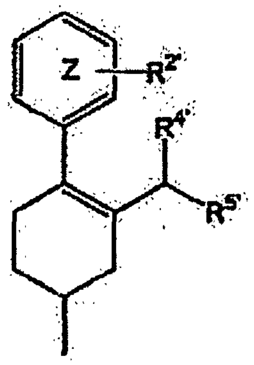

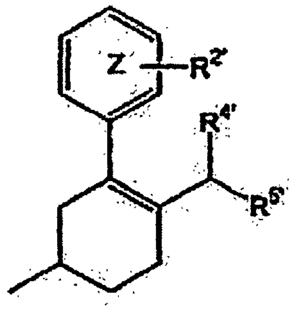

X4 X5 X6

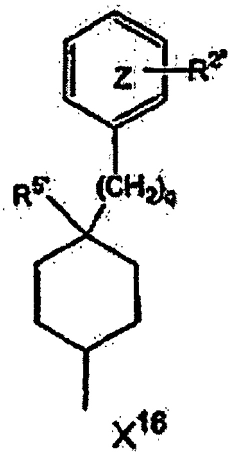

X16

in which

$R^{1'}$ denotes hydrogen, a linear or branched, saturated or unsaturated aliphatic $C_{1-10}$ residue, a saturated or unsaturated cycloaliphatic $C_{3-7}$ residue, an aryl or heteroaryl residue,

$R^{2'}$ denotes a linear or branched, saturated or unsaturated aliphatic $C_{1-10}$ residue, a saturated or unsaturated cycloaliphatic $C_{3-7}$ residue or an aryl or heteroaryl residue wherein all above-stated residues may optionally be joined via an ether, thioether or $SO_2$ bridge, or hydrogen, a halogen, a hydroxy, thiol, cyano or nitro group or a group of the formula $-NR^{1'}_2$, wherein the two residues $R^{1'}$ are identical or different and have the above-stated meaning,

$R^{3'}$ denotes a linear or branched, saturated or unsaturated aliphatic $C_{1-10}$ residue, a saturated or unsaturated cycloaliphatic $C_{3-7}$ residue, an aryl or heteroaryl residue, wherein all the above-stated residues may optionally be joined via an ether or an ester bridge, hydrogen, a halogen, a hydroxy group,

$R^{4'}$ denotes hydrogen, an aryl or heteroaryl residue, wherein the aryl or heteroaryl residue may comprise at least one substituent $R^{2'}$ with the above meaning, with the exception of hydrogen,

$R^{5'}$ denotes a residue of the formula $-NR^{6'}_2$, wherein the two residues $R^{6'}$ may be identical or different and have the meaning stated hereinafter or may form a 3-7-membered ring together with the nitrogen atom connecting them as a ring member, which ring may optionally contain at least one oxygen and/or at least one further nitrogen as a ring atom, wherein the nitrogen may comprise a substituent $R^{10'}$ with the meaning stated hereinafter,

$R^{6'}$ denotes a linear or branched, saturated or unsaturated aliphatic $C_{1-6}$ residue, a saturated or unsaturated or cycloaliphatic $C_{3-7}$ residue, an aryl or heteroaryl residue,

$R^{10'}$ denotes hydrogen, a linear or branched, saturated or unsaturated aliphatic $C_{1-10}$ residue, an aryl or heteroaryl residue and

Z denotes at least one optionally present oxygen, sulfur or nitrogen as a ring atom,

and q denotes 0, 1, 2 or 3,

optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular hydrates.

**2.** Substituted benzo[b]azepin-2-one compounds and in each case the tautomers thereof according to claim 1, **characterised in that** $R^2$ and $R^3$, identical or different, denote a linear or branched, saturated or unsaturated aliphatic $C_{1-3}$ residue or a halogen and $R^1$ and $R_4$ in each case denote hydrogen, $R^5$ denotes hydrogen or a linear or branched, saturated or unsaturated aliphatic $C_{1-3}$ residue and $R^6$ denotes hydrogen or a residue of the formula $-CH_2-NR^7{}_2$, in which $R^7$ denotes a linear or branched, saturated or unsaturated aliphatic $C_{1-3}$ residue, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular hydrates.

**3.** Substituted benzo[b]azepin-2-one compounds and in each case the tautomers thereof according to claim 1, **characterised in that** $R^2$ and $R^3$ in each case denote a methyl group or a chlorine and $R^1$ and $R^4$ in each case denote hydrogen, $R^5$ denotes hydrogen or a methyl group and $R^6$ denotes hydrogen or a residue of the formula $-CH_2-N(CH_3)_2$, optionally in the form of the racemates thereof, the pure stereoisomers thereof, in particular enantiomers or diastereomers, or in the form of mixtures of the stereoisomers, in particular enantiomers or diastereomers, in any desired mixing ratio or in each case in the form of the acids or bases thereof or in the form of the salts thereof, in particular physiologically acceptable salts, or in the form of the solvates thereof, in particular hydrates.

**4.** Substituted benzo[b]azepin-2-one compounds and in each case the tautomers thereof according to claim 1, **characterised in that** $R^3$ denotes a linear or branched, saturated or unsaturated aliphatic $C_{1-3}$ residue or a halogen and $R^1$, $R^2$ and $R^4$ in each case denote hydrogen, $R^5$ denotes hydrogen or a linear or branched, saturated or unsaturated aliphatic $C_{1-3}$ residue and $R^6$ denotes hydrogen or a residue of the formula $-CH_2-N(R^7)_2$, in which $R^7$ denotes a linear or branched, saturated or unsaturated aliphatic $C_{1-3}$ residue, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular hydrates.

**5.** Substituted benzo[b]azepin-2-one compounds and in each case the tautomers thereof according to claim 1, **characterised in that** $R^3$ denotes a methyl group or a chlorine and $R^1$, $R^2$ and $R^4$ in each case denote hydrogen, $R^5$ denotes hydrogen or a methyl group and $R^6$ denotes hydrogen or a residue of the formula $-CH_2-N(CH_3)_2$, optionally in the form of the racemates thereof, the pure stereoisomers thereof, in particular enantiomers or diastereomers, or in the form of mixtures of the stereoisomers, in particular enantiomers or diastereomers, in any desired mixing ratio or in each case in the form of the acids or bases thereof or in the form of the salts thereof, in particular physiologically acceptable salts, or in the form of the solvates thereof, in particular hydrates.

**6.** Substituted benzo[b]azepin-2-one compounds and in each case the tautomers thereof according to claim 1, **characterised in that** $R^1$ and $R^3$, identical or different, denote a linear or branched, saturated or unsaturated aliphatic $C_{1-3}$ residue or a halogen and $R^2$ and $R^4$ in each case denote hydrogen, $R^5$ denotes hydrogen or a linear or branched, saturated or unsaturated aliphatic $C_{1-3}$ residue and $R^6$ denotes hydrogen or a residue of the formula $-CH_2-NR^7{}_2$, in which $R^7$ denotes a linear or branched, saturated or unsaturated aliphatic $C_{1-3}$ residue, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular hydrates.

**7.** Substituted benzo[b]azepin-2-one compounds and in each case the tautomers thereof according to claim 1, **characterised in that** $R^1$ and $R^3$ in each case denote a methyl group or a chlorine and $R^2$ and $R^4$ in each case denote hydrogen, $R^5$ denotes hydrogen or a methyl group and $R^6$ denotes hydrogen or a residue of the formula $-CH_2-N(CH_3)_2$, optionally in the form of the racemates thereof, the pure stereoisomers thereof, in particular enantiomers or diastereomers, or in the form of mixtures of the stereoisomers, in particular enantiomers or diastereomers, in any desired mixing ratio or in each case in the form of the acids or bases thereof or in the form of the salts thereof, in particular physiologically acceptable salts, or in the form of the solvates thereof, in particular hydrates.

**8.** Substituted benzo[b]azepin-2-one compounds and in each case the tautomers thereof according to one of claims 1-7, **characterised in that** A denotes a bridge of the formula $-CH_2-COO-$ or $-CH_2CONH-$ optionally in form of the racemates thereof, the pure stereoisomers thereof, in particular enantiomers or diastereomers, or in the form of mixtures of the stereoisomers, in particular the enantiomers or diastereomers, in any desired mixing ratio or in each

case in the form of the acids or bases thereof or in the form of the salts thereof, in particular physiologically acceptable salts, or in the form of the solvates thereof, in particular hydrates.

**9.** Substituted benzo[b]azepin-2-one compounds and in each case the tautomers thereof according to one of claims 1-8, **characterised in that** X denotes a residue of the following formula:

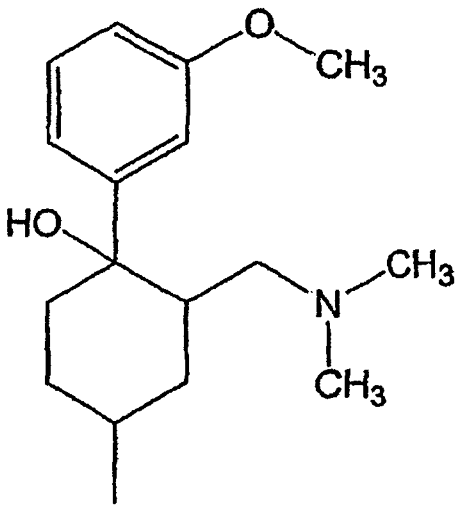

optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular hydrates.

**10.** Substituted benzo[b]azepin-2-one compounds and the tautomers thereof according to claim 1:

2'-(8-Chloro-2-oxo-2,3-dihydro-1H-benzo[b]azepin-5-yl) acetic acid [3"-(N,N-dimethylaminomethyl)-4"-hydroxy-4"-(m-methoxyphenyl)cyclohexyl] ester,
2'-(8-Chloro-1-methyl-2-oxo-2,3-dihydro-1H-benzo[b]azepin-5-yl) acetic acid [3"-(N,N-dimethylaminomethyl)-4"-hydroxy-4"-(m-methoxyphenyl)cyclohexyl] ester,
2'-(8-Chloro- 2-oxo- 2,3-dihydro- 1H-benzo [b] azepin- 5-yl) -N-[3"-(N, N-dimethylaminomethyl)- 4"-hydroxy-4"-(m-methoxyphenyl)cyclohexyl]acetamide,
2'-(8-Chloro- 1-methyl- 2-oxo- 2,3-dihydro- 1H-benzo [b] azepin- 5-yl) -N-[3"-(N, N-dimethylaminomethyl)- 4"-hydroxy-4"-(m-methoxyphenyl)cyclohexyl]acetamide

optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular hydrates.

**11.** A process for the production of substituted benzo[b]azepin-2-one compounds, the tautomers and corresponding stereoisomers thereof according to one of claims 1-10, **characterised in that**

A) an optionally substituted 2-aminobenzoic alkyl ester of the general formula (1), in which $R^1$, $R^2$, $R^3$, $R^4$ and $R^5$ have the same meaning as in one of claims 1-7 and R denotes an alkyl group, preferably a methyl or ethyl group,

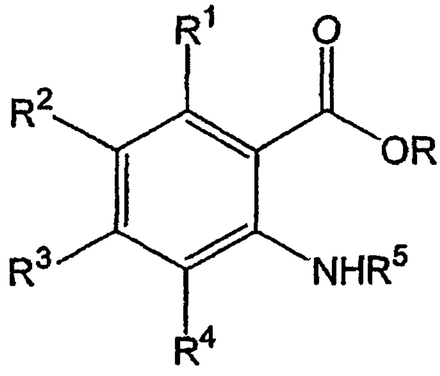

(1)

is reacted with succinic acid dialkyl ester of the general formula (2), in which R' denotes an alkyl group, preferably a methyl or ethyl group and $R^x$ denotes chlorine or an alkoxy group, preferably a methoxy or ethoxy group,

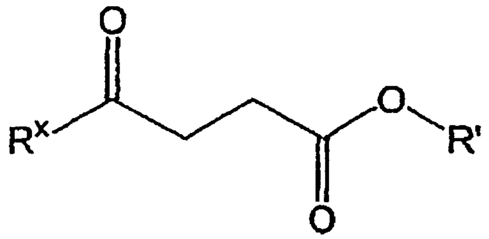

(2)

under suitable reaction conditions, in a suitable solvent, preferably pyridine, and is then worked up, optionally followed by purification of the optionally substituted N-(2-carbalkoxyphenyl)succinic acid alkyl ester amide formed of the general formula (3), in which R, R', $R^1$ $R^2$, $R^3$, $R^4$ and $^5$ have the above-stated meaning,

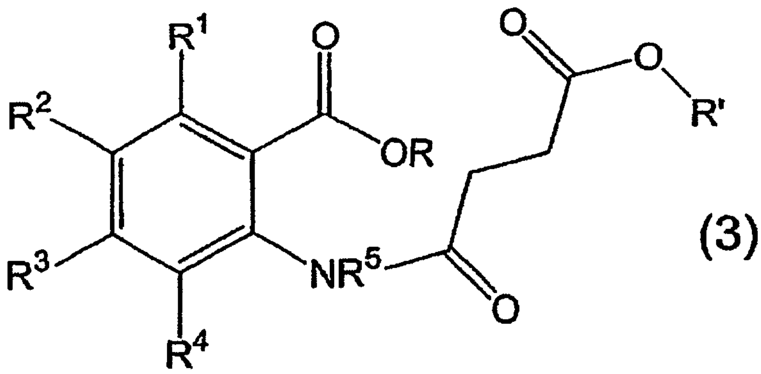

(3)

B) an optionally substituted N-(2-carboalkoxyphenyl)succinic acid alkyl ester amide of the general formula (3) is reacted in the presence of potassium tert-butanolate in a suitable solvent and then worked up, optionally followed by purification of the optionally substituted 5-hydroxy-2-oxo-2,3-dihydro-1H-benzo[b]azepin-4-carboxylic acid alkyl ester formed of the general formula (4), in which R', $R^1$, $R^2$, $R^3$, $R^4$ and $R^5$ have the above-stated meaning,

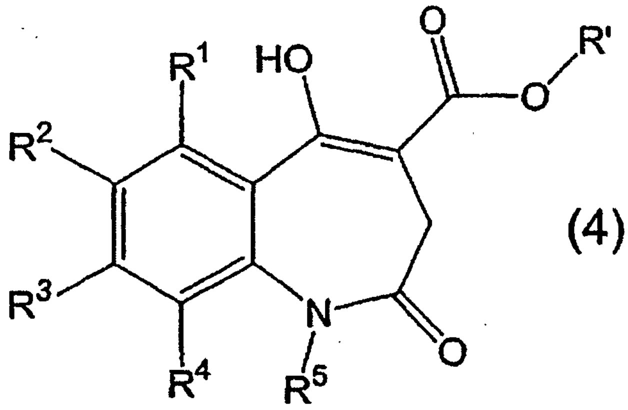

(4)

C) an optionally substituted 5-hydroxy-2-oxo-2,3-dihydro-1H-benzo[b]azepin-4-carboxylic acid alkyl ester of the general formula (4) is reacted in a dimethyl sulfoxide/water mixture at elevated temperature and then worked up, optionally followed by purification of the optionally substituted 2,3,4,5-tetrahydro-1H-benzo[b]azepin-2,5-dione of the general formula (5), in which $R^1$, $R^2$, $R^3$ $R^4$ and $^5$ have the above-stated meaning,

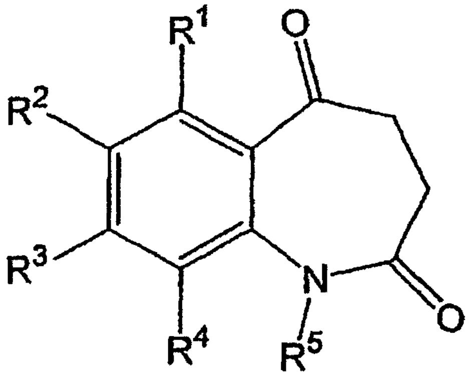

(5)

D) an optionally substituted 2,3,4,5-tetrahydro-1H-benzo[b]azepin-2,5-dione of the general formula (5) is reacted with a substituted aminomethylene hydrochloride of the general formula (6), in which the residue $R^7$ has the meaning stated in claim 1,

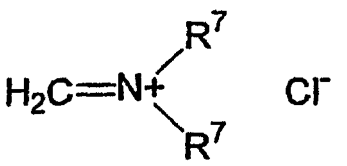

(6)

in the presence of an acid, preferably acetyl chloride, in a suitable solvent, preferably acetonitrile, and then worked up, optionally followed by purification of the optionally substituted aminomethyl-2,3,4,5-tetrahydro-1H-benzo[b]azepin-2,5-dione of the general formula (7), in which $R^1$, $R^2$, $R^3$, $R^4$, $R^5$ and $R^7$ have the above-stated meaning,

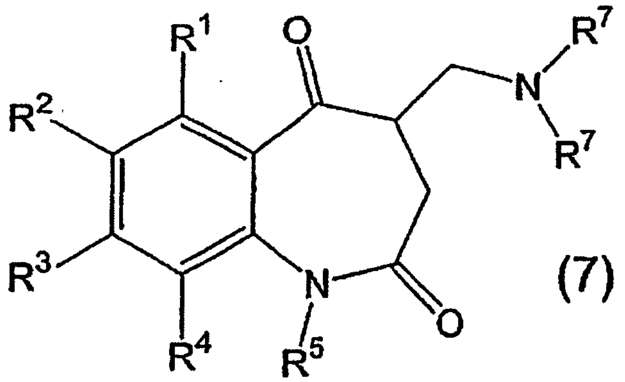

(7)

E) an optionally substituted 2,3,4,5-tetrahydro-1H-benzo[b]azepin-2,5-dione of the general formula (8), in which $R^1$, $R^2$, $R^3$, $R^4$, $R^5$ and $R^6$ have the same meaning as in one of claims 1-7 and which combines the compounds of the general formulae (5) and (7),

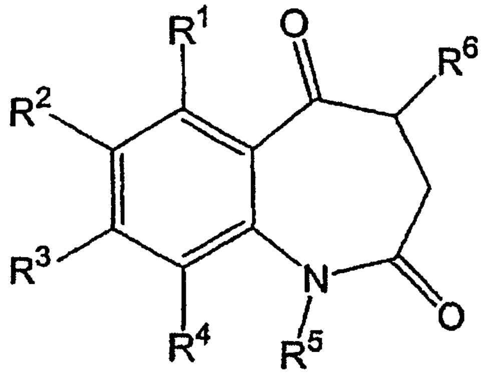

(8)

is reacted with a phosphonoalkanoic acid trialkyl ester of the general formula (9), in which n has the same meaning as in claim 1 and R" denotes an alkyl group, preferably a methyl or ethyl group,

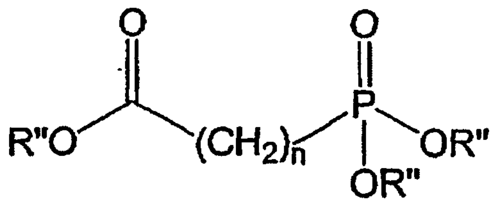

(9)

in the presence of a base, preferably potassium tert-butanolate, in a suitable solvent, preferably dimethylformamide, and then worked up, optionally followed by purification of the compound formed of the formula Y-CO-OR", in which R" has the above-stated meaning and Y denotes a residue of the general formula Y, in which the unoccupied bond line symbolises the bond to the residue -COOR" and

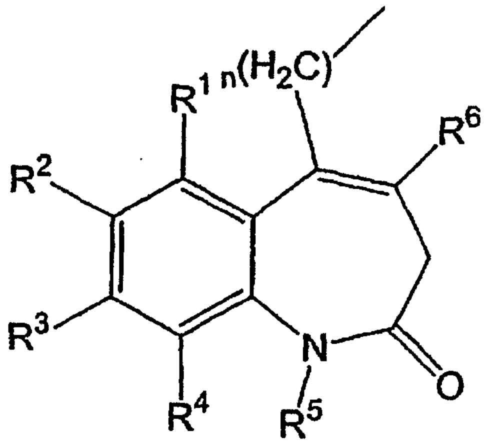

Y

in which $R^1$, $R^2$, $R^3$, $R^4$, $R^5$, $R^6$ and n have the above-stated meaning.

F) optionally an ester of the formula Y-COOR" is reacted in the presence of a base, preferably sodium or potassium hydroxide, in a suitable solvent, preferably an alcohol/water mixture, and then worked up, optionally followed by purification of the carboxylic acid formed of the formula Y-COOH, in which Y has the above-stated meaning,

G) optionally a carboxylic acid of the formula Y-COOH or a carboxylic acid ester of the formula Y-COOR", in which Y and R" have the above-stated meaning, is derivatised, **in that**

a) a carboxylic acid or carboxylic acid ester of the formula Y-COOH or Y-COOR" is reduced with the assistance of reducing agents, preferably lithium aluminium hydride, in a suitable solvent, preferably tetrahydrofuran, to the corresponding alcohol of the formula $Y\text{-}CH_2\text{-}OH$,

b) a carboxylic acid or carboxylic acid ester of the formula Y-COOH or Y-COOR" is reduced with the assistance of reducing agents, preferably diisobutylaluminium hydride, in a suitable solvent, preferably hexane, to the corresponding aldehyde of the formula Y-CHO or

c) an alcohol of the formula $Y\text{-}CH_2\text{-}OH$ according to a) is reacted with a brominating agent, preferably $PBr_3$ or $Ph_3PBr_2$ to yield the corresponding bromide of the formula $Y\text{-}CH_2\text{-}Br$

and then worked up and the product is optionally purified,

H) a compound of the formula $X^1\text{-}R^{IV}$, in which $X^1$ has the above-stated meaning and $R^{IV}$ denotes a functional group, is optionally produced **in that**

a) 1,4-cyclohexanedione monoethylene ketal, 4-aminocyclohexan-1-one ethylene ketal or 4-oxocyclohexane carboxylic acid is reacted with magnesium and a brominated or chlorinated, optionally substituted aromatic or heteroaromatic compound in a suitable solvent, preferably dry diethyl ether, at elevated temperature to yield the corresponding coupling product and then the ketal is optionally cleaved by reaction with hydrochloric acid in a suitable solvent, preferably tetrahydrofuran and worked up, optionally followed by purification of the product of the formula $X^{1a}$=O, $X^{1a}\text{-}NHR^{1'}$ or $X^{1a}\text{-}CO_2H$, in which $X^{1a}$ denotes a residue of the formula $X^{1a}$ and $R^{1'}$, $R^{2'}$ and Z have the above-stated meaning and the unoccupied bond line symbolises the bond to the residue =O, $-NHR^{1'}$ or $-CO_2H$,

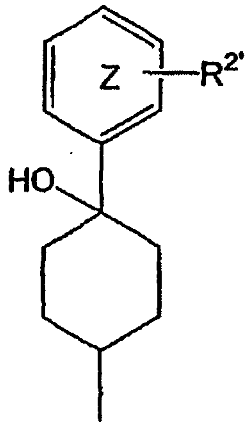

$X^{1a}$

b) optionally a ketone of the formula $X^{1a}$=O is reacted in the presence of a suitable reducing agent, preferably sodium borohydride, in a suitable solvent, preferably methanol, to yield the corresponding alcohol of the formula $X^{1a}$-OH, worked up and the product is optionally purified,
c) optionally a ketone of the formula $X^{1a}$=O is reacted under nitrogen in a suitable solvent, preferably tetrahydrofuran, firstly with ammonium trifluoroacetate and then with glacial acetic acid and sodium triacetoxy borohydride, to yield the corresponding amine of the formula $X^{1a}$-$NH_2$, worked up and the product is optionally purified,
d) optionally a carboxylic acid of the formula $X^{1a}$=$CO_2H$ is activated by reaction with dicyclohexylcarbodiimide or by conversion into the carboxylic acid chloride or a mixed anhydride, reacted with diazomethane in a suitable solvent, preferably ether, and then treated with water, worked up and the product of the formula $X^{1a}$-CO-$CH_2$-OH is optionally purified,
e) optionally the hydroxy group in position 4 of the cyclohexane ring in the residue $X^{1a}$ is converted into hydrogen, a halogen, an ether, ester, alkyl, aryl or heteroaryl group, **in that**

α) in order to introduce an ether group, a compound from one of steps a)-d) is reacted with an aliphatic or cycloaliphatic compound in the presence of a suitable catalyst in a suitable solvent, preferably in the presence of sodium hydride in dimethylformamide or in the presence of potassium hydroxide in dimethyl sulfoxide, or with an alkylating agent in a suitable solvent, preferably with a diazo compound in diethyl ether, or with an aryl or heteroaryl compound in the presence of diethylazo dicarboxylate and triphenylphosphine,
β) in order to introduce a halogen, a compound from one of steps a)-d) is reacted with a halogenating agent in a suitable solvent, preferably with $POCl_3$ in dimethylformamide, with $PPh_3/Cl_2$, with $PPh_3/Br_2$, with triphenylphosphine/n-chlorosuccinimide or with $HCl/ZnCl_2$,
γ) in order to introduce a hydrogen, a compound from step β) is reacted with hydrogen in the presence of a suitablecatalyst, preferably palladium/carbon, in a suitable solvent,
δ) in order to introduce an aliphatic or cycloaliphatic residue or an aryl or heteroaryl group, a compound from step β) is reacted with an aliphatic or cycloaliphatic boronic acid or a boronic acid ester or an aryl or heteroaryl borodihydroxide compound in the presence of palladium(II) acetate and potassium carbonate in a suitable solvent, preferably a dimethylformamide/water mixture, or
ε) in order to introduce an ester group, a compound from one of steps a)-d) is reacted with a carboxylic acid chloride in the presence of a suitable catalyst in a suitable solvent

and then worked up, optionally followed by purification of the compound formed of the formula $X^1$-$R^{IV}$, in which $X^1$ denotes the formula $X^1$

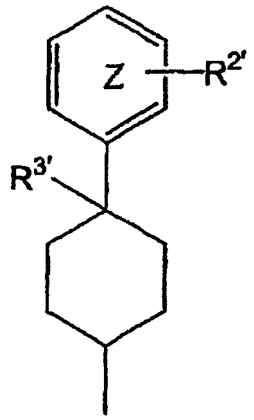

$X^1$

and $R^{IV}$, $R^{2'}$ and $R^{3'}$ have the above-stated meaning,

I) a compound of the formula $X-R^{IV}$, in which X has the above-stated meaning and $R^{IV}$ denotes a functional group, is optionally derivatised **in that**

a) a ketone of the formula X=O is reacted 1) with methoxymethyl triphenylphosphinium chloride under protective gas in a suitable solvent, preferably in dimethylformamide, in the presence of sodium hydride and then with hydrochloric acid or 2) with $Me_3S^+BF_4^-$ to yield the corresponding aldehyde X-CHO extended by one carbon atom,
b) an aldehyde of the formula X-CHO according to a) is reacted with a reducing agent, preferably sodium borohydride, in a suitable solvent, preferably an ethanol/water mixture, to yield the corresponding alcohol $X-CH_2-OH$,
c) an alcohol $C-CH_2-OH$ according to b) or of the formula X-OH is reacted with a brominating agent, preferably triphenylphosphine dibromide, in a suitable solvent, preferably acetonitrile, to yield the corresponding bromide of the formula $X-CH_2-Br$ or X-Br,
d) a bromide of the formula $X-CH_2-Br$ according to c) is reacted with a phosphine of the formula $PR^V_3$, in which $R^V$ denotes an organic residue, preferably a phenyl residue, in a suitable solvent, preferably toluene, ether, tetrahydrofuran or acetone, with cooling and under protective gas to yield the corresponding phosphonium salt $R^V_3P^+-CHX^-$ or
e) a bromide of the formula $X-CH_2-Br$ according to c) is reacted with a phosphite of the formula $HP(O)(OR^{VI})_2$, in which $R^{VI}$ denotes an organic residue, at elevated temperature, preferably 200°C, to yield the corresponding phosphonate $(R^{VI}O)_2P(O)-CH_2-X$

and then worked up and the product is optionally purified,
J) a compound from step F) or G), in which Y has the above-stated meaning, is reacted with a compound of the formula $X^1-R^{IV}$ from step H) or a compound $X-R^{IV}$ from step I), in which X, $X^1$ and $R^{IV}$ have the above-stated meaning, **in that**

a) a carboxylic acid of the formula Y-COOH is reacted with an amine of the formula $X-NH_2$ in the presence of a suitable condensing agent, preferably dicyclohexylcarbodiimide, 1-hydroxybenzotriazole and N-methylmorphine, in a suitable solvent, preferably dimethylformamide, with formation of an amide bridge,
b) a carboxylic acid of the formula Y-COOH is reacted with an alcohol of the formula X-OH in the presence of a suitable condensing agent in a suitable solvent with formation of an ester bridge, the reaction preferably taking place in the presence of methylimidazole and 1-(mesitylene-2'-sulfonyl)-3-nitro-1,2,4-triazole in tetrahydrofuran or in the presence of dicyclohexylcarbodiimide, 1-hydroxybenzotriazole and N-methylmorphine in dimethylformamide,
c) a bromide of the formula $Y-CH_2-Br$ is reacted with a compound of the formula $X-CO(CH_2)_p-OH$, in which p has the above-stated meaning, under protective gas in the presence of a suitable catalyst, preferably sodium hydride or potassium tert-butylate, in a suitable solvent, preferably dimethylformamide, with formation of a bridge of the formula $-CO(CH_2)_p-O-CH_2$,
d) an alcohol of the formula $Y-CH_2-OH$ is reacted with a bromide of the formula X-Br under protective gas in the presence of a suitable condensing agent, preferably sodium hydride or potassium tert-butylate, in a suitable solvent, preferably dimethylformamide, with formation of an ether bridge,

e) a bromide of the formula Y-$CH_2$-Br is reacted with an alcohol of the formula X-OH under protective gas in the presence of a suitable condensing agent, preferably sodium hydride or potassium tert-butylate, in a suitable solvent, preferably dimethylformamide, with formation of an ether bridge,
f) an aldehyde of the formula Y-CHO is reacted with an amine of the formula X-$NHR^{1'}$ in the presence of a suitable reducing agent, preferably sodium cyanoborohydride and sodium triacetoxyborohydride, in a suitable solvent, preferably a mixture of tetrahydrofuran and 1,2-dichloroethane, with formation of an amino bridge,
g) an aldehyde of the formula Y-CHO is reacted with a phosphonium salt $R^V{}_3P^+$-$CHX^-$, in which $R^V$ has the above-stated meaning, under protective gas in the presence of suitable catalysts in a suitable solvent, preferably in the presence of sodium methanolate in a mixture of hexane, diethyl ether and/or diisopropyl ether or in the presence of sodium hydride, potassium tert-butylate or a lithium amide in dimethylformamide or dimethyl sulfoxide, with formation of a -CH=CH- bridge or
h) an aldehyde of the formula Y-CHO is reacted with a phosphonate of the formula $(R^{VI}O)_2P(O)$-$CH_2$-X, in which $R^{VI}$ has the above-stated meaning, under protective gas in the presence of suitable catalysts, preferably sodium methanolate, sodium hydroxide, potassium hydroxide, sodium hydride, potassium tert-butylate or a lithium amide, in a suitable solvent, preferably dimethylformamide, dimethyl sulfoxide, diethyl ether, tetrahydrofuran, with formation of a -CH=CH- bridge and
i) optionally the -CH=CH- bridge from step g) or h) is hydrogenated by hydrogen, preferably at standard pressure or elevated pressure of up to 100 bar, in the presence of suitable catalysts, preferably transition metals or transition metal compounds, preferably palladium or the salts thereof, rhodium or the complexes thereof, in a suitable solvent, preferably dimethylformamide, methanol or ethanol, at a temperature of between 20 and 100°C with formation of a -$CH_2$-$CH_2$ bridge

and then worked up and the product is optionally purified,
K) optionally the double bond in the 7-membered ring of one of the reaction products from step J) is hydrogenated by hydrogen, preferably at standard pressure or elevated pressure of up to 100 bar, in the presence of suitable catalysts, preferably transition metals or transition metal compounds, preferably palladium or the salts thereof, rhodium or the complexes thereof, in a suitable solvent, preferably dimethylformamide, methanol or ethanol, at a temperature of between 20 and 100°C and then worked up and the product is optionally purified.

**12.** A pharmaceutical preparation containing at least one substituted benzo[b]azepin-2-one compound or a corresponding tautomer, optionally in the form of the racemate thereof, the pure stereoisomer thereof, in particular enantiomer or diastereomer, or in the form of mixtures of the stereoisomers, in particular the enantiomers or diastereomers, in any desired mixing ratio or in each case in the form of the acid or base thereof or in the form of the salt thereof, in particular a physiologically acceptable salt, or in each case in the form of the solvate thereof, in particular the hydrate, according to one of claims 1-10, and optionally physiologically acceptable auxiliary substances.

**13.** A pharmaceutical preparation according to claim 12 for combatting pain.

**14.** A pharmaceutical preparation according to claim 13 for combatting chronic pain.

**15.** A pharmaceutical preparation according to claim 13 for combatting neuropathic pain.

**16.** A pharmaceutical preparation according to claim 12 for the treatment or prevention of neurodegenerative diseases, preferably of Alzheimer's disease, Parkinson's disease or Huntington's chorea.

**17.** A pharmaceutical preparation according to claim 12 for the treatment or prevention of stroke.

**18.** A pharmaceutical preparation according to claim 12 for the treatment or prevention of cerebral ischaemia.

**19.** A pharmaceutical preparation according to claim 12 for the treatment or prevention of cerebral infarct.

**20.** A pharmaceutical preparation according to claim 12 for the treatment or prevention of cerebral oedema.

**21.** A pharmaceutical preparation according to claim 12 for the treatment or prevention of insufficiency states of the central nervous system, preferably hypoxia or anoxia.

**22.** A pharmaceutical preparation according to claim 12 for the treatment or prevention of epilepsy.

**23.** A pharmaceutical preparation according to claim 12 for the treatment or prevention of schizophrenia.

**24.** A pharmaceutical preparation according to claim 12 for the treatment or prevention of psychoses brought about by elevated amino acid levels.

**25.** A pharmaceutical preparation according to claim 12 for the treatment or prevention of AIDS dementia.

**26.** A pharmaceutical preparation according to claim 12 for the treatment or prevention of Tourette's syndrome.

**27.** A pharmaceutical preparation according to claim 12 for the treatment or prevention of encephalomyelitis.

**28.** A pharmaceutical preparation according to claim 12 for the treatment or prevention of perinatal asphyxia.

**29.** A pharmaceutical preparation according to claim 12 for the treatment or prevention of tinnitus.

**30.** A pharmaceutical preparation according to claim 12 for the treatment or prevention of migraine.

**31.** A pharmaceutical preparation according to claim 12 for the treatment or prevention of inflammatory and/or allergic reactions.

**32.** A pharmaceutical preparation according to claim 12 for the treatment or prevention of depression.

**33.** A pharmaceutical preparation according to claim 12 for the treatment or prevention of mental health conditions.

**34.** A pharmaceutical preparation according to claim 12 for the treatment or prevention of urinary incontinence.

**35.** A pharmaceutical preparation according to claim 12 for the treatment or prevention of pruritus.

**36.** A pharmaceutical preparation according to claim 12 for the treatment or prevention of diarrhoea.

**37.** A pharmaceutical preparation according to claim 12 for anxiolysis.

**38.** A pharmaceutical preparation according to claim 12 for anaesthesia.

**39.** Use of at least one substituted benzo[b]azepin-2-one compound or a tautomer thereof, optionally in the form of the racemate thereof, the pure stereoisomer thereof, in particular enantiomer or diastereomer, or in the form of mixtures of the stereoisomers, in particular the enantiomers or diastereomers, in any desired mixing ratio or in each case in the form of the acid or base thereof or in the form of the salt thereof, in particular a physiologically acceptable salt, or in each case in the form of the solvate thereof, in particular the hydrate, according to one of claims 1-10 for the production of a pharmaceutical preparation for the combatting of pain, preferably of chronic or neuropathic pain.

**40.** Use of at least one substituted benzo[b]azepin-2-one compound or a tautomer thereof, optionally in the form of the racemate thereof, the pure stereoisomer thereof, in particular enantiomer or diastereomer, or in the form of mixtures of the stereoisomers, in particular the enantiomers or diastereomers, in any desired mixing ratio or in each case in the form of the acid or base thereof or in the form of the salt thereof, in particular a physiologically acceptable salt, or in each case in the form of the solvate thereof, in particular the hydrate, according to one of claims 1-10 for the production of a pharmaceutical preparation for the treatment or prevention of neurodegenerative diseases, preferably Alzheimer's disease, Parkinson's disease or Huntington's chorea, for the treatment or prevention of stroke, cerebral ischaemia, cerebral infarct, cerebral oedema, insufficiency states of the central nervous system, preferably hypoxia or anoxia, epilepsy, schizophrenia, psychoses brought about by elevated amino acid levels, AIDS dementia, encephalomyelitis, Tourette's syndrome, perinatal asphyxia, tinnitus, migraine, inflammatory and/or allergic reactions, depression, mental health conditions, urinary incontinence, pruritus or diarrhoea or for anxiolysis or anaesthesia.

## Revendications

**1.** Composés substitués de benzo[b]azépin-2-one des formules générales I et II et à chaque fois leurs tautomères,

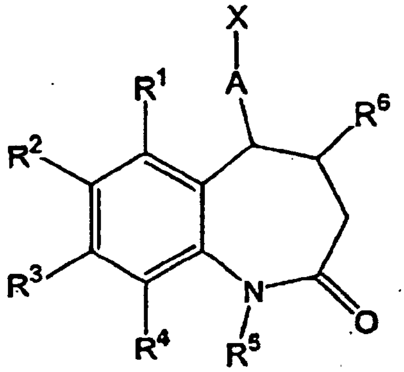

I

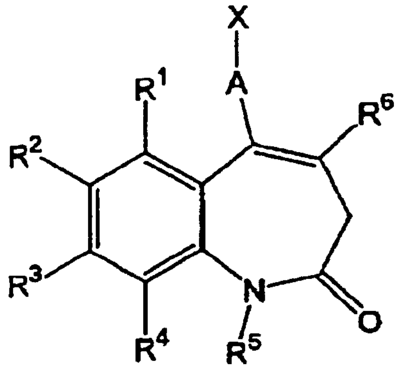

II

dans lesquelles

$R^1$, $R^2$, $R^3$ et $R^4$ sont identiques ou différents et représentent un radical aliphatique en $C_1$ à $C_{10}$ linéaire ou ramifié, saturé ou insaturé ou un radical cycloaliphatique en $C_3$ à $C_7$ saturé ou insaturé, chacun des radicaux susmentionnés pouvant le cas échéant être lié via un pont éther, ou hydrogène, un halogène ou un groupe hydroxy,

$R^5$ représente hydrogène, un radical aliphatique en $C_1$ à $C_{10}$ linéaire ou ramifié, saturé ou insaturé, un radical aryle ou hétéroaryle,

$R^6$ représente hydrogène ou un radical de formule $-CH_2-NR^7{}_2$, les deux radicaux étant identiques ou différents et ayant la signification ci-après ou pouvant former ensemble, avec l'atome d'azote qui les relie comme élément de cycle, un cycle de 3 à 8 chaînons,

$R^7$ représente un radical aliphatique en $C_1$ à $C_8$ linéaire ou ramifié, saturé ou insaturé ou un radical cycloaliphatique en $C_3$ à $C_6$ saturé ou insaturé

A représente un pont présentant une des formules suivantes : $-(CH_2)_{n+2}-$, $-(CH_2)_n-CH=CH-$, $-(CH_2)_nCOO-$, $-(CH_2)_nCONH-$, $-(CH_2)_{n+1}O(CH_2)_pCO-$, $-(CH_2)_{n+1}O-$, $-(CH_2)_{n+1}NR^{1'}-$, dans lesquelles n représente 0,1,2,3 et p représente 0 ou 1, $R^{1'}$ a la signification indiquée dans la suite et la liaison au radical X est toujours mentionnée en dernier lieu

et X représente un des radicaux suivants des formules générales $X^1$ à $X^6$ et $X^{16}$, dans lesquelles le trait libre représente la liaison au pont A et

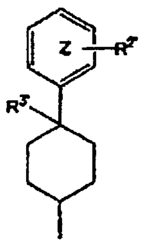

$X^1$

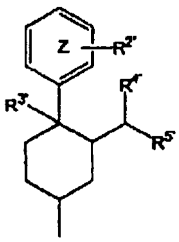

$X^2$

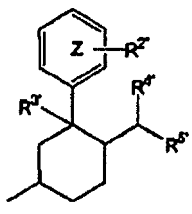

$X^3$

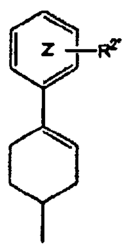

$X^4$

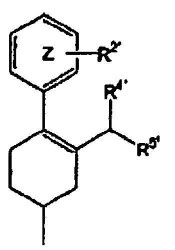

$X^5$

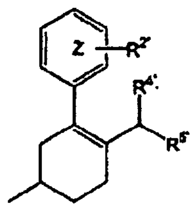

$X^6$

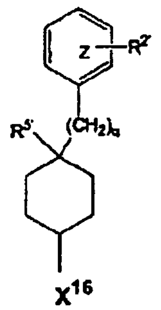

dans lesquelles
$R^{1'}$ représente hydrogène, un radical aliphatique en $C_1$ à $C_{10}$ linéaire ou ramifié, saturé ou insaturé, un radical cycloaliphatique en $C_3$ à $C_7$ saturé ou insaturé, un radical aryle ou hétéroaryle,
$R^{2'}$ représente un radical aliphatique en $C_1$ à $C_{10}$ linéaire ou ramifié, saturé ou insaturé, un radical cycloaliphatique en $C_3$ à $C_7$ saturé ou insaturé ou un radical aryle ou hétéroaryle, tous les radicaux susmentionnés pouvant le cas échéant être liés via un pont éther, thioéther ou $SO_2$, ou il représente hydrogène, un halogène, un groupe hydroxy, thiol, cyano ou nitro ou un groupe de formule $-NR^{1'}_2$, les deux radicaux $R^{1'}$ étant identiques ou différents et ayant la signification susmentionnée,
$R^{3'}$ représente un radical aliphatique en $C_1$ à $C_{10}$ linéaire ou ramifié, saturé ou insaturé, un radical cycloaliphatique en $C_3$ à $C_7$ saturé ou insaturé, un radical aryle ou hétéroaryle, tous les radicaux susmentionnés pouvant le cas échéant être liés via un pont éther ou un pont ester, hydrogène, un halogène, un groupe hydroxy,
$R^{4'}$ représente hydrogène, un radical aryle ou hétéroaryle, le radical aryle ou hétéroaryle pouvant présenter au moins un substituant $R^{2'}$ ayant la signification susmentionnée à l'exception de l'hydrogène,
$R^{5'}$ représente un radical de formule $-NR^{6'}_2$, les deux radicaux $R^{6'}$ pouvant être identiques ou différents et présentant la signification ci-après ou pouvant former ensemble, avec l'atome d'azote qui les relie comme élément de cycle, un cycle de 3 à 7 chaînons, qui peut le cas échéant contenir comme atome de cycle au moins un oxygène et/ou au moins un autre azote, l'azote pouvant présenter un substituant $R^{10'}$ ayant la signification ci-après,
$R^{6'}$ représente un radical aliphatique en $C_1$ à $C_6$ linéaire ou ramifié, saturé ou insaturé, un radical cycloaliphatique en $C_3$ à $C_7$ saturé ou insaturé, un radical aryle ou un radical hétéroaryle,
$R^{10'}$ représente hydrogène, un radical aliphatique en $C_1$ à $C_{10}$ linéaire ou ramifié, saturé ou insaturé, un radical aryle ou hétéroaryle et
Z représente au moins un oxygène, un soufre ou un azote comme atome de cycle, le cas échéant présent,
q représente 0,1,2,3,
le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

2. Composés substitués de benzo[b]azépin-2-one et à chaque fois leurs tautomères selon la revendication 1, **caractérisés en ce que** $R^2$ et $R^3$ sont identiques ou différents et représentent un radical aliphatique en $C_1$ à $C_3$ linéaire ou ramifié, saturé ou insaturé ou un halogène et $R^1$ et $R^4$ représentent à chaque fois hydrogène, $R^5$ représente hydrogène ou un radical aliphatique en $C_1$ à $C_3$ linéaire ou ramifié, saturé ou insaturé et $R^6$ représente hydrogène ou un radical de formule $-CH_2-NR^7_2$, dans laquelle $R^7$ représente un radical aliphatique en $C_1$ à $C_3$ linéaire ou ramifié, saturé ou insaturé, le cas échéant sous forme de leurs racémates, leurs stéréo-isomères purs, en particulier leurs énantiomères ou les diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

3. Composés substitués de benzo[b]azépin-2-one et à chaque fois leurs tautomères selon la revendication 1, **caractérisés en ce que** $R^2$ et $R^3$ représentent à chaque fois un groupe méthyle ou un chlore et $R^1$ et $R^4$ représentent à chaque fois hydrogène, $R^5$ représente hydrogène ou un groupe méthyle et $R^6$ représente hydrogène ou un radical de formule $-CH_2-N(CH_3)_2$, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme

de leurs acides ou de leurs bases ou sous forme de leurs sels, en particulier des sels physiologiquement acceptables, ou sous forme de leurs solvates, en particulier des hydrates.

4. Composés substitués de benzo[b]azépin-2-one et à chaque fois leurs tautomères selon la revendication 1, **caractérisés en ce que** $R^3$ représente un radical aliphatique en $C_1$ à $C_3$ linéaire ou ramifié, saturé ou insaturé ou un halogène et $R^1$, $R^2$ et $R^4$ représentent à chaque fois hydrogène, $R^5$ représente hydrogène ou un radical aliphatique en $C_1$ à $C_3$ linéaire ou ramifié, saturé ou insaturé et $R^6$ représente hydrogène ou un radical de formule $-CH_2-N(R^7)_2$, dans laquelle $R^7$ représente un radical aliphatique en $C_1$ à $C_3$ linéaire ou ramifié, saturé ou insaturé, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

5. Composés substitués de benzo[b]azépin-2-one et à chaque fois leurs tautomères selon la revendication 1, **caractérisés en ce que** $R^3$ représente un groupe méthyle ou un chlore et $R^1$, $R^2$ et $R^4$ représentent à chaque fois hydrogène, $R^5$ représente hydrogène ou un groupe méthyle et $R^6$ représente hydrogène ou un radical de formule $-CH_2-N(CH_3)_2$, le cas échéant sous forme de leurs racémates, leurs stéréo-isomères purs, en particulier leurs énantiomères ou diastéréo-isomères, ou sous forme de mélanges des stéréo-isomères, en particulier des. énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

6. Composés substitués de benzo[b]azépin-2-one et à chaque fois leurs tautomères selon la revendication 1, **caractérisés en ce que** $R^1$ et $R^3$ sont identiques ou différents et représentent un radical aliphatique en $C_1$ à $C_3$ linéaire ou ramifié, saturé ou insaturé ou un halogène et $R^2$ et $R^4$ représentent à chaque fois hydrogène, $R^5$ représente hydrogène ou un radical aliphatique en $C_1$ à $C_3$ linéaire ou ramifié, saturé ou insaturé et $R^6$ représente hydrogène ou un radical de formule $-CH_2-NR^7_2$, dans laquelle $R^7$ représente un radical aliphatique en $C_1$ à $C_3$ linéaire ou ramifié, saturé ou insaturé, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

7. Composés substitués de benzo[b]azépin-2-one et à chaque fois leurs tautomères selon la revendication 1, **caractérisés en ce que** $R^1$ et $R^3$ représentent à chaque fois un groupe méthyle ou un chlore et $R^2$ et $R^4$ représentent à chaque fois hydrogène, $R^5$ représente hydrogène ou un groupe méthyle et $R^6$ représente hydrogène ou un radical de formule $-CH_2-N(CH_3)_2$, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

8. Composés substitués de benzo[b]azépin-2-one et à chaque fois leurs tautomères selon l'une quelconque des revendications 1 à 7, **caractérisés en ce que** A représente un pont des formules $-CH_2-COO-$ ou $-CH_2-CONH-$, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

9. Composés substitués de benzo[b]azépin-2-one et à chaque fois leurs tautomères selon l'une quelconque des revendications 1 à 8, **caractérisés en ce que** X représente un radical présentant la formule suivante :

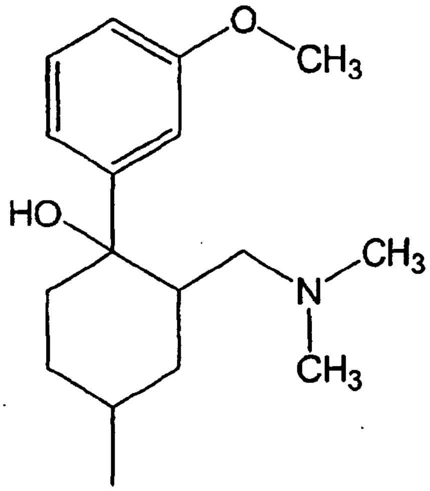

le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**10.** Composés substitués de benzo[b]azépin-2-one et leurs tautomères selon la revendication 1:

ester 3"-(N,N-diméthylaminométhyl)-4"-hydroxy-4"-(m-méthoxyphényl)-cyclohexylique de l'acide 2'-(8-chloro-2-oxo-2,3-dihydro-1H-benzo[b]azépin-5-yl)acétique,
ester 3"-(N,N-diméthylaminométhyl)-4"-hydroxy-4"-(m-méthoxyphényl)cyclohexylique de l'acide 2'-(8-chloro-1-méthyl-2-oxo-2,3-dihydro-1H-benzo[b]azépin-5-yl)acétique,
2'-(8-chloro-2-oxo-2,3-dihydro-1H-benzo[b]azépin-5-yl)-N-[3"-(N,N-diméthylaminométhyl)-4"-hydroxy-4"-(m-méthoxyphényl)cyclohexyl]acétamide,
2'-(8-chloro- 1-méthyl- 2-oxo- 2,3-dihydro- 1H-benzo [b] azépin- 5-yl) -N-[3"-(N, N-diméthylaminométhyl)- 4"-hydroxy-4"-(m-méthoxyphényl)-cyclohexyl]acétamide

le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**11.** Procédé pour la préparation de composés substitués de benzo[b]azépin-2-one, leurs tautomères ainsi que les stéréo-isomères correspondants selon l'une quelconque des revendications 1 à 10, **caractérisé**

A) en ce qu'on transforme un ester alkylique de l'acide 2-aminobenzoïque le cas échéant substitué de formule générale (1), dans laquelle $R^1$, $R^2$, $R^3$, $R^4$ et $R^5$ ont la même signification que dans les revendications 1 à 7 et R représente un groupe alkyle, de préférence un groupe méthyle ou éthyle,

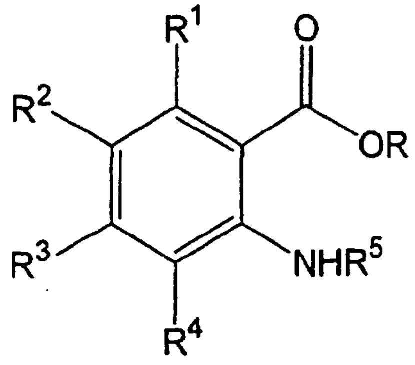

(1)

avec un ester dialkylique de l'acide succinique de formule générale (2), dans laquelle R' représente un groupe alkyle, de préférence un groupe méthyle ou éthyle, et $R^x$ représente un chlore ou un groupe alcoxy, de préférence un groupe méthoxy ou éthoxy,

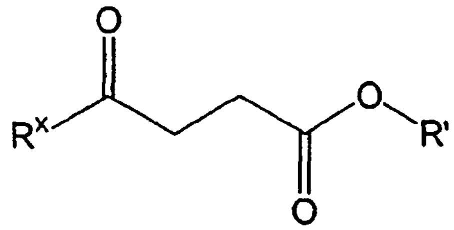

(2)

dans des conditions de réaction appropriées, dans un solvant approprié, de préférence la pyridine, puis on traite et on purifie le cas échéant l'amide-ester alkylique de l'acide N-(2-carboalcoxyphényl)succinique formé, le cas échéant substitué, de formule générale (3) dans laquelle R, R', $R^1$, $R^2$, $R^3$, $R^4$ et $R^5$ ont la signification susmentionnée,

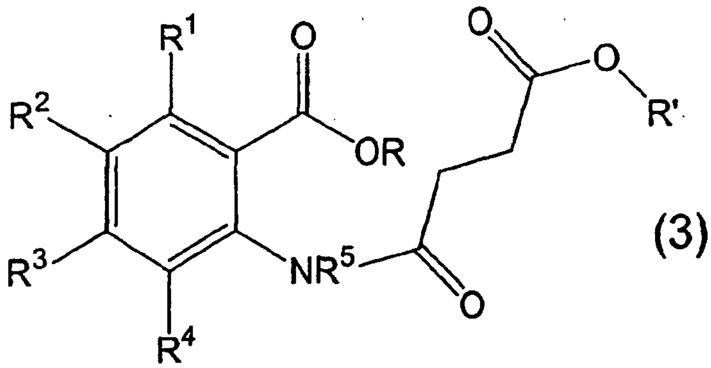

(3)

B) on transforme un amide-ester alkylique de l'acide N-(2-carboalcoxyphényl)succinique le cas échéant substitué de formule générale (3) en présence de tert-butanolate de potassium dans un solvant approprié, puis on traite et on purifie le cas échéant l'ester alkylique de l'acide 5-hydroxy-2-oxo-2,3-dihydro-1H-benzo[b]azépine-4-carboxylique formé le cas échéant substitué de formule générale (4), dans laquelle R', $R^1$, $R^2$, $R^3$, $R^4$ et $R^5$ ont la signification susmentionnée,

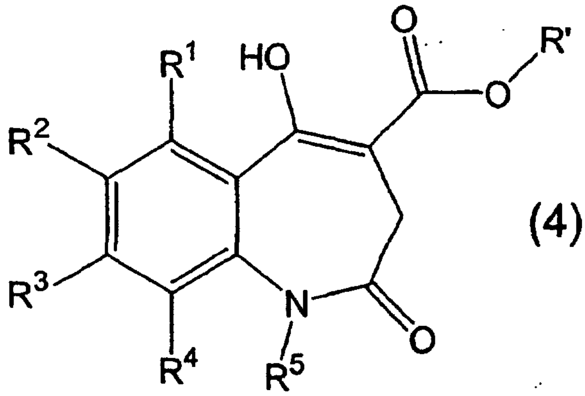

(4)

C) on transforme un ester alkylique de l'acide 5-hydroxy-2-oxo-2,3-dihydro-1H-benzo[b]azépine-4-carboxylique le cas échéant substitué de formule générale (4) dans un mélange diméthylsulfoxyde/eau à température élevée, puis on traite et on purifie le cas échéant la 2,3,4,5-tétrahydro-1H-benzo[b]azépine-2,5-dione formée le cas échéant substituée de formule générale (5), dans laquelle $R^1$, $R^2$, $R^3$, $R^4$ et $R^5$ ont la signification susmentionnée,

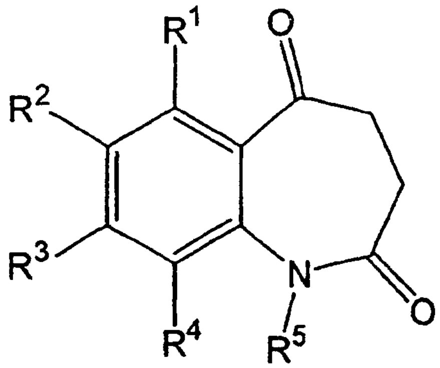

(5)

D) on transforme une 2,3,4,5-tétrahydro-1H-benzo[b]azépine-2,5-dione le cas échéant substituée de formule générale (5) avec un chlorhydrate d'aminométhylène substitué de formule générale (6), dans laquelle le radical $R^7$ a la signification mentionnée dans la revendication 1,

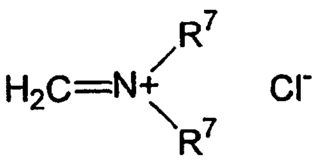

(6)

en présence d'un acide, de préférence le chlorure d'acétyle, dans un solvant approprié, de préférence l'acétone et on traite ensuite et on purifie l'aminométhyl-2,3,4,5-tétrahydro-1H-benzo[b]azépine-2,5-dione formée le cas échéant substituée de formule générale (7), dans laquelle $R^1$, $R^2$, $R^3$, $R^4$, $R^5$ et $R^7$ ont la signification susmentionnée,

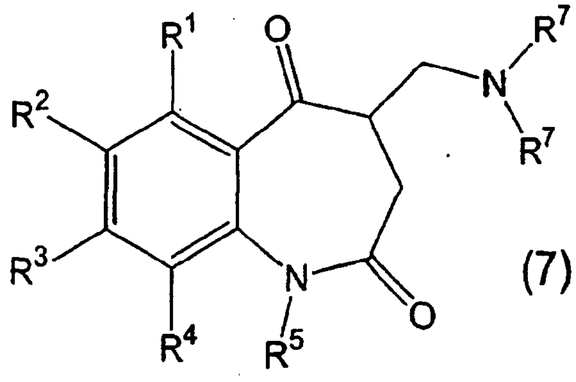

(7)

E) on transforme une 2,3,4,5-tétrahydro-1H-benzo[b]azépine-2,5-dione le cas échéant substituée de formule générale (8), dans laquelle $R^1$, $R^2$, $R^3$, $R^4$, $R^5$ et $R^6$ ont la même signification que dans l'une quelconque des revendications 1 à 7 et qui résume les composés des formules générales (5) et (7),

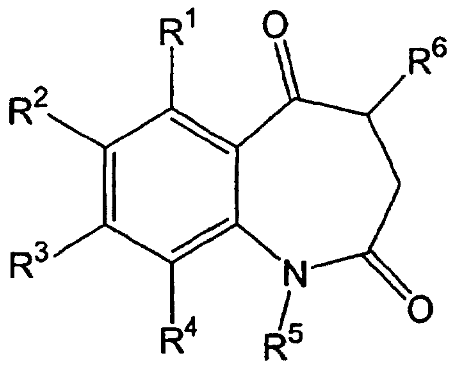

(8)

avec un ester trialkylique d'un acide phosphonoalcanoïque de formule générale (9), dans laquelle n a la même signification que dans la revendication 1 et R" représente un groupe alkyle, de préférence un groupe méthyle ou éthyle,

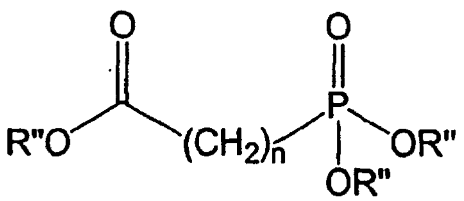

(9)

en présence d'une base, de préférence le tert-butanolate de potassium, dans un solvant approprié, de préférence le diméthylformamide, puis on traite et on purifie le cas échéant le composé formé de formule Y-COOR", dans laquelle R" a la signification susmentionnée et Y représente un radical de formule générale Y, dans laquelle le trait libre symbolise la liaison avec le radical -COOR" et

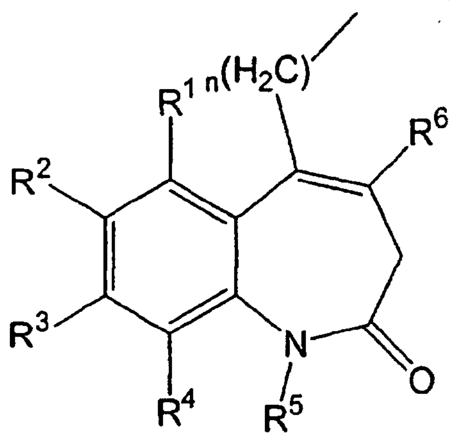

Y

dans laquelle $R^1$, $R^2$, $R^3$, $R^4$, $R^5$, $R^6$ et n ont la signification susmentionnée,
F) on transforme le cas échéant un ester de formule générale Y-COOR" en présence d'une base, de préférence l'hydroxyde de sodium ou de potassium, dans un solvant approprié, de préférence un mélange alcool/eau, puis on traite et on purifie le cas échéant l'acide carboxylique de formule Y-COOH formé, dans laquelle Y a la signification susmentionnée,
G) on dérive le cas échéant un acide carboxylique de formule Y-COOH ou un ester carboxylique de formule Y-COOR", dans lesquelles Y et R" ont la signification susmentionnée,

a) en ce qu'on réduit un acide carboxylique ou un ester d'acide carboxylique de formule Y-COOH ou Y-COOR" à l'aide de réducteurs, de préférence l'hydrure de lithium et d'aluminium, dans un solvant approprié, de préférence le tétrahydrofuranne, en alcool correspondant de formule Y-$CH_2$-OH,
b) on réduit un acide carboxylique ou un ester d'acide carboxylique de formule Y-COOH ou Y-COOR" à l'aide de réducteurs, de préférence l'hydrure de diisobutylaluminium, dans un solvant approprié, de préférence l'hexane, en aldéhyde correspondant de formule Y-CHO ou
c) on transforme un alcool de formule Y-$CH_2$-OH selon a) avec un agent de bromuration, de préférence $PBr_3$ ou $Ph_3PBr_2$, en bromure correspondant de formule Y-$CH_2$-Br

puis on traite et on purifie le cas échéant le produit,
H) on prépare le cas échéant un composé de formule $X^1$-$R^{IV}$, dans laquelle $X^1$ a la signification susmentionnée et $R^{IV}$ représente un groupe fonctionnel, préparé

a) en ce qu'on transforme du 1,4-cyclohexanedionemonoéthylènecétal, du 4-aminocyclohexan-1-onéthylènecétal ou de l'acide 4-oxocyclohexanecarboxylique avec du magnésium et un aromatique ou un hétéroaromatique bromé ou chloré, le cas échéant substitué, dans un solvant approprié, de préférence le diéthyléther anhydre, à température élevée en produit de couplage correspondant puis on dissocie le cas échéant le cétal par transformation avec de l'acide chlorhydrique dans un solvant approprié, de préférence le tétrahydrofuranne, puis on traite et on purifie le cas échéant le produit de formule $X^{1a}$=O, $X^{1a}$-$NHR^{1'}$ ou $X^{1a}$-$CO_2H$, dans laquelle $X^{1a}$ représente un radical de formule $X^{1a}$ et $R^{1'}$, $R^{2'}$ et Z ont la signification susmentionnée et le trait libre symbolise la liaison avec le radical =O, -$NHR^{1'}$ ou -$CO_2H$,

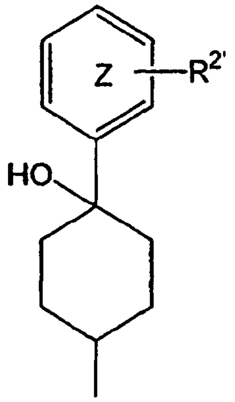

$X^{1a}$

b) on transforme le cas échéant une cétone de formule $X^{1a}$=O en présence d'un réducteur approprié, de préférence le borohydrure de sodium, dans un solvant approprié, de préférence le méthanol, en alcool correspondant de formule $X^{1a}$-OH, puis on traite et on purifie le cas échéant le produit,

c) on transforme le cas échéant une cétone de formule $X^{1a}$=O sous azote dans un solvant approprié, de préférence le tétrahydrofuranne, d'abord avec du trifluoroacétate d'ammonium puis avec de l'acide acétique glacial et du triacétoxyborohydrure de sodium en amine correspondante de formule $X^{1a}$-$NH_2$, puis on traite et on purifie le cas échéant le produit,

d) on active le cas échéant un acide carboxylique de formule $X^{1a}$-$CO_2H$ par transformation avec du dicyclohexylcarbodiimide ou par transformation en chlorure d'acide carboxylique ou un anhydride mixte, on transforme avec du diazométhane dans un solvant approprié, de préférence l'éther, puis on traite avec de l'eau et on purifie le cas échéant le produit de formule $X^{1a}$-CO-$CH_2$-OH,

e) on transforme le cas échéant le groupe hydroxy en 4ème position du cycle cyclohexane dans le radical $X^{1a}$ en hydrogène, un halogène, un groupe éther, ester, alkyle, aryle ou hétéroaryle,

α) en ce qu'on transforme un composé provenant d'une des étapes a) à d), pour l'introduction d'un groupe éther, avec un composé aliphatique ou cycloaliphatique en présence d'un catalyseur approprié dans un solvant approprié, de préférence en présence d'hydrure de sodium dans du diméthylformamide ou en présence d'hydroxyde de potassium dans du diméthylsulfoxyde ou avec un agent d'alkylation dans un solvant approprié, de préférence avec un composé diazo dans du diéthyléther ou avec un composé aryle ou hétéroaryle en présence d'azodicarboxylate de diéthyle et de triphénylphosphine,

β) on transforme un composé provenant d'une des étapes a) à d) pour l'introduction d'un halogène avec un agent d'halogénation dans un solvant approprié, de préférence avec du $POCl_3$ dans du diméthylformamide, avec du $PPh_3/Cl_2$, du $PPh_3/Br_2$, avec un mélange de triphénylphosphine/n-chlorosuccinimide ou avec du $HCl/ZnCl_2$,

γ) on transforme un composé de l'étape β) pour l'introduction d'un hydrogène avec de l'hydrogène en présence d'un catalyseur approprié, de préférence du palladium/carbone, dans un solvant approprié,

δ) on transforme un composé de l'étape β) pour l'introduction d'un radical aliphatique ou cycloaliphatique ou un groupe aryle ou hétéroaryle avec un acide boronique ou un ester d'acide boronique aliphatique ou cycloaliphatique ou un composé de dihydroxyde d'arylbore ou d'hétéroarylbore en présence d'acétate de palladium (II) et de carbonate de potassium dans un solvant approprié, de préférence dans un mélange diméthylformamide/eau ou

ε) on transforme un composé d'une des étapes a) à d) pour l'introduction d'un groupe ester avec un chlorure d'acide carboxylique en présence d'un catalyseur approprié dans un solvant approprié

puis on traite et on purifie le cas échéant le composé formé de formule $X^1$-$R^{IV}$, dans laquelle $X^1$ représente la formule $X^1$

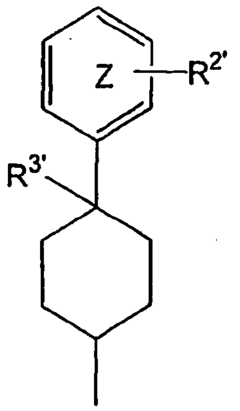

$X^1$

et $R^{IV}$, $R^{2'}$ et $R^{3'}$ ont la signification susmentionnée,

I) on dérive le cas échéant un composé de formule X-$R^{IV}$, dans laquelle X a la signification susmentionnée et $R^{IV}$ représente un groupe fonctionnel,

a) en ce qu'on transforme une cétone de formule X=O 1) avec du chlorure de méthoxyméthyltriphénylphosphinium sous gaz protecteur dans un solvant approprié, de préférence dans du diméthylformamide, en présence d'hydrure de sodium et ensuite avec de l'acide chlorhydrique ou 2) avec du $Me_3S^+BF_4^-$ en aldéhyde X-CHO correspondant, allongé d'un atome de carbone
b) on transforme un aldéhyde de formule X-CHO selon a) avec un réducteur, de préférence le borohydrure de sodium, dans un solvant approprié, de préférence un mélange éthanol/eau en alcool correspondant X-$CH_2$-OH,
c) on transforme un alcool X-$CH_2$-OH selon b) ou de formule X-OH avec un agent de bromuration, de préférence le dibromure de triphénylphosphine, dans un solvant approprié, de préférence l'acétonitrile, en bromure correspondant de formule X-$CH_2$-Br ou X-Br,
d) on transforme un bromure de formule X-$CH_2$-Br selon c) avec une phosphine de formule $PR^V_3$, dans laquelle $R^V$ représente un radical organique, de préférence un radical phényle, dans un solvant approprié, de préférence le toluène, l'éther, le tétrahydrofuranne ou l'acétone, sous refroidissement et un gaz protecteur en sel de phosphonium correspondant $R^V_3P^+$-$CHX^-$ ou
e) on transforme un bromure de formule X-$CH_2$-Br selon c) avec un phosphite de formule $HP(O)(OR^{VI})_2$, dans laquelle $R^{VI}$ représente un radical organique, à température élevée, de préférence 200°C, en phosphonate correspondant $(R^{VI}O)_2P(O)$-$CH_2$-X

puis on traite et on purifie le cas échéant le produit,
J) on transforme un composé de l'étape F) ou G), dans laquelle Y a la signification susmentionnée, avec un composé de formule $X^1$-$R^{IV}$ de l'étape H) ou un composé X-$R^{IV}$ de l'étape I), dans laquelle X, $X^1$ et $R^{IV}$ ont la signification susmentionnée,

a) en ce qu'on transforme un acide carboxylique de formule Y-COOH avec une amine de formule X-$NH_2$ en présence d'un agent de condensation approprié, de préférence le dicyclohexylcarbodiimide, le 1-hydroxybenzotriazole et la N-méthylmorphine, dans un solvant approprié, de préférence le diméthylformamide, avec formation d'un pont amide,
b) on transforme un acide carboxylique de formule Y-COOH avec un alcool de formule X-OH en présence d'un agent de condensation approprié dans un solvant approprié avec formation d'un pont ester, la réaction s'effectuant de préférence en présence de méthylimidazole et de 1-(mésitylène-2'-sulfonyl)-3-nitro-1,2,4-triazole dans du tétrahydrofuranne ou en présence de dicyclohexylcarbodiimide, de 1-hydroxybenzotriazole et de N-méthylmorphine dans du diméthylformamide,
c) on transforme un bromure du formule Y-$CH_2$-Br avec un composé de formule X-$CO(CH_2)_pOH$, dans laquelle p a la signification susmentionnée, sous gaz protecteur en présence d'un catalyseur approprié, de préférence l'hydrure de sodium ou le tert-butylate de potassium, dans un solvant approprié, de préférence le diméthylformamide, avec formation d'un pont de formule -$CO(CH_2)_p$-O-$CH_2$-,
d) on transforme un alcool de formule Y-$CH_2$-OH avec un bromure de formule X-Br sous gaz protecteur

en présence d'un agent de condensation approprié, de préférence l'hydrure de sodium ou le tert-butylate de potassium, dans un solvant approprié, de préférence le diméthylformamide, avec formation d'un pont éther,

e) on transforme un bromure de formule Y-$CH_2$-Br avec un alcool de formule X-OH sous gaz protecteur en présence d'un agent de condensation approprié, de préférence l'hydrure de sodium ou le tert-butylate de potassium, dans un solvant approprié, de préférence le diméthylformamide, avec formation d'un pont éther,

f) on transforme un aldéhyde de formule Y-CHO avec une amine de formule X-$NHR^{1'}$ en présence d'un réducteur approprié, de préférence le cyanoborohydrure de sodium et le triacétoxyborohydrure de sodium, dans un solvant approprié, de préférence un mélange de tétrahydrofuranne et de 1,2-dichloréthane, avec formation d'un pont amino,

g) on transforme un aldéhyde de formule Y-CHO avec un sel de phosphonium $R^V{}_3P^+$-$CHX^-$, dans lequel $R^V$ a la signification susmentionnée, sous gaz protecteur en présence de catalyseurs appropriés dans un solvant approprié, de préférence en présence de méthanolate de sodium dans un mélange d'hexane, de diéthyléther et/ou de diisopropyléther ou en présence d'hydrure de sodium, de tert-butylate de potassium ou d'un amide de lithium dans du diméthylformamide ou du diméthylsulfoxyde, avec formation d'un pont -CH=CH- ou

h) on transforme un aldéhyde de formule Y-CHO avec un phosphonate de formule $(R^{VI}O)_2P(O)$-$CH_2$-X, dans laquelle $R^{VI}$ a la signification susmentionnée, sous gaz protecteur en présence de catalyseurs appropriés, de préférence le méthanolate de sodium, l'hydroxyde de sodium, l'hydroxyde de potassium, l'hydrure de sodium, le tert-butylate de potassium ou un amide de lithium, dans un solvant approprié, de préférence le diméthylformamide, le diméthylsulfoxyde, le diéthyléther, le tétrahydrofuranne, avec formation d'un pont -CH=CH- et

i) on hydrogène le cas échéant le pont -CH=CH- de l'étape g) ou h) avec de l'hydrogène, de préférence à pression normale ou une pression élevée jusqu'à 100 bars, en présence de catalyseurs appropriés, de préférence les métaux de transition ou les composés de métaux de transition, de préférence le palladium ou ses sels, le rhodium ou ses complexes, dans un solvant approprié, de préférence le diméthylformamide, le méthanol ou l'éthanol, à une température entre 20 et 100°C, avec formation d'un pont -$CH_2$-$CH_2$-

puis on traite et on purifie le cas échéant le produit,

K) on hydrogène le cas échéant la double liaison dans l'heptacycle des produits de réaction provenant de l'étape J) avec de l'hydrogène, de préférence à pression normale ou à pression élevée jusqu'à 100 bars, en présence de catalyseurs appropriés, de préférence les métaux de transition ou les composés de métaux de transition, de préférence le palladium ou ses sels, le rhodium ou ses complexes, dans un solvant approprié, de préférence le diméthylformamide, le méthanol ou l'éthanol, à une température entre 20 et 100°C puis on traite et on purifie le cas échéant le produit.

**12.** Médicament contenant au moins un composé substitué de benzo[b]azépin-2-one ou un tautomère correspondant, le cas échéant sous forme de son racémate, ses stéréo-isomères purs, en particulier les énantiomères ou les diastéréo-isomères, ou sous forme de mélanges des stéréo-isomères, en particulier des énantiomères ou des diastéréo-isomères, dans un rapport de mélange quelconque ou à chaque fois sous forme de son acide ou de sa base ou sous forme de son sel, en particulier d'un sel physiologiquement acceptable, ou sous forme de son solvate, en particulier de l'hydrate, selon l'une quelconque des revendications 1 à 10 et le cas échéant des adjuvants physiologiquement acceptables.

**13.** Médicament selon la revendication 12 destiné à lutter contre la douleur.

**14.** Médicament selon la revendication 13 destiné à lutter contre la douleur chronique.

**15.** Médicament selon la revendication 13 destiné à lutter contre la douleur neuropathique.

**16.** Médicament selon la revendication 12 destiné au traitement ou à la prophylaxie de maladies de neuro-dégénérescence, de préférence la maladie d'Alzheimer, de Parkinson ou de Huntington.

**17.** Médicament selon la revendication 12 destiné au traitement ou à la prophylaxie d'une attaque.

**18.** Médicament selon la revendication 12 destiné au traitement ou à la prophylaxie d'une ischémie cérébrale.

**19.** Médicament selon la revendication 12 destiné au traitement ou à la prophylaxie d'un infarctus cérébral.

**20.** Médicament selon la revendication 12 destiné au traitement ou à la prophylaxie d'un oedème cérébral.

**21.** Médicament selon la revendication 12 destiné au traitement ou à la prophylaxie d'états de sous-approvisionnement du système nerveux central, de préférence de l'hypoxie ou de l'anoxie.

**22.** Médicament selon la revendication 12 destiné au traitement ou à la prophylaxie de l'épilepsie.

**23.** Médicament selon la revendication 12 destiné au traitement ou à la prophylaxie de la schizophrénie.

**24.** Médicament selon la revendication 12 destiné au traitement ou à la prophylaxie de psychoses provoquées par un niveau élevé d'acides aminés.

**25.** Médicament selon la revendication 12 destiné au traitement ou à la prophylaxie de la démence liée au SIDA.

**26.** Médicament selon la revendication 12 destiné au traitement ou à la prophylaxie du syndrome de Tourette.

**27.** Médicament selon la revendication 12 destiné au traitement ou à la prophylaxie de l'encéphalomyélite.

**28.** Médicament selon la revendication 12 destiné au traitement ou à la prophylaxie de l'asphyxie périnatale.

**29.** Médicament selon la revendication 12 destiné au traitement ou à la prophylaxie de Tinnitus.

**30.** Médicament selon la revendication 12 destiné au traitement ou à la prophylaxie de la migraine.

**31.** Médicament selon la revendication 12 destiné au traitement ou à la prophylaxie de réactions inflammatoires et/ou allergiques.

**32.** Médicament selon la revendication 12 destiné au traitement ou à la prophylaxie de dépressions.

**33.** Médicament selon la revendication 12 destiné au traitement ou à la prophylaxie de maladies psychiques.

**34.** Médicament selon la revendication 12 destiné au traitement ou à la prophylaxie de l'incontinence urinaire.

**35.** Médicament selon la revendication 12 destiné au traitement ou à la prophylaxie du prurit.

**36.** Médicament selon la revendication 12 destiné au traitement ou à la prophylaxie de la diarrhée.

**37.** Médicament selon la revendication 12 destiné à l'anxiolyse.

**38.** Médicament selon la revendication 12 destiné à l'anesthésie.

**39.** Utilisation d'au moins un composé substitué de benzo[b]azépin-2-one ou de son tautomère, le cas échéant sous forme de son racémate, ses stéréo-isomères purs, en particulier les énantiomères ou les diastéréo-isomères, ou sous forme de mélanges des stéréo-isomères, en particulier des énantiomères ou des diastéréo-isomères, dans un rapport de mélange quelconque ou à chaque fois sous forme de son acide ou de sa base ou sous forme de son sel, en particulier d'un sel physiologiquement acceptable, ou sous forme de son solvate, en particulier de l'hydrate, selon l'une quelconque des revendications 1 à 10 pour la préparation d'un médicament destiné à lutter contre la douleur, de préférence la douleur chronique ou neuropathique.

**40.** Utilisation d'au moins un composé substitué de benzo[b]azépin-2-one ou de son tautomère, le cas échéant sous forme de son racémate, ses stéréo-isomères purs, en particulier les énantiomères ou les diastéréo-isomères, ou sous forme de mélanges des stéréo-isomères, en particulier des énantiomères ou des diastéréo-isomères, dans un rapport de mélange quelconque ou à chaque fois sous forme de son acide ou de sa base ou sous forme de son sel, en particulier d'un sel physiologiquement acceptable, ou sous forme de son solvate, en particulier de l'hydrate, selon l'une quelconque des revendications 1 à 10 pour la préparation d'un médicament destiné au traitement ou à la prophylaxie de maladies de neuro-dégénérescence, de préférence de la maladie d'Alzheimer, de Parkinson ou

de Huntington, destiné au traitement ou à la prophylaxie d'une attaque, d'une ischémie cérébrale, d'un infarctus cérébral, d'un oedème cérébral, d'états de sous-approvisionnement du système nerveux central, de préférence de l'hypoxie ou de l'anoxie, de l'épilepsie, de la schizophrénie, de psychoses provoquées par un niveau élevé d'acides aminés, de la démence liée au SIDA, de l'encéphalomyélite, du syndrome de Tourette, de l'asphyxie périnatale, du Tinnitus, de la migraine, de réactions inflammatoires et/ou allergiques, de dépressions, de maladies psychiques, de l'incontinence urinaire, du prurit ou de la diarrhée ou destiné à l'anxiolyse ou l'anesthésie.